# EUROPEAN PATENT APPLICATION

(11) **EP 4 501 945 A1**
(43) Date of publication of application: **05.02.2025**
(21) Application number: 23773968.5
(22) Date of filing: 23.03.2023
(51) Int. Cl.: C07K 7/08, C07K 7/50, A61K 47/64, A61K 38/00, A61K 47/54, A61K 31/00

(54) **CLEAVABLE FRAGMENT DIRECTED BY AFFINITY FRAGMENT, DESIGN AND SYNTHESIS THEREOF, AND USE THEREOF IN PREPARATION OF SITE-DIRECTED DRUG CONJUGATE**

(30) Priority: 25.03.2022 CN 202210307276
(71) Applicant: Shanghai Institute of Materia Medica, Chinese Academy of Sciences, Shanghai 201203 (CN)
(72) Inventor: HUANG, Wei, Shanghai 201203 (CN); TANG, Feng, Shanghai 201203 (CN); ZENG, Yue, Shanghai 201203 (CN)
(74) Representative: Lavoix
(86) International application number: PCT/CN2023/083443
(87) International publication number: WO 2023/179723

(57) **Abstract**

Provided in the present invention are a cleavable fragment directed by an affinity fragment, the design and the synthesis thereof, and the use thereof in the preparation of a site-directed drug conjugate. Specifically, provided in the present invention is a conjugate with a ligand affinity directing group. The conjugate is as represented by formula I: AT-CL-R (I), wherein AT is an affinity moiety for a target protein (TP); CL is a cleavable fragment which has a self-cleaving reactivity; and R is a group to be modified to the target protein.

## Description

### TECHNICAL FIELD

The present invention belongs to the field of pharmaceutical chemistry, specifically, relates to a class of cleavable fragment directed by affinity fragment, design and synthesis thereof, and use thereof in preparation of site-specific drug conjugate.

### BACKGROUND

Antibody-drug conjugate is a type of targeting drug that connects biologically active cytotoxic drug molecules to antibodies through chemical linkages, and transports cytotoxic small molecules to target cells through the targeting effect of antibodies so as to exert effect. Since random conjugation of small molecule drugs is not conducive to the study of pharmacokinetic and pharmacodynamics, and it is difficult to obtain uniform data in clinical evaluation, and it is also difficult to have consistent quality in different batches during the production process, many conjugation strategies for the site-directed and quantitative introduction of small molecule drugs have been developed, and show good application prospects.

The antibodies in antibody-drug conjugates are mainly IgG type antibodies, which have variable Fab regions and constant crystallizable Fc domains. The site-specific connection of small molecule drugs in the Fc domain does not affect the recognition of antibody-antigen, and at the same time shows better efficacy and stability. Therefore, the site-specific conjugation of small molecule drugs in the Fc domain is an important research direction. At present, there are many technical methods for selectively modifying antibodies aiming at the Fc domain of antibodies. Among them, the research on achieving selective modification of specific amino acids based on Fc ligand guidance provides a good method for site-specific ADC drug research. However, the site-specific modification method based on Fc ligand guidance either cannot remove Fc ligand or the removal process is complicated, which is not conducive to the stability and safety of antibodies. Therefore, it is of great research significance to develop an efficient Fc ligand-guided complex to directly achieve site-specific and quantitative modification of natural antibodies.

### SUMMARY OF THE INVENTION

The purpose of the present invention is to provide a cleavable fragment directed by affinity fragment, design and synthesis thereof, and use thereof in the preparation of site-specific drug conjugate. Specifically, the present invention provides a class of thioester structure, which is used to prepare a thioester-based acyl transfer reagent, such reagant can achieve site-specific and quantitative modification of antibodies under the guidance of an affinity fragment. The present invention relates to the design and synthesis of the fragments and uses thereof.

In the first aspect of the present invention, provided is a conjugate containing a ligand affinity directing group, wherein the conjugate is of Formula I:

AT-CL-R (I)

wherein,
AT is an affinity moiety for target protein (TP);
CL is a cleavable fragment; and possesses divalent fragments as shown below in the CL; 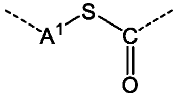
wherein A¹ is each independently optionally substituted C₁₋₁₀ alkylene (preferably C₁₋₃ alkylene), optionally substituted C₆₋₁₀ aryl, or optionally substituted 5 to 10 membered heteroaryl:
   R is a group that needs to be modified onto the target protein (the group can be any desired group or a group containing a functional substance, and the R group may contain a single desired group, or two or more same or different desired groups or functional substances);
   unless otherwise specified, the substituted refers to that one or more H in the group is substituted with a substituent selected from the group consisting of: halogen (such as F, Cl, Br, I), C₁₋₆ alkyl (preferably, C₁₋₄ alkyl, such as methyl, ethyl), and C₁₋₆ haloalkyl (preferably, C₁₋₄ haloalkyl).

In another preferred embodiment, the cleavable fragment has auto cleaving reactivity (e.g. it can be cleaved under specific reaction conditions/when contacts with specific reactants or reactive groups).

In another preferred embodiment, the affinity moiety is derived from small molecule compound that can form reversible covalent bond with the target protein, or derived from protein or peptide structure that has an affinity for the target protein.

In another preferred embodiment, in 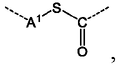 A¹ is the end close to AT, and CO is the end close to R.

In another preferred embodiment, the conjugate is an acyl transfer reagent used for site-specific modification of the target protein (such as antibody or Fc fusion protein).

In another preferred embodiment, the affinity moiety for target protein refers to a moiety that has an affinity for or can reversibly bind to (can reversibly covalently bind to) the target protein.

In another preferred embodiment, the target protein is an antibody or fusion protein. In another preferred embodiment, the antibody is an antibody or fusion protein containing Fc domain.

In another preferred embodiment, the target protein is an antibody, and the antibody is an antibody containing Fc domain; in this case, the corresponding AT is a small molecule compound that can reversibly covalently bind to the antibody, or protein or peptide structure that has affinity.

In another preferred embodiment, the antibody comprises: monoclonal antibody, bifunctional antibody, monoclonal antibody, nano-antibody containing Fc fragment, Fc fusion protein, or combinations thereof.

In another preferred embodiment, the selected antibody comprises: Trastuzumab, Pertuzumab, Rituximab, Cetuximab, Muromonab, Gemtuzumab, Abciximab, Daclizumab, Adalimumab, Palivizumab, Basiliximab, Bevacizumab, Panitumumab, Nimotuzumab, Denosumab, Disitamab, Ramucirumab, Necitumumab, Ipilimumab, daratumumab, Brentuximab, Alemtuzumab, Elotuzumab, Blinatumomab, Nivolumab, Pembrolizumab, Atezolizumab, Avelumab, Durvalumab, Toripalimab, Catumaxomab, Blinatumomab, Emicizumab, Amivantamab (Rybrevant), or combinations thereof.

In another preferred embodiment, the antibody comprises: Trastuzumab, Rituximab, Pertuzumab, Bevacizumab, Toripalimab, Nivolumab (IgG4), Panituzumab (IgG2), or combinations thereof.

In another preferred embodiment, AT is a moiety derived from a small molecule compound that can reversibly bind to the target protein (preferably, a small molecule compound that forms a reversible covalent bond), or a moiety derived from a protein or polypeptide that has an affinity for the target protein.

In another preferred embodiment, AT is a moiety derived from a small molecule compound that can reversibly bind to the antibody (preferably, a small molecule compound that forms a reversible covalent bond), or a moiety derived from a protein or polypeptide that has an affinity for the antibody (preferably, derived from Fc binding peptide; more preferably, derived from a polypeptide fragment that is derived from ProteinA or ProteinG and has the ability to bind to Fc (Fc segment)).

In another preferred embodiment, AT is derived from a sequence in Protein A (Protein A that has an affinity for Fc).

In another preferred embodiment, AT is derived from a binding peptide that has an affinity for antibody Fab.

In another preferred embodiment, the polypeptide is a cyclic peptide.

In another preferred embodiment, AT is derived from a peptide selected from the group consisting of: Fc-III peptide (such as DCAWHLGELVWCT(SEQ ID No.2)), Fc binding peptides (such as GPDCAYHRGELVWCTFH(SEQ ID No.3)), RGNCAYHRGQLVWCTYH(SEQ ID No.4), CDCAWHLGELVWCTC(SEQ ID No.5), and the like), and combinations thereof.

In another preferred embodiment, AT is a core sequence derived from an Fc binding polypeptide containing the sequence of formula II:

(Xₛ₁)t₁-Cys-Aa1-Aa2-Aa3-Aa4-Aa5-Aa6-Aa7-Aa8-Aa9-Cys-(Yₛ₂)ₜ₂ (II)

wherein,
X is located at the N-terminal of the polypeptide, and Y is located at the C-terminal of the polypeptide;
s1 = 0, 1, 2 or 3;
s2 = 0, 1, 2 or 3;
X and Y are each independently amino acid residues;
t1 and t2 are each independently an integer of 0 to 10 (preferably, t1 and t2 are each independently 0, 1, 2 or 3, more preferably, t1 and t2 are each independently 0 or 1);
Cys is a cysteine residue;
Aa1, Aa2, Aa3, Aa5, Aa7, Aa8, and Aa9 are each independently amino acid residues;
Aa4 and Aa6 are each independently amino acid residues, and at least one of Aa4 and Aa6 is an amino acid residue containing an NH₂ group on its side chain (such as lysine residue (K), etc.) or an amino acid residue containing -COOH group on its side chain (such as aspartic acid residue (D), glutamic acid residue (E), etc.).

In another preferred embodiment, Aa1, Aa2, Aa3, Aa4, Aa5, Aa6, Aa7, Aa8, and Aa9 are not cysteine residues.

In another preferred embodiment, said derived refers to the removal of an H from -NH₂ in the side chain of Aa4 or Aa6 to form -NH-, or the formation of -CONH- from -COOH in the side chain of Aa4 or Aa6 and amino group (i.e. AT is connected to CL through the side chain of Aa4 or Aa6).

In another preferred embodiment, Aa6 is an amino acid residue containing an NH₂ group on its side chain or an amino acid residue containing an -COOH group on its side chain.

In another preferred embodiment, said derived refers to the removal of an H from -NH₂ in the side chain of Aa6 to form -NH-, or the formation of -CONH- from -COOH in the side chain of Aa6 and amino group (i.e. AT is connected to CL through the side chain of Aa6).

In another preferred embodiment, the amino acid residue is an amino acid residue derived from natural or non-natural amino acid.

In another preferred embodiment, AT is derived from a cyclic peptide.

In another preferred embodiment, AT is derived from a cyclic peptide formed by forming a -S-S- bond between two Cys in the sequence shown in Formula II.

In another preferred embodiment, Xₛ₁ and Yₛ₂ are each independently null or 1 to 3 consecutive the same or different amino acid residues; preferably, Yₛ₂ and Yₛ₃ are each independently 1 to 3 consecutive the same or different amino acid residues (i.e., s1=1, 2 or 3, s2=1, 2 or 3).

In another preferred embodiment,
Aa1 is an alanine residue (A), a serine residue (S), or a threonine residue (T); and/or
Aa2 is a tyrosine residue (Y), a histidine residue (H), or a tryptophan residue (W); and/or
Aa3 is a histidine residue (H), a phenylalanine residue (F), a tyrosine residue (Y), a tryptophan residue (W), an arginine residue (R), or a glycine residue (G); and/or
Aa5 is a glycine residue (G), a serine residue (S), an asparagine residue (N), a glutamine residue (Q), an aspartic acid residue (D), a glutamic acid residue (E), a phenylalanine residue (F), a tyrosine residue (Y), a tryptophan residue (W), a histidine residue (H), a threonine residue (T), a leucine residue (L), an alanine residue (A), a valine residue (V), an isoleucine residue (I), or an arginine residue (R); and/or
Aa7 is a leucine residue (L), an isoleucine residue (I), a valine residue (V), an alanine residue (A), a glutamine residue (Q), or a glutamic acid residue (E); and/or
Aa8 is a valine (V), a isoleucine residue (I), or a leucine residue (L); and/or
Aa9 is a tryptophan residue (W) or a phenylalanine residue (F).

In another preferred embodiment, the amino acid residue containing an NH₂ group on its side chain is as shown below 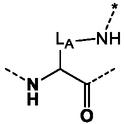 wherein * refers to the connection site with CL; L_{A} is substituted or unsubstituted C1-8 alkylene; preferably, is -(CH₂)ₙ₁- and n1=1, 2, 3, 4, 5, or 6; preferably, n1=3, 4, or 5.

In another preferred embodiment, the amino acid residue containing an -COOH group on its side chain is as shown below 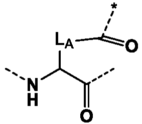 wherein * refers to the connection site with -NH-CL; L_{A} is substituted or unsubstituted C1-8 alkylene; preferably, is -(CH₂)ₙ₁- and n1=1, 2, 3, 4, 5, or 6; preferably, n1=2, 3, 4, or 5.

In another preferred embodiment, the amino acid residue containing an NH₂ group on its side chain is a lysine residue (K).

In another preferred embodiment, the amino acid residue containing a -COOH group on its side chain is an aspartic acid residue (D), or a glutamic acid residue (E).

In another preferred embodiment, Aa4 is an amino acid residue containing an NH₂ group on its side chain, and Aa6 is a glutamine residue (Q), a glutamic acid residue (E), a histidine residue (H), an asparagine residue (N), a proline residue (P), an aspartic acid residue (D), a lysine residue (K), or a glycine residue (G); or Aa6 is an amino acid residue containing an NH₂ group on its side chain, and Aa4 is an arginine residue (R), a leucine residue (L), a lysine residue (K), an aspartic acid residue (D), a glutamic acid residue (E), a 2-aminooctanedioic acid, or a diaminopropionic acid.

In another preferred embodiment, Aa4 is an amino acid residue containing a -COOH group on its side chain, a 2-aminooctanedioic acid, or a diaminopropionic acid, and Aa6 is a glutamine residue (Q), a glutamic acid residue (E), a histidine residue (H), an asparagine residue (N), a proline residue (P), an aspartic acid residue (D), a lysine residue (K), or a glycine residue (G); or Aa6 is an amino acid residue containing a -COOH group on its side chain, 2-aminooctanedioic acid, or diaminopropionic acid, and Aa4 is an arginine residue (R), a leucine residue (L), a lysine residue (K), an aspartic acid residue (D), a glutamic acid residue (E), a 2-aminooctanedioic acid, or a diaminopropionic acid.

In another preferred embodiment, the sequence shown in formula II is HYTCWVLKGRHYACNGR (SEQ ID No.6), HYTCWVLDGRHYACNGR (SEQ ID NO. 7), or HYTCWVLEGRHYACNGR (SEQ ID No.8).

In another preferred embodiment, AT is a small molecule fragment that has a reversible covalent interaction with a protein; preferably, AT is selected from the group consisting of:

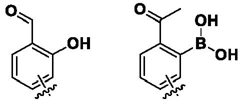

In another preferred embodiment, CL is as shown below: 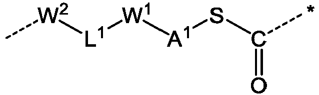 wherein,
* represents the connection site with R
W¹ is selected from the group consisting of: null (single bond), -NH-, -C(O)-, -C(O)-NH-, and -NH-C(O)-;
W² is null (single bond), -NH-, or -C(O)-;
L¹ is null (single bond) or a divalent linker group;
A¹ is defined as above.

In another preferred embodiment, L¹ is null (single bond), or a divalent linker group consisting of one or more (preferably 1 to10; more preferably 1, 2, 3, 4, or 5, most preferably 1, 2 or 3) units selected from the group consisting of: amino acid residue, optionally substituted C₁₋₄ alkylene, -(CH₂-NH-CO)-, -(CH₂-CH₂-CO)-, -(CH₂-CH₂-O)-, and -(CH₂-O-CH₂)-.

In another preferred embodiment, W² is -NH- or -C(O)-.

In another preferred embodiment, W¹ and L¹ are null.

In another preferred embodiment, W¹ is -C(O)- and L¹ is C₁₋₁₀ alkylene (preferably C₁₋₃ alkylene).

In another preferred embodiment, CL is selected from Group 1, and Group 1 comprises the following groups: 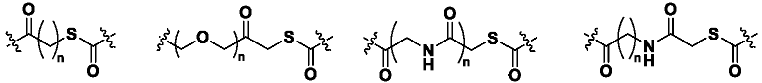 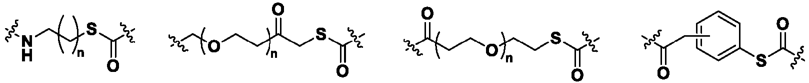 wherein n is an integer of 0 - 10, preferably, n=1, 2, 3, 4, or 5.

In another preferred embodiment, the conjugate is of formula III;

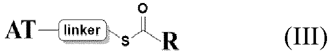

Wherein 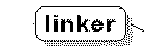 is -W²-L¹-W¹-A¹-; W², L¹, W¹, A¹, AT, and R are defined as above.

In another preferred embodiment, 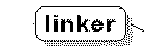 is -CO-L¹-W¹-A¹-.

In another preferred embodiment, R is a moiety comprising one or more active groups selected from the group consisting of: active group that can be further modified, and active group that biologically active and/or detectable.

In another preferred embodiment, the active group that can be further modified refers to the active group that can undergo bioorthogonal reaction (also called bioorthogonal group); preferably, the active group that can undergo bioorthogonal reaction comprises: one or more of azido (-N₃), aldehyde group (-CHO), thiol (-SH), alkynyl (such as C2-C6 alkynyl, ring strain alkynyl (BCN, DBCO, etc.), alkenyl (such as C2-C6 alkenyl), halogen (such as F, Cl, Br and I), tetrazinyl, nitrone group, hydroxyl amino, nitrile group, hydrazine group, ketone group, boronic acid residue, cyanobenzothiazolyl, allyl, phosphine group, maleimide group, disulfide group, thioester group, α-halogenated carbonyl, isonitrile group, sydnone group, seleno residue, conjugated dienyl, phosphate group, cycloalkynyl (such as C3-C7 cycloalkynyl) and cycloalkenyl (such as C3-C7 cycloalkenyl).

In another preferred embodiment, the active group that biologically active and/or detectable is any one or more of a small molecule drug, cytotoxin, or other functional molecules.

In another preferred embodiment, the small molecule drug is a radioactive therapeutic substance or a molecular imaging agent.

In another preferred embodiment, the active group that biologically active and/or detectable is derived from a compound selected from the group consisting of: maytansine, DM-1, DM-4, MMAE (Monomethyl auristatin E), MMAF, SN-38, Dxd, PBD and its analogs, amanitin, vincristine, vinblastine, vinorelbine, VP-16, camptothecin, paclitaxel, docetaxel, epothilone A, epothilone B, nocodazole, colchicine, estramustine, cemadotin, eleutherobin, fluorescent reagents, monosaccharides, disaccharides, oligosaccharides, polyethylene glycol (PEG), cytotoxin, immune agonists, or radioactive therapeutic substances, and molecular imaging agents.

In another preferred embodiment, R is -L₂-(L₃-B)ₒ, wherein subscript o is an integer from 1 to 10, L₂ and L₃ are each independently none or a linker fragment, and B is each independently the same or different active groups that can be further modified (such as bioorthogonal groups).

In another preferred embodiment, R is -L₂-(L₃-D)ₒ, wherein subscript o is an integer from 1 to 10, L₂ and L₃ are each independently none or a linker fragment, and D is each independently the same or different active groups that are biologically active and/or detectable (such as small molecule compounds).

In another preferred embodiment, o is 1.

In another preferred embodiment, o is 2, 3, 4, or 5.

In another preferred embodiment, L₂ and L₃ are each independently null or a linker fragment composed of one or more (such as 1-10, preferably 1, 2, 3, 4, 5, or 6) unit structures selected from the group consisting of CH₂OCH₂, C₁-C₄ alkylene, CO, NH, and amino acid residue.

In another preferred embodiment, L2 and L3 do not contain a linker group formed by an orthogonal reaction (for example, a linker group formed by an orthogonal reaction of B as defined above).

In another preferred embodiment, the conjugate is of formula III-A1, III-A2 or III-A3; 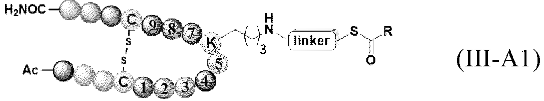 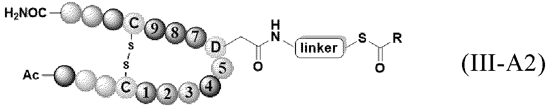 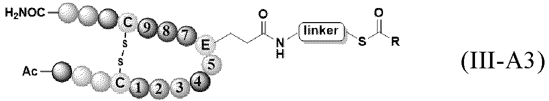 wherein,
the circles labeled with 1, 2, 3, 4, 5, 7, 8, and 9 are defined as Aa1 Aa2, Aa3, Aa4, Aa5, Aa7, Aa8, and Aa9 in formula II;
the end close to the circle labeled with 1 is the N-terminal of the polypeptide, and the end close to the circle labeled with 9 is the C-terminal of the polypeptide;
Ac is an N-terminal protecting group or absent;
each blank circle is defined as X and Y in formula II;
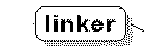 is -W²-L¹-W¹-A¹-; preferably, is -CO-L¹-W¹-A¹-;
Circle C represents a cysteine residue, and - S-S- represents a disulfide bond formed by the side chain SH of the cysteine residue;
Circle K is the main chain moiety of a lysine residue (the amino acid side chain has been shown); Circle D is the main chain moiety of an aspartic acid residue (D) (the amino acid side chain has been shown); Circle E is the main chain moiety of a glutamic acid residue (E) (the amino acid side chain has been shown);
R is as defined above.

In another preferred embodiment,
The blank circle (such as any one circle in 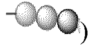 is each independently null (absent) or an amino acid residue (preferably, the amino acid residue is not a lysine residue or a cysteine residue);
the circles labeled with a number (i.e. Circle 1, Circle 2, Circle 3, Circle 4, Circle 5, Circle 7, Circle 8, and Circle 9) are each independently an amino acid residue (preferably, the amino acid residue is not a lysine residue or a cysteine residue);
the circle labeled with C (i.e. Circle C) represents a cysteine residue, and - S-S-represents a disulfide bond formed by the side chain SH of the cysteine residue;
the circle labeled with K (i.e. Circle K) is the main chain moiety of a lysine residue; the circle labeled with D (i.e. Circle D) is the main chain moiety of an aspartic acid residue (D); the circle labeled with E (i.e. Circle E) is the main chain moiety of a glutamic acid residue (E);
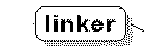 is -W²-L¹-W¹-A¹-; preferably, -CO-L¹-W¹-A¹- (i.e., connected to the Aa6 site of the above polypeptide chain of Formula II);
R is defined as above;
Ac is an N-terminal protecting group or absent (preferably, is acetyl), and is connected to the N-terminal of the polypeptide; and
-CONH₂ is amide, which is the C-terminal of the polypeptide;
when CL is connected to the Aa6 position of the cyclic peptide, combined with a preferred linker structure, any R group or substance can be modified onto the target protein (preferably, the target protein is an antibody).

In another preferred embodiment, the conjugate is a conjugate of formula III-A1-1, III-A2-1 or III-A3-1; 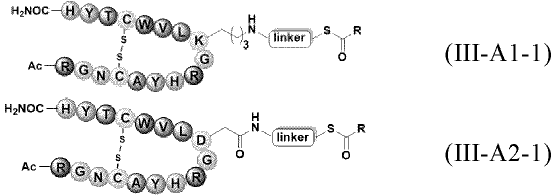 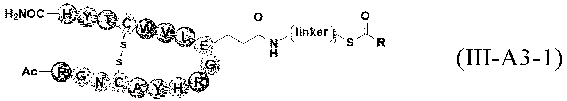 wherein,
Circle R is the N-terminal of the polypeptide, and Circle H is the C-terminal of the polypeptide;
Ac is an N-terminal protecting group or absent;
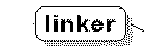 is -W² -L¹-W¹-A¹- (preferably, -CO-L¹-W¹-A¹-).
Circle R represents an arginine residue, G represents a glycine residue, N represents an asparagine residue, A represents an alanine residue, Y represents a tyrosine residue, H represents a histidine residue, L represents a leucine residue, V represents a valine residue, W represents a tryptophan residue, and T represents a threonine residue;
Circle C represents a cysteine residue, and - S-S- represents a disulfide bond formed by the side chain SH of the cysteine residue;
Circle K is the main chain moiety of a lysine residue; Circle D is the main chain moiety of an aspartic acid residue (D); Circle E is the main chain moiety of a glutamic acid residue (E);
R is as defined above.

In another preferred embodiment, Formula III-A1 is 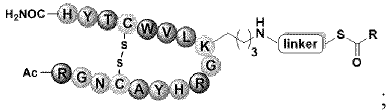 wherein except for the circles labeled with C and K, the circles labeled with letters represent the amino acid residues represented by the letters, respectively.

In another preferred embodiment, formula III-A2 is 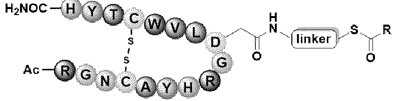 wherein except for the circles labeled with C and D, the circles labeled with letters represent the amino acid residues represented by the letters, respectively.

In another preferred embodiment, Formula III-A3 is 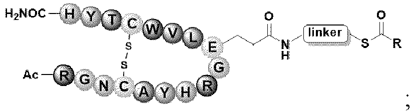 wherein except for the circles labeled with C and E, the circles labeled with letters represent the amino acid residues represented by the letters, respectively.

In another preferred embodiment the conjugate is of formula III-B 1 III-B2 or III-B3; 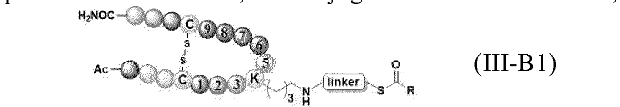 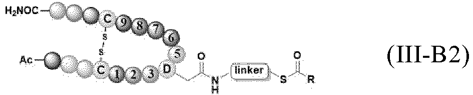 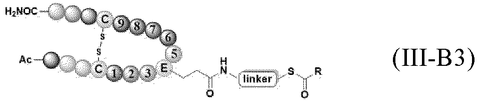 wherein
the circles labeled with 1, 2, 3, 5, 6, 7, 8, and 9 are defined as Aa1 Aa2, Aa3, Aa5, Aa6, Aa7, Aa8, and Aa9;
the end close to the circle labeled with 1 is the N-terminal of the polypeptide, and the end close to the circle labeled with 9 is the C-terminal of the polypeptide;
Ac is an N-terminal protecting group or absent;
each blank circle is defined as X and Y in formula II;
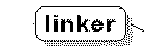 is -W²-L¹-W¹-A¹-; preferably, -CO-L¹-W¹-A¹-;
Circle C represents a cysteine residue, and - S-S- represents a disulfide bond formed by the side chain SH of the cysteine residue;
Circle K is the main chain moiety of a lysine residue (the amino acid side chain has been shown); Circle D is the main chain moiety of an aspartic acid residue (D) (the amino acid side chain has been shown); Circle E is the main chain moiety of a glutamic acid residue (E) (the amino acid side chain has been shown);
R is as defined above.

In another preferred embodiment,
the blank circle is each independently null (absent) or an amino acid residue (preferably, the amino acid residue is not a lysine residue or a cysteine residue);
the circles labeled with a number (i.e. Circle 1, Circle 2, Circle 3, Circle 5, Circle 6, Circle 7, Circle 8, and Circle 9) are each independently an amino acid residue (preferably, the amino acid residue is not a lysine residue or a cysteine residue);
the circle labeled with C (i.e. Circle C) represents a cysteine residue, and - S-S-represents a disulfide bond formed by the side chain SH of the cysteine residue;
the circle labeled with K (i.e. Circle K) is the main chain moiety of a lysine residue; the circle labeled with D (i.e. Circle D) is the main chain moiety of an aspartic acid residue (D); the circle labeled with E (i.e. Circle E) is the main chain moiety of a glutamic acid residue (E);
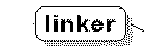 is -W²-L¹-W¹-A¹-; preferably, -CO-L¹-W¹-A¹- (i.e., connected to the Aa4 site of the above polypeptide chain of Formula II);
R is defined as above;
Ac is an N-terminal protecting group or absent (preferably, is acetyl), and is connected to the N-terminal of the polypeptide; and
-CONH₂ is amide, which is the C-terminal of the polypeptide.

In another preferred embodiment, the conjugate is of formula III-C1, III-C2 or III-C3; 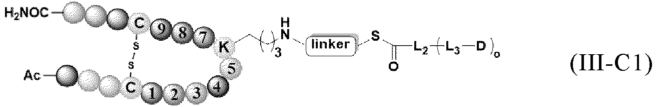 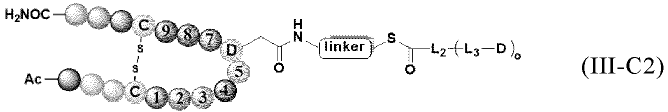 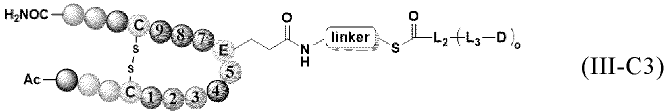 wherein
the circles labeled with 1, 2, 4, 3, 5, 7, 8, and 9 are defined as Aa1 Aa2, Aa3, Aa4, Aa5, Aa7, Aa8, and Aa9;
the end close to the circle labeled with 1 is the N-terminal of the polypeptide, and the end close to the circle labeled with 9 is the C-terminal of the polypeptide;
Ac is an N-terminal protecting group or absent; each blank circle is defined as X and Y in formula II;
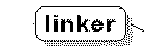 is -W²-L¹-W¹-A¹-; preferably, is -CO-L¹-W¹-A¹;
Circle C represents a cysteine residue, and - S-S- represents a disulfide bond formed by the side chain SH of the cysteine residue;
Circle K is the main chain moiety of a lysine residue; Circle D is the main chain moiety of an aspartic acid residue (D); Circle E is the main chain moiety of a glutamic acid residue (E).

In another preferred embodiment,
the blank circle is each independently null (absent) or an amino acid residue (preferably, the amino acid residue is not a lysine residue or a cysteine residue);
the circles labeled with a number (i.e. Circle 1, Circle 2, Circle 3, Circle 4, Circle 5, Circle 7, Circle 8, and Circle 9) are each independently an amino acid residue (preferably, the amino acid residue is not a lysine residue or a cysteine residue);
the circle labeled with C represents a cysteine residue, and - S-S- represents a disulfide bond formed by the side chain SH of the cysteine residue;
the circle labeled with K is the main chain moiety of a lysine residue; the circle labeled with D is the main chain moiety of an aspartic acid residue (D); the circle labeled with E is the main chain moiety of a glutamic acid residue (E);
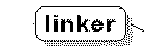 is -W²-L¹-W¹-A¹-; preferably, is -CO-L¹-W¹-A¹ (i.e., connected to the Aa6 site of the above polypeptide chain of Formula II);
L2, L3, subscript o, and R are as defined above;
Ac is an N-terminal protecting group or absent (preferably, is acetyl), and is connected to the N-terminal of the polypeptide; and
-CONH₂ is amide, which is the C-terminal of the polypeptide;
when CL is connected to the Aa6 position of the cyclic peptide, combined with a preferred linker structure, any L₂-(L₃-D)ₒ group or substance can be modified onto the target protein (preferably, the target protein is an antibody).

In another preferred embodiment, Formula III-C1 is 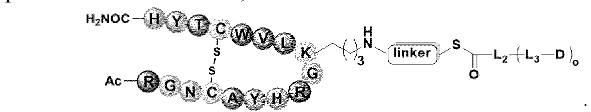 , wherein except for the circles labeled with C and K, the circles labeled with letters represent the amino acid residues represented by the letters, respectively.

In another preferred embodiment, Formula III-C2 is 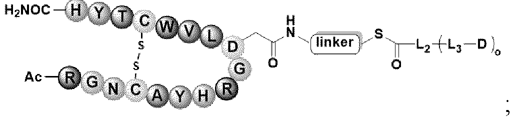 wherein except for the circles labeled with C and D, the circles labeled with letters represent the amino acid residues represented by the letters, respectively.

In another preferred embodiment, Formula III-C3 is 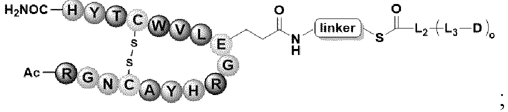 wherein except for the circles labeled with C and E, the circles labeled with letters represent the amino acid residues represented by the letters, respectively. In another preferred embodiment, in the above formulas, the circles labeled with 1, 2, 3, 4, 5, 6, 7, 8 and 9 are respectively defined as Aa1, Aa2, Aa3, Aa4, Aa5, Aa6, Aa7, Aa8 and Aa9 in Formula II, and the blank circles are defined as X and Y in Formula II.

In another preferred embodiment, the conjugate is selected from Table A1 and Table A2.

In the second aspect of the present invention, provided is an intermediate I, the intermediate I is of formula I-A 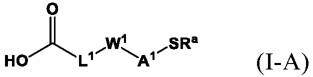 wherein R^{a} is H or a protecting group (such as trityl (Trt)); L¹, W¹ and A¹ are as defined above.

In the third aspect of the present invention, provided is an intermediate II, the intermediate is of formula I-B 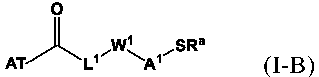 wherein R^{a} is H or a protecting group (such as trityl (Trt)) (preferably, R^{a} is H); AT, L¹, W¹ and A¹ are as defined above.

In another preferred embodiment, AT is a core sequence derived from an Fc binding polypeptide containing the sequence of II:
In the fourth aspect of the present invention, provided is a preparation method for preparing the the conjugate according to the first aspect, comprising steps of:
(1) reacting the intermediate of formula I-A with the core sequence of the Fc binding polypeptide containing the sequence of formula II, thereby forming an intermediate of formula I-B; and
(2) reacting the intermediate of formula I-B with an ester of R-COOH (active ester of a carboxylic acid compound) (such as 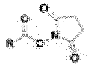 ), thereby obtaining the conjugate of formula I.

In the fifth aspect of of the present invention, provided is a method for site-specific modification of protein, comprising steps of:
(1) providing the conjugate according to the first aspect;
(2) contacting the target protein to be modified with the conjugate, and allowing the conjugate to react with the side chain amino group and/or the terminal amino group on the protein, thereby obtaining the modified target protein.

In another preferred embodiment, the modified target protein is modified by one or more of the R groups.

In another preferred embodiment, the modification is site-specific modification.

In another preferred embodiment, the amino includes the side chain amino group of lysine (K).

In another preferred embodiment, the modification can obtain the modified target protein through a one-step reaction.

In another preferred embodiment, the modified target protein is a protein modified with one or more active groups selected from the groups consisting of: active groups that are biologically active and/or detectable, and active groups that can be further modified.

In another preferred embodiment, the modified target protein is of formula IV

TP-(NHCO-R)ₚ (IV)

wherein R is as defined above, TP is the target protein moiety, and subscript p is 1-8.

In another preferred embodiment, p=2-4.

In another preferred embodiment, TP is the remaining moiety after the NH₂ in the amino acid residue containing the NH₂ group on its side chain in the target protein (preferably, lysine residue; more preferably, lysine residue located in the Fc region; most preferably, lysine residue at positions 246 to 248 in the Fc region) being connected with the -CO-R in the conjugate to form -NHCO-R.

In another preferred embodiment, the target protein is an antibody or a Fc fusion protein.

In another preferred embodiment, the target protein is an antibody, and the modified target protein is of formula V

Ab-(NHCO-R)ₚ (V).

wherein R and subscript p are as defined above, Ab is an antibody.

In another preferred embodiment, Ab is the remaining moiety after the NH₂ in the amino acid residue containing the NH₂ group on its side chain in the antibody (preferably, lysine residue; more preferably, lysine residue located in the Fc region; most preferably, lysine residue at positions 246 to 248 in the Fc region) being connected with the -CO-R in the conjugate to form -NHCO-R.

In another preferred embodiment, the target protein has a fragment or sequence that has an affinity for the complex of formula I.

In another preferred embodiment, the antibody or Fc structure is derived from human IgG.

In another preferred embodiment, the modified position of the target protein is the Fc region.

In another preferred embodiment, the modified position of the target protein is a region containing 80% homologous sequence of Fc of human IgG.

In another preferred embodiment, the Fc original sequence of human IgG is shown as SEQ ID No. 1:

DKTHTCPPCPAPELLGGPSVFLFPPKPKDTLMISRTPEVTCVVVDVSHEDPEVKF

NWYVDGVEVHNAKTKPREEQYNSTYRVVSVLTVLHQDWLNGKEYKCKVSNK
ALPAPIEKTISKAKGQPREPQVYTLPPSREEMTKNQVSLTCLVKGFYPSDIAVEW
ESNGQPENNYKTTPPVLDSDGSFFLYSKLTVDKSRWQQGNVFSCSVMHEALHN
HYTQKSLSLSPGK (SEQ ID No.1).

In another preferred embodiment, the modified position of the target protein is the target region formed by the amino acid residues at positions 246 to 248 in the Fc region of human IgG.

In another preferred embodiment, the covalently modified region where interacts with the conjugate of formula I is a region consisting of 1-10 consecutive amino acid residues, and the region includes a target region formed by the amino acid residues at positions 246 to 248 in the Fc region of human IgG.

In another preferred embodiment, the target region is composed of a lysine residue at position 246 or 248 in the human IgG Fc region.

In another preferred embodiment, the region selectivity of the method is ≥ 90%.

In another preferred embodiment, the modification site is the lysine residue at position 248 in the Fc region of human IgG.

In the sixth aspect of the present invention, provided is a site-specificly modified protein of formula IV,

TP-(NHCO-R)ₚ (IV)

wherein TP, R and subscript p are as defined above.

In another preferred embodiment, R is -L₂-(L₃-D)ₒ; wherein L₂, L₃, D, and subscript o are as defined above.

In another preferred embodiment, R is -L₂-(L₃- B)ₒ; wherein L₂, L₃, B, and subscript o are as defined above.

In another preferred embodiment, R is -L₂-(L₃-B'-L₄-D)ₒ; wherein L₂, L₃, B, and subscript o are as defined above; B' is a linker group formed by orthogonal reaction between B and L4'-D, L4 is a linker fragment, L4' is a group having an active group that can react orthogonally with B and can form L4 after the reaction.

In another preferred embodiment, the site specificly modified protein is an antibody drug conjugate, i.e., TP is an antibody and R is -L₂-(L₃-D)ₒ or R is -L₂-(L₃-B'-L₄-D)ₒ.

In another preferred embodiment, TP is an antibody, and the site- specificly modified protein is of formula V;

Ab-(NHCO-R)ₚ (V)

wherein Ab is an antibody, R and subscript p are as defined above.

In another preferred embodiment, the site- specificly modified protein is of formula IV-A;

TP-(NHCO-L₂-(L₃-D)₆)ₚ (IV-A)

wherein TP, L₂, L₃, D, subscript o, and subscript p are as defined above.

In another preferred embodiment, the site- specificly modified protein is of formula V-A;

Ab-(NHCO-L₂-(L₃-D)ₒ)ₚ (V-A)

wherein Ab, L₂, L₃, D, subscript o, and subscript p are as defined above.

In another preferred embodiment, the antibody-drug conjugate is selected from the group consisting of: ADC-1, ADC-2, ADC-3, ADC-4, ADC-5, ADC-6, ADC-7, ADC-8, ADC-9, ADC-10, ADC-11, ADC-12, ADC13, ADC-14, ADC-15, ADC-16, ADC-17, ADC-18, ADC-19, ADC-20 and ADC-21.

In the seventh aspect of the present invention, provided is a use of the conjugate according the first aspect for site-specific modification of proteins.

In the eighth aspect of the present invention, provided is use of the conjugate according the first aspect for preparing antibody drug conjugates (ADC).

In another preferred embodiment, the use does not rely on bioorthogonal reactions, and the conjugate can directly modify the drug molecule that needs to be modified to the target protein onto the antibody in a site-specific manner through a one-step reaction.

In the ninth aspect of the present invention, provided is a site selectively modified antibody of formula V

Ab-(NHCO-R)ₚ (V)

wherein Ab is an antibody, R and subscript p are as defined above.

In the tenth aspect of the present invention, provided is an antibody-drug conjugate (ADC), and the antibody-drug conjugate is of formula V-A:

Ab-(NHCO-L₂-(L₃-D)ₒ)ₚ (V-A)

wherein Ab, L₂, L₃, D, subscript o, and subscript p are as defined above.

In another preferred embodiment, the antibody-drug conjugate is a site- specific antibody-drug conjugate that is prepared in one step without relying on bioorthogonal reaction, and in which the antibody is site- specificly modified.

In another preferred embodiment, Ab is an antibody, subscript p is 1~8 (preferably, p=2-4), o is an integer from 1 to 10, L₂ and L₃ are linker fragments, D is a small molecule drug; when o is 2-10, D can be the same molecule drug or can independently be different small molecule drugs.

In another preferred embodiment, in the site-specific antibody-drug conjugate, D (such as a small molecule drug) is site-specificly coupled to the antibody constant region (Fc region).

In another preferred embodiment, in the site- specific antibody-drug conjugate, D (such as a small molecule drug) is site-specificly coupled to the region consisting of amino acid residues at positions 246-248 in the human IgG Fc region.

In another preferred embodiment, the antibody-drug conjugate is selected from the group consisting of: ADC-1, ADC-2, ADC-3, ADC-4, ADC-5, ADC-6, ADC-7, ADC-8, ADC-9, ADC-10, ADC-11, ADC-12, ADC13, ADC-14, ADC-15, ADC-16, ADC-17, ADC-18, ADC-19, ADC-20 and ADC-21. In another preferred embodiment, the antibody-drug conjugate is selected from the group consisting of: ADC-5, ADC-6, ADC-7, ADC-8, ADC-9, ADC-10, ADC-11, ADC-12, ADC13, ADC-14, ADC-15, ADC-16, ADC-17, ADC-18, ADC-19, ADC-20 and ADC-21.

In another preferred embodiment, the preparation method for above-mentioned site-specific ADC is: after incubating the natural antibody with the affinity fragment-cleavable linker fragment-functional fragment complex of formulas (III, III-A1-3, III-B1-3, III-C1-3), the carbonyl group contained in the functional fragment directly covalently forms an amide bond with the lysine residue in the Fc region of the antibody, and the site-specific ADC compound is prepared in a one-step reaction without relying on a bioorthogonal reaction.

In the eleventh aspect of the present invention, provided is a site-specific, bivalent or multivalent bioorthogonal group-modified antibody of formula V-B, wherein

Ab-(NHCO-L₂-(L₃-B)ₒ)ₚ (V-B)

wherein Ab, L₂, L₃, B, subscript o, and subscript p are as defined above.

In another preferred embodiment, Ab is an antibody, subscript p is 1~8 (preferably, p=2-4), o is an integer from 2 to 10, L₂ and L₃ are linker fragments;
B is bioorthogonal group, multiple B may be the same or independently different, and are preferably selected from the group consisting of: azide residues, aldehyde residues, thiol residues, alkyne residues, olefin residues, halogen residues, tetrazine residues, nitrone residues, hydroxylamine residues, nitrile residues, hydrazine residues, ketone residues, boronic acid residues, cyanobenzothiazole residues, allyl residues, phosphine residues, maleimide residues, disulfide residues, thioester residues, α-halogenated carbonyl residues, isonitrile residues, sydnone residues, selenium residues, conjugated diene residues, phosphoric acid residues, cycloalkyne residues and cycloalkene residues.

The above bioorthogonal groups are site-specifically coupled to the antibody constant region (Fc region);
the above bioorthogonal groups are site-specifically coupled to the region consisting of amino acid residues at positions 246-248 in the human IgG Fc region.

In the twelfth aspect of the present invention, provided is a site-specific, bivalent or multivalent drug-modified antibody of formula V- C, wherein

Ab-(NHCO-L₂-(L₃-B'-L₄-D)ₒ)ₚ (V-C)

wherein L₂, L₃, B, and subscript o are as defined above; and B' is a linker group formed by orthogonal reaction between B and L4'-D, L4 is a linker fragment, L4' is a group having an active group that can react orthogonally with B and can form L4 after the reaction.

In another preferred embodiment, Ab is an antibody, subscript p is 1~8 (preferably, p=2, -4, more preferably, p=2), o is an integer from 2 to 10, L₂ and L₃ are linker fragments, B' is a linker fragment generated by the reaction between the drug fragment and the above-mentioned bioorthogonal group B, B' is the same or different, L4 is a linker connecting B' and drug D, D is as defined above, and the structures of D herein may be the same or different.

The above bioorthogonal groups are site-specifically coupled to the antibody constant region (Fc region);
the above bioorthogonal groups are site-specifically coupled to the region consisting of amino acid residues at positions 246-248 in the human IgG Fc region.

It should be understood that within the scope of the present invention, the above technical features of the present invention and the technical features specifically described in the following (eg, embodiments) can be combined with each other, thereby forming a new or preferred technical solution. Due to space limitations, it will not be repeated herein.

### DESCRIPTION OF THE DRAWINGS

FIGs. 1a-e show each characterization result of biotinylated trastuzumab Ab-1 prepared in Example 47. Wherein FIGs. 1a, 1b, and 1c show the ESI-TOFMS determination result, FIG. 1d shows MS spectrum of a peptide fragment of trastuzumab digested with trypsin that contains the modified site on lysine and consists of 33 amino acid residues (THTCPPCPAPELLGGPSVFLFPPKPK(C₁₀H₁₄N₂O₂S)DTLMISR), and FIG. 1e shows that through Mascot 2.3 software analysis, the modification was determined to occur at the lysine residue at position 251 with high selectivity.
FIGs. 2a-c show each characterization result of azide group coupled trastuzumab Ab-2 prepared in Example 48. Wherein FIGs. 2a, 2b, and 2c show the ESI-TOFMS determination results.
FIG. 3 shows the ESI-TOFMS determination results of Trastuzumab Ab-3 before and after being coupled with biofluorescein FITC in Example 49.
FIG. 4 shows the ESI-TOFMS determination results of Trastuzumab Ab-4 before and after being coupled with bioorthogonal group cyclopropene in Example 50.
FIG. 5 shows the ESI-TOFMS determination results of Rituximab Ab-5 before and after being coupled with bioorthogonal group DBCO in Example 51.
FIG. 6 shows the ESI-TOFMS determination results of Rituximab Ab-6 before and after being coupled with bioorthogonal group DBCO in Example 52.
FIGs. 7a-e show each characterization result of ADC-1 prepared in Example 53. Wherein FIGs. 7a, 7b, and 7c show the ESI-TOFMS determination results, FIG. 7d shows the analysis results of hydrophobic interaction chromatography, and FIG. 7e shows the analysis results of molecular-exclusion chromatography.
FIGs. 8a-e show each characterization result of ADC-2 prepared in Example 54. Wherein FIGs. 8a, 8b, and 8c show the ESI-TOFMS determination results, FIG. 8d shows the analysis results of hydrophobic interaction chromatography, and FIG. 8e shows the analysis results of molecular-exclusion chromatography.
FIGs. 9a-e show each characterization result of ADC-3 prepared in Example 55. Wherein FIGs. 9a, 9b, and 9c show the ESI-TOFMS determination results, FIG. 9d shows the analysis results of hydrophobic interaction chromatography, and FIG. 9e shows the analysis results of molecular-exclusion chromatography.
FIG. 10 shows the ESI-TOFMS determination result of ADC-4 prepared in Example 56.
FIGs. 11a-e show each characterization result of ADC-5 prepared in Example 57. Wherein FIGs. 11a, 11b, and 11c show the ESI-TOFMS determination results, FIG. 11d shows the analysis results of hydrophobic interaction chromatography, and FIG. 11e shows the analysis results of molecular-exclusion chromatography.
FIGs. 12a-c show each characterization result of ADC-12 prepared in Example 58. Wherein FIGs. 12a, 12b, and 12c show the ESI-TOFMS determination results, FIG. 12d shows the analysis results of hydrophobic interaction chromatography, and FIG. 12e shows the analysis results of molecular-exclusion chromatography.
FIGs. 13a-c show each characterization result of ADC-7 prepared in Example 59. Wherein FIGs. 13a, 13b, and 13c show the ESI-TOFMS determination result, FIG. 13d shows the analysis results of hydrophobic interaction chromatography, and FIG. 13e shows the analysis results of molecular-exclusion chromatography.
FIGs. 14a-c show each characterization result of ADC-8 prepared in Example 60. Wherein FIGs. 14a, 14b, and 14c show the ESI-TOFMS determination results, FIG. 14d shows the analysis results of hydrophobic interaction chromatography, and FIG. 14e shows the analysis results of molecular-exclusion chromatography.
FIG. 15 shows the ESI-TOFMS determination result of Rituximab before and after being coupled with Drug Linker **D6** in Example 61.
FIG. 16 shows the ESI-TOFMS determination result of Pertuzumab before and after being coupled with Drug Linker **D6** in Example 62.
FIG. 17 shows the ESI-TOFMS determination result of Bevacizumab before and after being coupled with Drug Linker **D6** in Example 63.
FIG. 18 shows the ESI-TOFMS determination result of Toripalimab ADC-12 before and after being coupled with Drug Linker **D6** in Example 64.
FIG. 19 shows the ESI-TOFMS determination result of Panituzumab before and after being coupled with Drug Linker **D6** in Example 65.
FIG. 20 shows the ESI-TOFMS determination result of Nivolumab before and after being coupled with Drug Linker **D6** in Example 66.
FIG. 21a shows the ESI-TOFMS determination results of raw material Trastuzumab and the prepared ADC-15 in Example 67, FIG. 21b shows the analysis results of hydrophobic interaction chromatography.
FIG. 22a shows the ESI-TOFMS determination results of raw material Trastuzumab and the prepared ADC-16 in Example 68, FIG. 22b shows the analysis results of hydrophobic interaction chromatography.
FIG. 23a shows the ESI-TOFMS determination results of raw material Trastuzumab and the prepared ADC-17 in Example 69, FIG. 23b shows the analysis results of hydrophobic interaction chromatography.
FIG. 24a shows the ESI-TOFMS determination results of raw material Trastuzumab and the prepared ADC-18 in Example 70, FIG. 24b shows the analysis results of hydrophobic interaction chromatography.
FIG. 25a shows the ESI-TOFMS determination results of raw material Trastuzumab and the prepared ADC-19 in Example 71, FIG. 25b shows the analysis results of hydrophobic interaction chromatography.
FIG. 26 shows the ESI-TOFMS determination result of raw material Trastuzumab and the prepared ADC-20 in Example 72,
FIG. 27 shows the ESI-TOFMS determination result of raw material Trastuzumab and the prepared ADC-21 in Example 73,
FIG. 28 shows the cell activity and cytotoxicity results of the ADC prepared in the examples of the present invention.

### EMBODIMENTS FOR CARRYING OUT THE INVENTION

After extensive and in-depth research, the present inventors unexpectedly discovered a conjugate with unique structures which has a cleavable fragment containing the bivalent fragment shown in 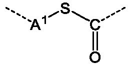 and an affinity moiety (AT). The conjugate of the present invention is very suitable for direct modification of proteins, especially the Fc segment of antibodies or Fc fusion proteins). By using the conjugate of the present invention, there is no need to pre-modify the active groups on the side chains of the protein before modification, and a direct reaction (such as a single-step direct reaction) can be performed to obtain the desired product modified with the R group (such as ADC, etc.). In particular, when a cyclic peptide with a specific sequence is used as the affinity moiety and the cleavable fragment is attached to the specific position of the cyclic peptide, the conjugate of the present invention can perform site-specific modification with extremely high efficiency. The inventor has completed the present invention on this basis.

### TERMS

Unless otherwise indicated, all abbreviations herein have the meanings commonly known to those skilled in the art.

As used herein, the term "halogen" refers to F, Cl, Br and I. More preferably, the halogen atom is selected from F, Cl, and Br.

Unless otherwise stated, the term "alkyl", by itself or as a part of another substituent, means a straight or branched chain hydrocarbon radical having a designated number of carbon atoms (i.e. C₁₋₆ means 1-6 carbons). Examples of alkyl groups include methyl, ethyl, *n*-propyl, isopropyl, *n*-butyl, *t*-butyl, *iso*-butyl, *sec*-butyl, *n*-pentyl, *n*-hexyl, *n*-heptyl, *n*-octyl, and the like.

As used herein, the term "alkylene" by itself or as a part of another substituent means a divalent radical derived from an alkane, as exemplified by -CH₂CH₂CH₂CH₂-. Alkyl (or alkylene) typically has 1-10 carbon atoms (i.e. C₁₋₁₀ alkylene). Examples of alkylene include, but are not limited to, methylene, ethylene.

The term "aryl" means, unless otherwise stated, a polyunsaturated, typically aromatic, hydrocarbon group which can be a single ring or multiple rings (up to three rings) which are fused together or linked covalently. Generally, aryl has 6 to 10 ring atoms. The term "heteroaryl" refers to aryl groups (or rings) that contain from one to five heteroatoms selected from N, O, and S, wherein the nitrogen and sulfur atoms are optionally oxidized, and the nitrogen atom(s) are optionally quaternized. Generally, the heteroaryl has 5 to 10 ring atoms, i.e. 5-10 membered heteroaryl, preferably, has 5 to 6 ring atoms, i.e. 5-6 membered heteroaryl, and contains 1, 2, 3, or 4 heteroatoms. A heteroaryl group can attach to the remainder of the molecule through a heteroatom. Non-limiting examples of aryl include phenyl, naphthyl, and biphenyl, and non-limiting examples of heteroaryl include pyridyl and the like.

As used herein, a bond represented by dashed lines ( 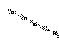 ) represent the site where the group (or fragment or moiety) is connected to the rest of the parts.

As used herein, unless otherwise specified, said substituted means that one or more H atoms in the group are substituted with substituents selected from the group consisting of halogen, C₁₋₆ alkyl, and C₁₋₆ haloalkyl.

As used in this article, the term "bioorthogonal group" refers to a group containing a functional group that can be used for bioorthogonal reactions, such as azide (-N₃), cyclooctyne, 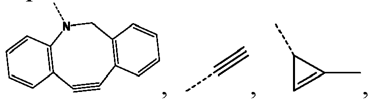 aldehyde, keto carbonyl, tetrazine, trans-cyclooctene, thiol, halogen, hydrazine, hydroxylamine, and the like.

As used herein, the term "amino acid residue" refers to a group formed by the removal of an H from -NH₂ at the N-terminal and the removal of -OH from -COOH at the C-terminal of the an amino acid. Generally, the chain segment of an amino acid (residue) containing the N-terminal and the C-terminal is called the main chain, and the part that determines the specific type of an amino acid is called the side chain. Unless otherwise defined, as used herein, amino acids include natural or non-natural amino acids, including D and/or L-type amino acids. Examples of amino acids include, but are not limited to, Ala (A), Arg (R), Asn (N), Asp (D), Cys (C), Gln (Q), Glu (E), Gly (G), His (H), Ile (I), Leu (L), Lys(K), Met (M), Phe (F), Pro (P), Ser (S), Thr (T), Trp (W), Tyr (Y), Val (V). Preferably, as used herein, the amino acid is an amino acid selected from the group consisting of: L-glycine (L-Gly), L-alanine (L-Ala), β-alanine (β-Ala), L-glutamic acid (L-Glu), L-aspartic acid (L-Asp), L-histidine (L-His), L-arginine (L-Arg), L-lysine (L-Lys), L-valine (L-Val), L-serine (L-Ser), L-threonine (L-Thr); in addition, when an amino acid has two or more amino groups and/or two or more carboxyl groups, the term also comprises groups formed by the removal of one H from -NH₂ and -OH from -COOH that are not on the same carbon atom, such as the divalent group -C(O)-(CH₂)₂-C(COOH)-NH- formed by the removal of one H from -NH₂ and non-α-position -COOH respectively of an glutamic acid.

As used herein, term "antibody" or "immunoglobulin" is a heterotetrameric glycoprotein of about 150,000 daltons with the same structural characteristics, which consists of two identical light chains (L) and two identical heavy chains (H). Each light chain is connected to the heavy chain by a covalent disulfide bond, and the number of disulfide bonds between the same type of heavy chains of different immunoglobulin isotypes are different. Each heavy and light chain also has regularly spaced intrachain disulfide bonds. Each heavy chain has a variable region (VH) at one end, followed by multiple constant regions. There are a variable region (VL) at one end of each light chain and a constant region at the other end. The constant region of the light chain is relative to the first constant region of the heavy chain, and the variable region of the light chain is relative to the variable region of the heavy chain. Special amino acid residues form an interface between the variable regions of the light chain and the heavy chain.

As used herein, term "variable" means that certain parts of the variable region of the antibody are different in sequence, which forms the binding and specificity of various specific antibodies to specific antigens. However, variabilities are not evenly distributed throughout the variable regions of antibodies. It is concentrated in three fragments that are called complementarity determining regions (CDR) or hypervariable regions in the variable regions of light chain and heavy chain. More conservative parts of the variable region are called the framework region (FR). The variable regions of the natural heavy and light chains each contain four FR regions, which are in a roughly β-folded conformation and are linked by three CDRs that form a linking loop, which in some cases can form a partially folded structure. The CDRs in each chain are closely placed together through the FR regions and form the antigen binding site of the antibody together with the CDRs in other chain. Constant regions do not directly participate in the binding of antibodies to antigens, but they exhibit different effector functions, such as participating in antibody-dependent cytotoxicity of antibodies.

The "light chains" of vertebrate antibodies (immunoglobulins) can be classified in one of two distinct categories (called κ and λ) based on the amino acid sequence of constant regions thereof. According to the amino acid sequence of the constant region in heavy chain thereof, immunoglobulins can be classified into different types. There are five main classes of immunoglobulins: IgA, IgD, IgE, IgG and IgM, some of which can be further classified into subclasses (isotypes), such as IgG1, IgG2, IgG3, IgG4, IgA and IgA2. The constant regions in heavy chains corresponding to different classes of immunoglobulins are called α, δ, ε, γ, andµ respectively. The subunit structures and three-dimensional configurations of different classes of immunoglobulins are well known to those skilled in the art.

Generally, the antigen-binding properties of antibodies can be described by 3 specific regions located in the variable regions of the heavy and light chains, called variable regions (CDR), which are divided into 4 framework regions (FRs). The amino acid sequences of the 4 FRs are relatively conservative and do not directly participate in the binding reaction. These CDRs form a circular structure, and close to each other in space structure through the β-pleated sheet formed by the FRs in between, and the CDRs on the heavy chain and the corresponding CDRs on the light chain constitute the antigen binding site of the antibody. It can be determined by comparing the amino acid sequences of antibodies of the same type which amino acids constitute the FR or CDR regions.

### Polypeptide

In the present invention, the term "Fc binding polypeptide" refers to a peptide that has the ability to reversibly bind to the target protein, especially the Fc region of an antibody; preferably, has a core sequence of formula II (i.e., (Xₛ₁)ₜ₁-Cys-Aa1-Aa2-Aa3-Aa4-Aa5-Aa6-Aa7-Aa8-Aa9-Cys-(Yₛ₂)ₜ₂). Furthermore, the term also encompasses variant forms of the Fc binding polypeptide. These variations include (but are not limited to): deletion, insertion and/or substitution of 1-5 (usually 1-4, preferably 1-3, more preferably 1-2, and most preferably 1) amino acids, and the addition or deletion of one or several (usually within 5, preferably within 3, and more preferably within 2) amino acids at the C-terminal and/or N-terminal. For example, in the art, the substitution of amino acids with amino acids having similar or close properties usually does not change the function of the protein. For example, the addition or deletion of one or several amino acids at the C-terminal and/or N-terminal generally does not change the structure and function of the protein as well. Furthermore, the term also encompasses both monomeric and multimeric forms of the polypeptides of the present invention. The term also includes linear as well as non-linear polypeptides (eg, cyclic peptides).

A preferred class of active derivatives refers to, compared with the amino acid sequence of formula I, polypeptides formed by replacing at most 5, preferably at most 3, more preferably at most 2, and most preferably 1 amino acid with amino acids having similar or close properties. These conservative variant polypeptides are preferably produced by performing amino acid substitutions according to Table 1.

**Table 1**

| The initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The invention also provides analogs of the polypeptides of the present invention. The difference between these analogs and the natural polypeptide of the present invention may be the difference in amino acid sequence, or the difference in modification form that does not affect the sequence, or both. Analogs also include analogs with residues different from natural L-amino acids (such as D-amino acids), and analogs with non-natural or synthetic amino acids (such as β, γ-amino acids). It should be understood that the polypeptides of the present invention are not limited to the representative polypeptides exemplified above.

Modifications (usually without altering the primary structure) include chemical derivatization of the polypeptide in vivo or in vitro such as acetylation or carboxylation. Modifications also include glycosylation, such as those polypeptides produced by modification of glycosylation during polypeptide synthesis and processing or during further processing steps. Such modifications can be achieved by exposing the polypeptide to a glycosylation enzyme (such as a mammalian glycosylase or deglycosylase). Modified forms also include sequences with phosphorylated amino acid residues (such as phosphotyrosine, phosphoserine, phosphothreonine). Also included are polypeptides modified to improve their anti-proteolytic properties or optimize solubility.

### Cleavable Fragment Directed By Affinity Fragment

In one aspect of the present invention, provided is a cleavable fragment directed by affinity fragment (also referred to as the conjugate of the present invention) or a derivative thereof (such as a salt thereof), represented by formula I below:

AT-CL-R (I)

As an acyl transfer reagent, the complex can directly couple the R group to the protein and form a new covalent bond with the amino acid at a specific site.

In some embodiments, AT (affinity tag) is an affinity substance for a protein; CL (cleavable linker) is a cleavable fragment containing a auto-hydrolysis-reactive cleavage fragment; and R (reagent) is a compound.

In another preferred embodiments, AT is an affinity fragment of a protein, which can be a small molecule compound that forms a reversible covalent bond with the protein, or a protein or polypeptide structure with affinity. In particular, in terms of modification of antibodies, AT can be a small molecule compound that forms a reversible covalent bond with the antibody, or a protein or a polypeptide structure such as an Fc binding peptide that has an affinity for the antibody, peptide fragment structures derived from ProteinA or ProteinG that have the ability to bind to Fc, etc.

In another preferred embodiments, AT can be Fc-III peptide (DCAWHLGELVWCT (SEQ ID No.2)), other Fc binding peptide (GPDCAYHRGELVWCTFH (SEQ ID No.3), RGNCAYHRGQLVWCTYH (SEQ No.4), CDCAWHLGELVWCTC(SEQ ID No.5)), and the like, the core structure sequence thereof is as shown in (II) below:

(X₀₋₃)ₜ₁-Cys-Aa1-Aa2-Aa3-Aa4-Aa5-Aa6-Aa7-Aa8-Aa9- Cys -(Y₀₋₃)ₜ₂ (II)

wherein C is a cysteine residue, X₀₋₃ and Y₀₋₃ are any amino acids except lysine and cysteine, and may be 1-3 consecutive identical or different amino acid residues, and m and n are independently a number of 0-10. Aa1-Aa3 and Aa5, and Aa7-Aa9 are any amino acids other than cysteine and lysine. Aa4 or Aa6 is independently a lysine residue, i.e., when Aa4 is a lysine, Aa6 is any amino acid other than cysteine and lysine, and when Aa6 is a lysine, Aa4 is any amino acid other than cysteine and lysine.

Preferably, the affinity fragment (AT) is or derived from a cyclic peptide fragment that has an affinity for antibody Fc, and the preferred structure is: 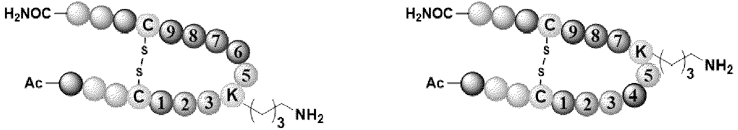 wherein unmarked or number-labeled circles represent any amino acid other than cysteine and lysine, and unmarked circles can also be absent, C represents a cysteine and -S-S- represents the -S-S- bond formed by the -SH groups of the two cysteines, and K represents the main chain of lysine, and its side chain structure is depicted.

More preferably, the affinity fragment (AT) and a part of the cleavable fragment (CL) (i.e., AT-W²-L¹-W¹-A¹-S-) are derived from an Fc binding peptide containing a thiol group, and the preferred modified structure thereof is any one of the following:

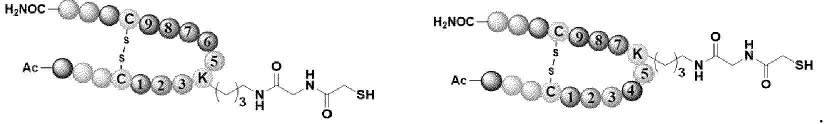

In other preferred embodiments, AT may also be a binding peptide having an affinity with antibody Fab, such as trastuzumab Fab binding peptide.

In some embodiments, AT may also be a small molecule fragment having a reversible covalent interaction with a protein, for example, o-hydroxybenzaldehyde, o-acetylphenylboronic acid, etc., as shown below:

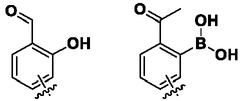

In some embodiments, CL is a cleavable fragment that can form a new chemical bond under the attack of a nucleophile reagent, and covalently connects the R fragment to a specific site of the protein, and at the same time the fragment breaks to release the affinity fragment AT. Preferably, CL is as shown below, and the CL fragment can be an acyl transfer reagent such as a thioester fragment, a maleimide ester fragment, an ester fragment, etc.: 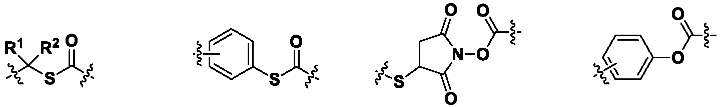 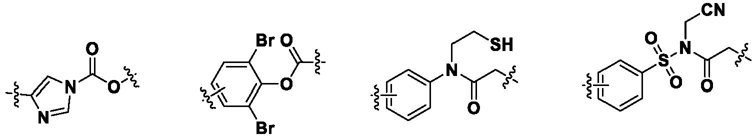 wherein, R1 and R2 are independently hydrogen atoms, aliphatic or aromatic fragments such as methyl and ethyl, or R1 and R2 form a cyclic alkane or alkene structure.

More preferably, CL is of any of the following: 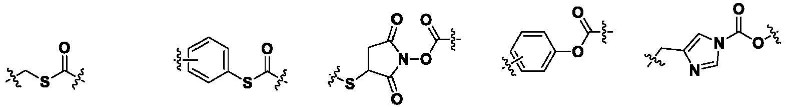 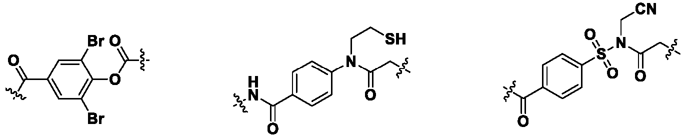 wherein the carbonyl group of the cleavable fragment is connected to the R fragment, and the S or O part of the cleavable fragment is coupled to the affinity fragment.

In some preferred embodiments, the CL fragment is an acyl transfer reagent using thioester as the core, and the structure thereof is shown in the below formula (III): 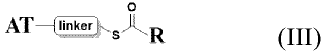 wherein the structure of the linker is a methylene structure, an amino acid, a polyglycine, a polyethanol (PEG) structure, etc of different length. More preferably, the structure of the linker is as follows: 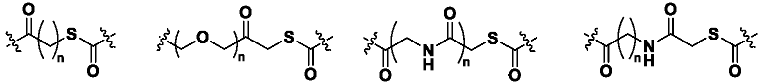 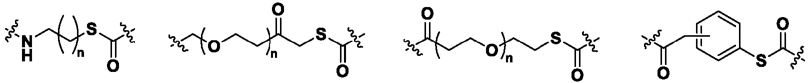 wherein n is an integer of 1 to 10.

In some embodiments, R is derived from a compound to be modified onto a protein, comprising active group that can be further modified such as bioorthogonal group, or contains or contains or derives from biotins, fluorescent agents, small molecule drugs, mRNA, polypeptide compounds, proteins, and the like. The source compound of R itself contains a carboxylic acid fragment or can be derived to obtain a carboxylic acid structure, and the carboxylic acid forms a -CO- structure in the CL cleavable fragment with thiol, maleimide, phenol, etc., i.e., an acyl transfer reagent.

In another preferred embodiment, -CO-R is selected from Group 2 (wherein -CO- is the -CO- in CL), and Group 2 includes groups as shown below: 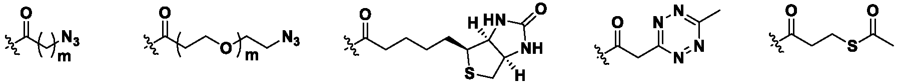 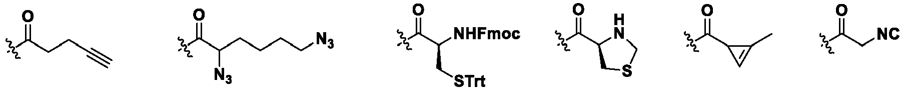 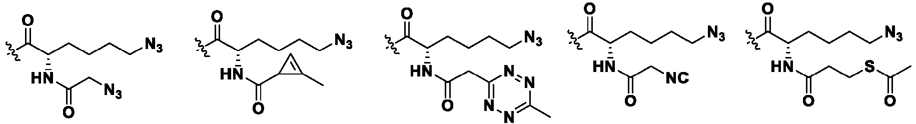 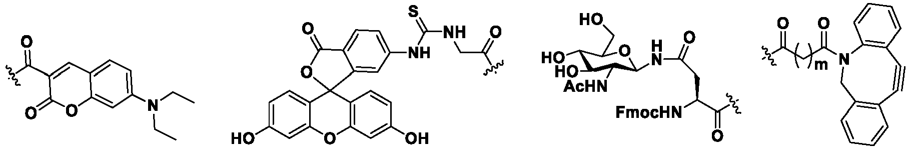 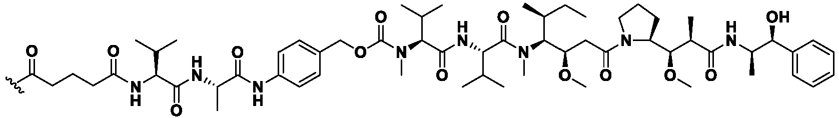 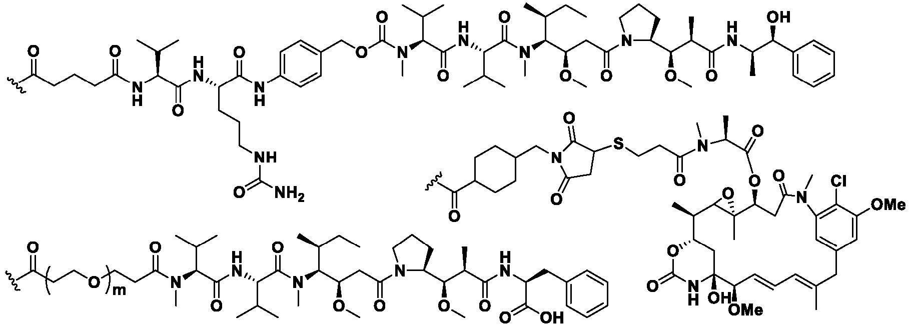 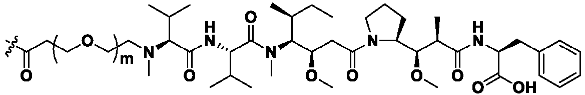 wherein, m is 0 or any positive integer; preferably a positive integer of 1 to 20. In another preferred embodiment, L₃-D is selected from the group consisting of: 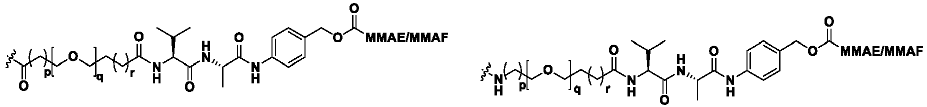 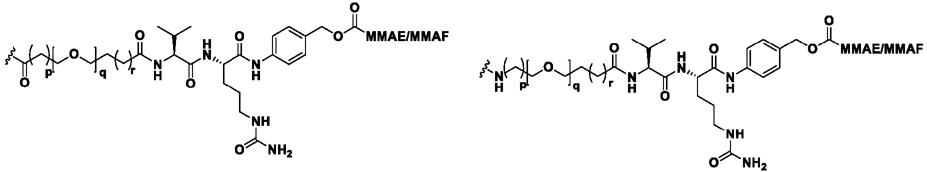 wherein
p, q and r are 0 or any positive integer; preferably are 0 or a positive integer of 1 to 20; more preferably, p and r are each independently 0, 1, 2 or 3, and q is 1, 2, 3, 4, 5 or 6;
MMAE/MMAF refers to MMAE or MMAF, and the structures of MMAE and MMAF are as follows:

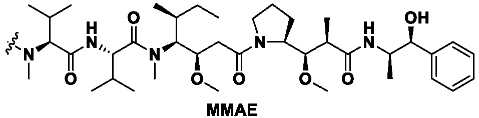

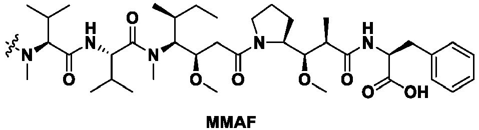

In some embodiments, the cleavable fragment directed by affinity fragment of formula III is as shown in any of the following formulas: 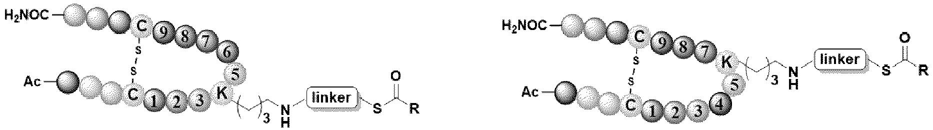 wherein R and linker are as defined above, circles 1, 2, 3, 4, 5, 6, 7, 8 and 9 refer to Aa1, Aa2, Aa3, Aa4, Aa5, Aa6, Aa7, Aa8, and Aa9 as defined above, respectively, circle C refers to Cys, and circle K refers to the main chain moiety of the lysine residue (i.e., the -NH-C-CO- moiety of the lysine residue).

In some embodiments, AT, CL and R are as defined in the first aspect.

In some embodiments, AT, CL and R are each independently the corresponding group in the conjugates shown in Table A1 and Table A2 or in each specific compound in the Examples.

In some specific embodiments, the conjugate (or affinity fragment-directed cleavable fragment) is selected from the compounds shown in Table A1 and Table A2 (the side chain structure of K in the cyclic peptide (i.e. -(CH₂)₄-NH-) is already shown):

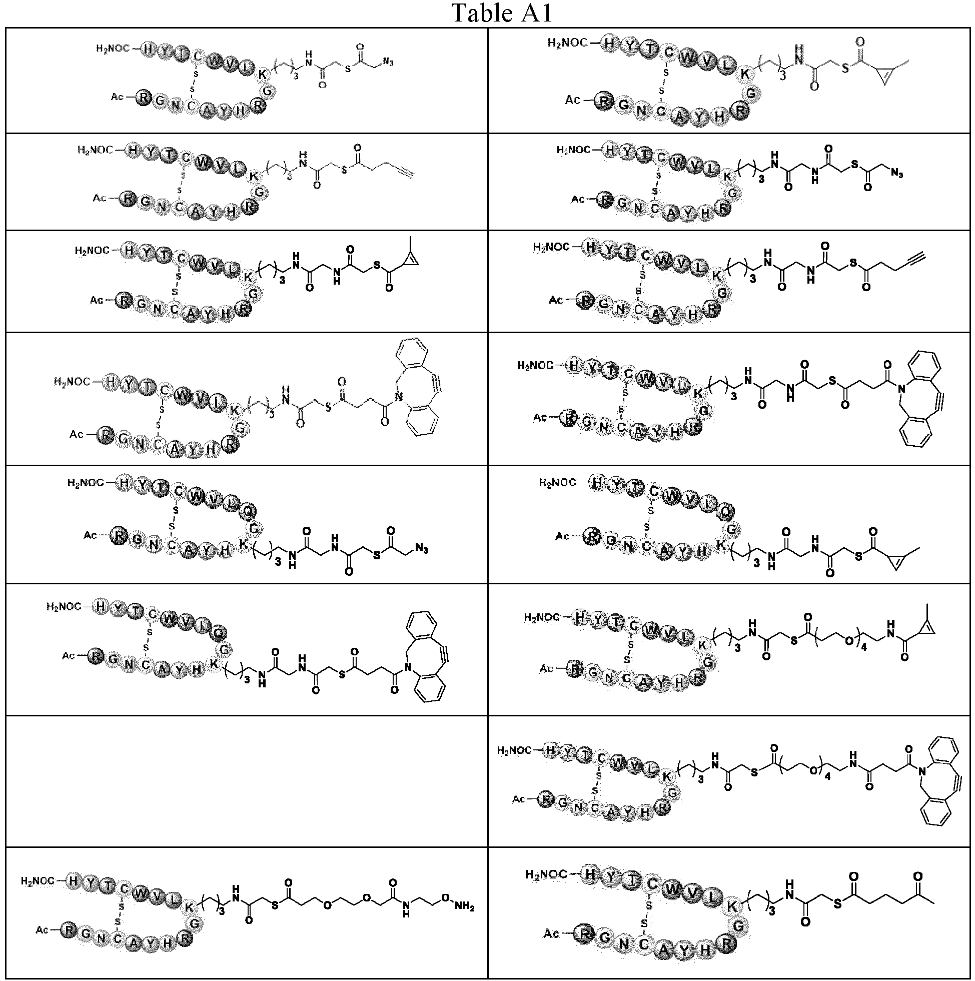

wherein Ac is an N-terminal protecting group or absent (preferably, is acetyl).

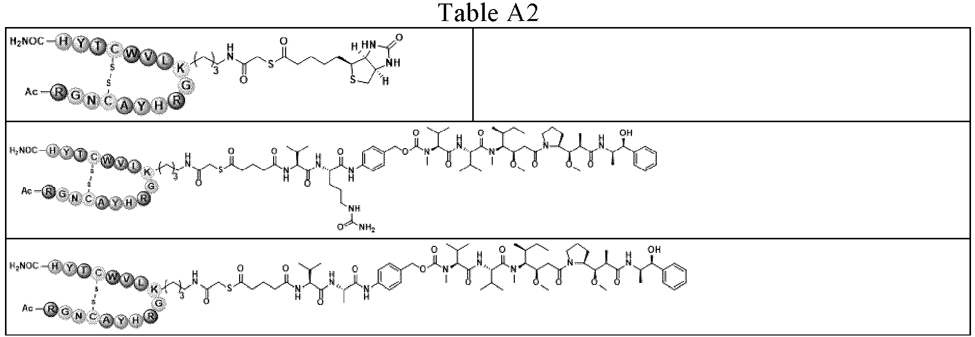

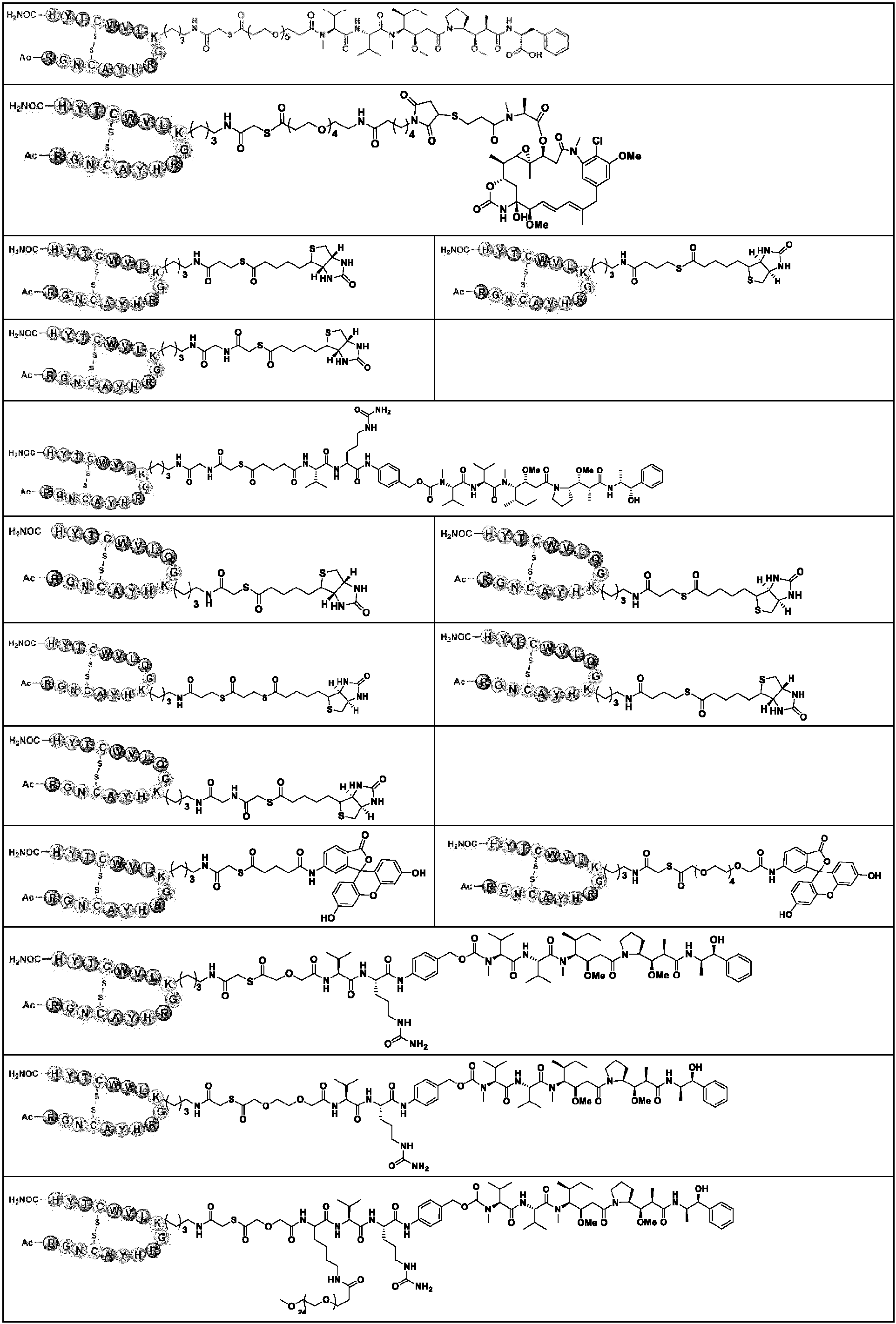

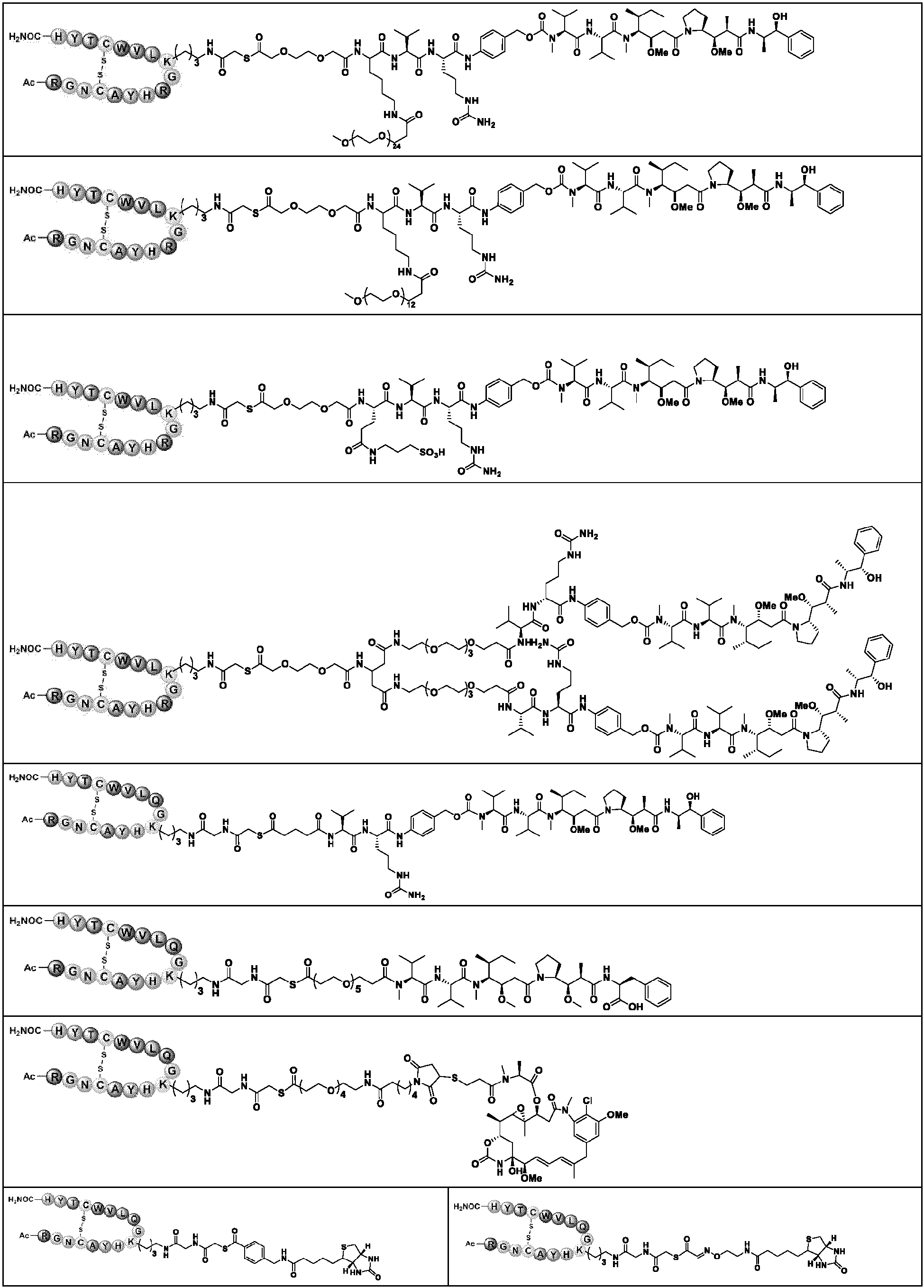

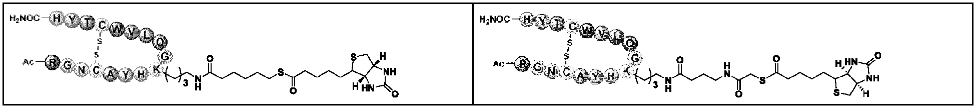

wherein Ac is an N-terminal protecting group or absent (preferably, is acetyl).

Preferably, the cleavable fragment directed by affinity fragment (acyl transfer reagent) can directly transfer the R group to the natural antibody (Ab), to achieve the selective modification of the amino acids of the antibody, forming functionalized modification, fluorescent modification, drug coupling, peptide coupling, mRNA coupling, etc., and the antibody modification structure as shown in below formula (V):

Ab-NHCO-R (V)

wherein, Ab is an antibody, -NHCO- is an amide bond structure, and R is a modifying fragment that is to be modified onto the antibody.

In another aspect of the present invention, provided is a method for preparing the cleavable fragment directed by affinity fragment as described above, as shown in any of the following reaction formulas: 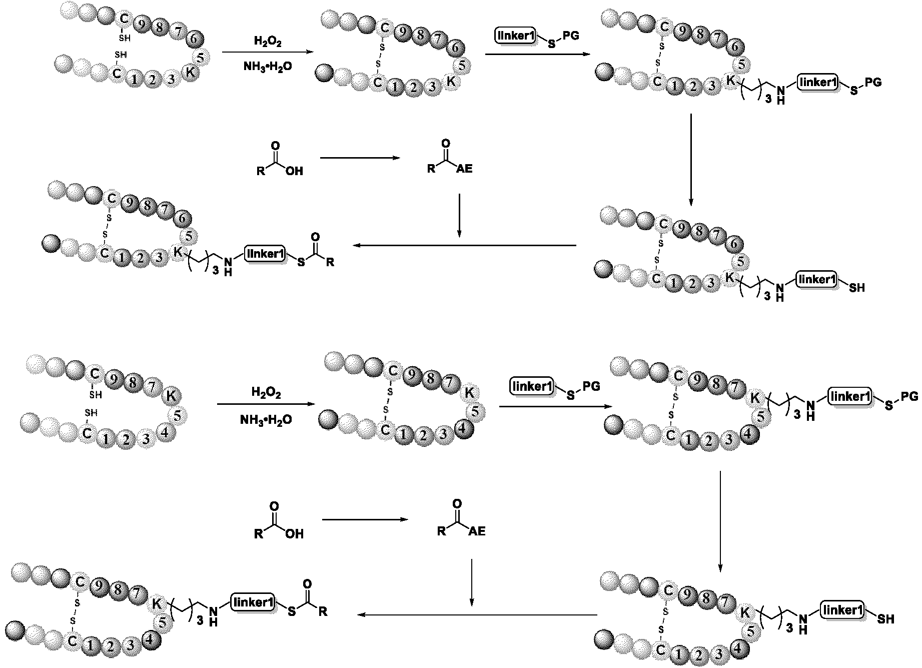 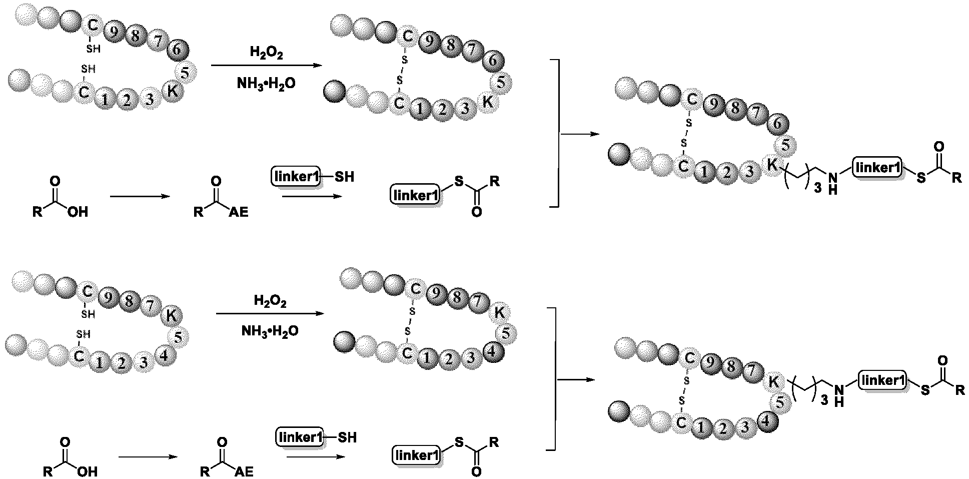 wherein linker 1 is -L¹-W¹-A¹-; PG is any sulfydryl protecting group other than thiol, such as Trt-, Acm-, Mob, etc.; AE is any active ester form, such as NHS ester, acyl chloride, acyl azide, thioester, p-nitrophenol ester, etc.; L¹, W¹, A¹, circles 1-9, circle K, circle C, 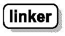 , and R are as defined above.

In another embodiment, the preparation method includes steps of:
Step 1: synthesis of an affinity fragment: after obtaining the polypeptide by solid phase synthesis, the polypeptide was dissolved in DMSO to a final concentration of 5 mM; hydrogen peroxide (10 mM) and ammonia water (100 mM) was added, and the mixture was stirred at room temperature overnight; after the reaction was completed as monitored by LC-MS, the reaction was subjected to semi-preparative separation and purification, and lyophilized to obtain the affinity fragment.
Step 2: synthesis of the protected affinity fragment-thiol derivative
Step 3: synthesis of the affinity fragment-thiol derivatives: obtaining the crude product of affinity fragment-thiol derivative via the corresponding deprotection strategies.
Step 4: preparation of cleavable fragment directed by affinity fragment: mixing the abovementioned crude product of affinity fragment-thiol derivative with the active ester form of carboxylic acid compound; after LC-MS monitoring of complete reaction, subjecting the reaction to semi-preparative separation and purification, and performing lyophilization to obtain the cleavable fragment directed by affinity fragment.

In another embodiment, the preparation method includes steps of:
Step 1: synthesis of affinity fragment: after obtaining the polypeptide by solid phase synthesis, dissolving the polypeptide in DMSO to a final concentration of 5 mM; adding hydrogen peroxide (10 mM) and ammonia water (100 mM), stirring the mixture at room temperature overnight; after LC-MS monitoring of complete reaction, subjecting the reaction to semi-preparative separation and purification, and performing lyophilization to obtain the affinity fragment.
Step 2: synthesis of cleavable fragment: reacting the active ester form of carboxylic acid compound with thiol derivative to obtain the cleavable fragmentthioester compound.
Step 3: preparation of cleavable fragment directed by affinity fragment: the affinity fragment and the cleavable fragment undergo reactions such as condensation, reductive amination and the like to obtain the cleavable fragment directed by affinity fragment.

In one aspect of present invention, provided is a method for preparing a site-specific antibody drug conjugate based on the above cleavable fragment directed by affinity fragment (i.e., directing group-thioester-carrier molecule complex)(or a method for site-specific modification of antibody or Fc fusion protein), as shown in the following reaction formula: 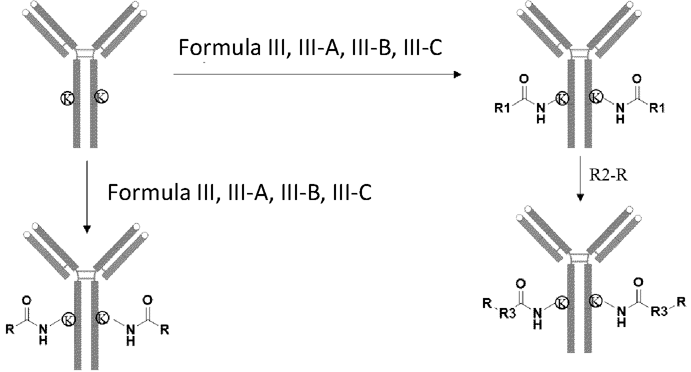 wherein, R is a modifying structure coupled to the antibody, R1 and R2 are bioorthogonal groups, and R3 is a linker structure formed by the reaction of R1 and R2.

Preferably, the preparation method or site-specific modification method comprises incubating the complex of formula (I) (as described above) with an antibody or Fc fusion protein to achieve site-specific modification of lysine at a specific site in the Fc region of the antibody or Fc fusion protein.
Preferably, the antibody or Fc fusion protein has fragment or sequence that has an affinity for the complex of formula (I).

Preferably, the antibody or Fc structure derived from human IgG

Preferably, the covalent modification region formed by interacting with the complex shown in (I) is a region consisting of 1-10 consecutive amino acid residues, wherein the target region is a region formed by the amino acid residues at positions 246-248 in the human IgG Fc region.

Preferably, the target region is composed of a lysine residue at position 246 or 248 in the human IgG Fc region.

Preferably, in the antibody or Fc fusion protein, the selectivity of the region is greater than 90%.

Preferably, antibody drug conjugates or site-specificly modified antibodies or Fc fusion proteins.

### The main advantages of the present invention include:

(a) When the conjugate provided by the present invention is used to modify proteins such as antibodies, the modification efficiency is high (>95%).
(b) The conjugate provided by the present invention can be directly modified on the side chain amino group of the amino acid residue at the site to be modified in the protein without the need to modify the side chain amino group in advance.
(c) The conjugate provided by the present invention has little influence on the activity of the antibody.
(d) The conjugate provided by the present invention is easy to operate for antibody modification, has no strict requirements on pH and concentration (with pH 5.5-8.0, an antibody concentration of 0.5 mg/mL-10 mg/mL), and is convenient for technology promotion.
(e) The method constructed by the present invention has a wide range of uses and can be used to construct an antibody rapid modification platform, namely, the diversified modification of antibodies and the preparation of antibody-drug conjugates (ADCs).
(f) The method constructed by the present invention realizes the one-step site-specific traceless modification of antibodies for the first time.
(g) The method constructed by the present invention can be used to prepare site-specificly modified ADCs in one step, and is expected to construct a large-capacity ADC library for systematic screening and optimization of drug linkers of ADCs.
(h) The thioester fragment provided by the present invention is used as an acyl transfer reagent and connected to a specific site of the Fc binding peptide, so as to achieve "one-step" efficient site-specific coupling of small molecule drugs, and is compatible with a variety of drug linker structures and has a wide range of substrate applicability.

The present invention was further described hereafter in combination with specific embodiments. It should be understood that these examples are only used to illustrate the present invention and not to limit the scope of the invention. The experimental methods without specific conditions in the following examples generally follow the conventional conditions or the conditions suggested by the manufacturer. Unless otherwise stated, percentages and parts are percentages by weight and parts by weight.

Unless otherwise stated, the detection methods and conditions in the examples are as follows:

### ESI-TOFMS

Mobile phase A: 0.1% FA in water, mobile phase B: 0.1% FA in acetonitrile.

Method A: Used for mass spectrometry analysis of small molecule compounds. A C18 column (ACQUITY UPLC BEH C18, 1.7 µm, 2.1 × 50 mm) is used with a liquid phase program of 0-0.2-1.5-1.8-2.0-2.5 min corresponding to the mobile phase B of 10-10-70-70-10-10%, a detection wavelength of 214 nm, and a column temperature of 35 °C.

Method B: Used for mass spectrometry analysis of antibodies and ADC molecules. A C4 column (ACQUITY UPLC Protein BEH C4, 1.7 µm, 2.1 mm x 50 mm) is used with a liquid phase program of 0-2.0-6.0-7.2-7.3-7.6-7.7-8.0-8.1-10.0 min corresponding to the mobile phase B of 5-5-90-90-5-90-5-90-5-5%, a detection wavelength of 280 nm, and a column temperature of 80 °C.

### Hydrophobic Interaction Chromatography Analysis

The ADC molecule to be tested is diluted to 5 mg/mL using 1 × PBS and 5 µL is injected. Mobile phase A: 0.1 M sodium phosphate, 1.5 M ammonium sulfate buffer, pH=7.0; mobile phase B: 0.1 M sodium phosphate buffer, pH=7.0, at a flow rate of 1 mL/min, 0-15 min, a linear gradient of 0-100% for mobile phase B. Detection wavelength: 280 nm.

### Molecular-exclusion chromatography

The ADC molecule to be tested is diluted to 1 mg/mL with 1 × PBS and heated at 60 °C in a PCR instrument for 48 h. Samples are taken at 0 h, 6 h, 12 h, 24 h, and 48 h for SEC analysis. 15 µL of sample is taken at each time point. SEC analysis is performed using a mobile phase of 150 mM sodium phosphate, pH = 7.4, at a flow rate of 1 mL/min, an isocratic elution of 15 min, and a detection wavelength of 280 nm.

### I: Preparation Of Affinity Fragment

### Preparation Example 1: Synthesis of Compound AT-1

**Structure and synthesis method of Compound AT-1 are as follows:**

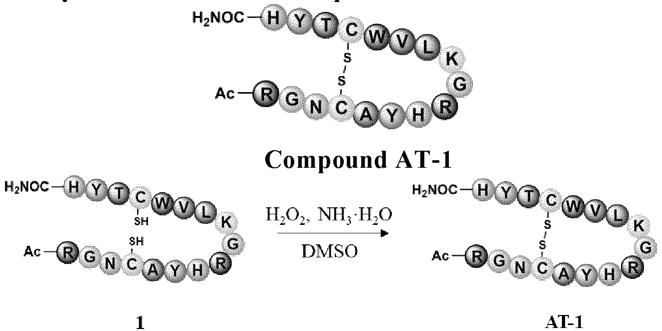

Compound 1 (200 mg, 0.095 mmol) was weighed and dissolved in DMSO to a final concentration of 5 mM. Hydrogen peroxide (10 mM) and ammonia water (100 mM) were added and the mixture was stirred at room temperature overnight. When the reaction was completed as monitored by LC-MS, the reaction was subjected to semi-preparative separation and purification, and freeze-dried to obtain a white powder, i.e., Compound AT-1 (yield 91%). HRMS, calculated for C₉₃H₁₃₅N₃₁O₂₂S₂: [M+H]⁺ 2102.9918, [M+2H]²⁺ 1051.9998, [M+3H]³⁺ 701.6691, [M+4H]⁴⁺ 526.5038 observed 701.6684, 526.5048.

### Preparation Example 2: Synthesis of Compound AT-2

**Structure and synthesis method of Compound AT-2 are as follows:**

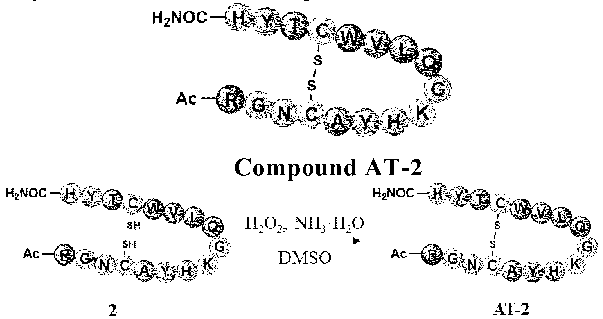

Compound 2 (200 mg, 0.096 mmol) was weighed and dissolved in DMSO to a final concentration of 5 mM. Hydrogen peroxide (10 mM) and ammonia water (100 mM) were added and the mixture was stirred at room temperature overnight. When the reaction was completed as monitored by LC-MS, the reaction was subjected to semi-preparative separation and purification, and freeze-dried to obtain a white powder, i.e., Compound AT-2 (yield 90%). HRMS, calculated for C₉₂H₁₃₁N₂₉O₂₃S₂: [M+H]⁺ 2074.9491, [M+2H]²⁺ 1037.9785, [M+3H]³⁺ 692.3216, observed 1037.9715, 692.3156.

### II: Preparation Of Affinity Fragments-Thiol Derivatives

### Preparation Example 3: Synthesis of Compound ATS-1

**Structure and synthesis method of Compound ATS-1 are as follows:**

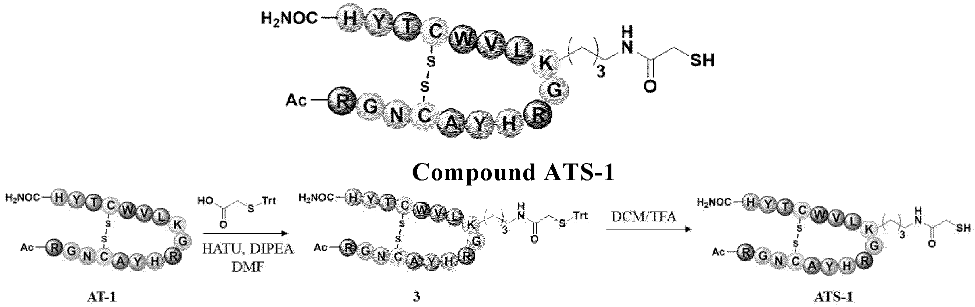

Step 1: 2-(triphenylmethylthio)acetic acid (15.7 mg, 0.047 mmol) was weighed and dissolved in DMF. HATU (7.14 mg, 0.0188 mmol), DIPEA (4.84 µL, 0.028 mmol) and Compound **AT-1** (20 mg, 0.0094 mmol) were added sequentially and the mixture was stirred at room temperature for 1 h. When the reaction was completed as monitored by LC-MS, the reaction was subjected to semi-preparative separation and purification, and lyophilized to obtain a white powder, i.e., Compound 3 (yield 92%). HRMS, calculated for C₁₁₄H₁₅₁N₃₁O₂₃S₃: [M+H]⁺ 2419.0839, [M+3H]³⁺ 807.0332, [M+4H]⁴⁺ 605.5269, observed 807.0342, 605.5232.

Step 2: Compound **3** (15 mg) was weighed and dissolved in 500 µL of dichloromethane. 450 µL of trifluoroacetic acid and 50 µL of triisopropylsilane were added under ice bath conditions. After reacting at room temperature for 1 h, the reaction was blown dry with N₂ to obtain Compound **ATS-1.** HRMS, calculated for C₉₅H₁₃₇N₃₁O₂₃S₃: [M+H]⁺ 2176.9744, [M+3H]³⁺ 726.3300, [M+4H]⁴⁺ 544.9995, observed 726.3278, 545.0035.

### Preparation Example 4: Synthesis of Compound ATS-2

**Structure and synthesis method of Compound ATS-2 are as follows:**

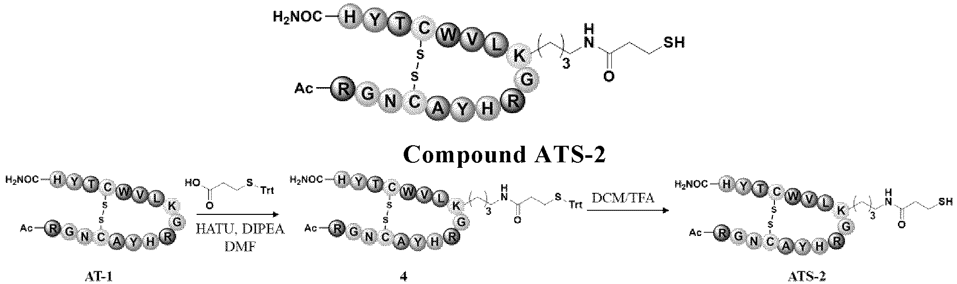

Step 1: 3-(triphenylmethylthio)propionic acid (16.36 mg, 0.047 mmol) was weighed and dissolved in DMF. HATU (7.14 mg, 0.0188 mmol), DIPEA (4.84 µL, 0.028 mmol) and Compound **AT-1** (20 mg, 0.0094 mmol) were added sequentially and the mixture was stirred at room temperature for 1 h. When the reaction was completed as monitored by LC-MS, the reaction was subjected to semi-preparative separation and purification, and lyophilized to obtain a white powder, i.e., Compound **4** (yield 89%). HRMS, calculated for C₁₁₅H₁₅₃N₃₁O₂₃S₃: [M+H]⁺ 2433.0996, [M+3H]³⁺ 811.7051, [M+4H]⁴⁺ 609.0308, observed 811.7012, 609.0299.

Step 2: Compound **4** (15 mg) was weighed and dissolved in 500 µL of dichloromethane. 450 µL of trifluoroacetic acid and 50 µL of triisopropylsilane were added under ice bath conditions. After reacting at room temperature for 1 h, the reaction was blown dry with N₂ to obtain Compound **ATS-2.** HRMS, calculated for C₉₆H₁₃₉N₃₁O₂₃S₃: [M+H]⁺ 2190.9900, [M+3H]³⁺ 731.0019, [M+4H]⁴⁺ 548.5034, observed 731.0054, 548.5102.

### Preparation Example 5: Synthesis of Compound ATS-3

**Structure and synthesis method of Compound ATS-3 are as follows:**

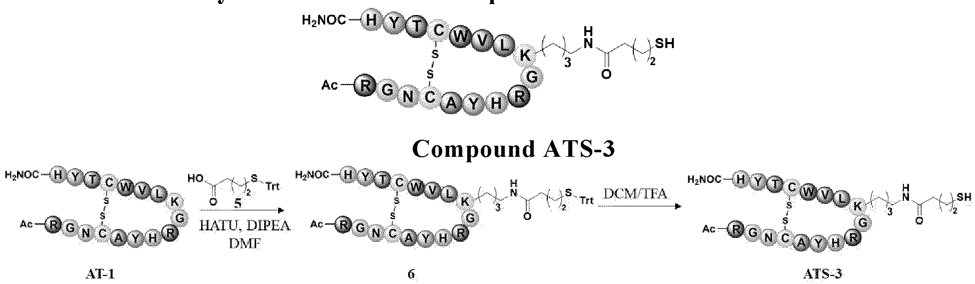

Step 1: NaH (34.5 mg, 1.44 mmol) was weighed and dissolved in DMF (5 mL). Under N₂ protection and ice bath conditions, triphenylmethyl mercaptan (200 mg, 0.72 mmol) was added at 0 °C. After stirring in ice bath for 30 min, 4-bromobutyric acid (60 mg, 0.36 mmol) was added. After the temperature slowly rose to room temperature, the mixture was stirred overnight to react, and separated and purified by silica gel column to obtain compound 5 (yield 76%). 1HNMR (600 MHz, DMSO-*d*₆) δ 12.02 (s, 1H), 7.33 - 7.15 (m, 15H), 2.12 (t, *J* = 7.3 Hz, 2H), 2.08 (t, *J =* 7.4 Hz, 2H), 1.48 (p, *J =* 7.3 Hz, 2H). ¹³C NMR (151 MHz, DMSO) δ 173.70, 144.50, 129.08, 128.02, 126.70, 66.02, 32.72, 30.72, 23.56.

Step 2: Compound **5** (17 mg, 0.047 mmol) was weighed and dissolved in DMF. HATU (7.14 mg, 0.0188 mmol), DIPEA (4.84 µL, 0.028 mmol) and Compound **AT-1** (20 mg, 0.0094 mmol) were added sequentially and the mixture was stirred at room temperature for 1 h. When the reaction was completed as monitored by LC-MS, the reaction was subjected to semi-preparative separation and purification, and lyophilized to obtain a white powder, i.e., Compound 6 (yield 91%). HRMS, calculated for C₁₁₆H₁₅₅N₃₁O₂₃S₃: [M+H]⁺ 2447.1152, [M+3H]³⁺ 816.3770, [M+4H]⁴⁺ 612.5347, observed 816.3715, 612.5382.

Step 3: Compound **6** (15 mg) was weighed and dissolved in 500 µL of dichloromethane. 450 µL of trifluoroacetic acid and 50 µL of triisopropylsilane were added under ice bath conditions. After reacting at room temperature for 1 h, the reaction was blown dry with N₂ to obtain Compound **ATS-3.** HRMS, calculated for C₉₇H₁₄₁N₃₁O₂₃S₃: [M+H]⁺ 2205.0057, [M+3H]³⁺ 735.6738, [M+4H]⁴⁺ 552.0073, observed 735.6742, 552.0153.

### Preparation Example 6: Synthesis of Compound ATS-4

**Structure and synthesis method of Compound ATS-4 are as follows:**

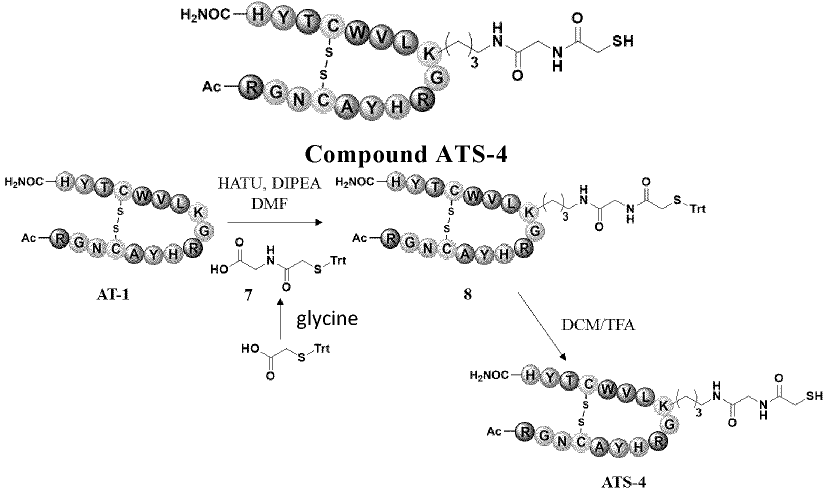

Step 1: 2-(triphenylmethylthio)acetic acid (334 mg, 1 mmol) was weighed and dissolved in 5 mL DMF. N-hydroxysuccinimide (138 mg, 1.2 mmol) and 1-(3-dimethylaminopropyl)-3-ethylcarbodiimide hydrochloride (230 mg, 1.2 mmol) were added and the mixture was stirred at room temperature overnight. Then glycine (75 mg, 1 mmol) was added, and the mixture was reacted at room temperature for 2 h. ¹H NMR (500 MHz, DMSO-*d*₆) δ 8.24 (t, *J =* 5.8 Hz, 1H), 7.38 - 7.23 (m, 15H), 3.67 (d, *J =* 5.8 Hz, 2H), 2.84 (s, 2H). ¹³C NMR (126 MHz, DMSO) δ 170.93, 167.63, 144.03, 129.10, 128.11, 126.83, 66.03, 40.89, 35.77.

Step 2: Compound 7 (18.4 mg, 0.047 mmol) was weighed and dissolved in DMF. HATU (7.14 mg, 0.0188 mmol), DIPEA (4.84 µL, 0.028 mmol) and Compound **AT-1** (20 mg, 0.0094 mmol) were added sequentially and the mixture was stirred at room temperature for 1 h. When the reaction was completed as monitored by LC-MS, the reaction was subjected to semi-preparative separation and purification, and lyophilized to obtain a white powder, i.e., Compound **8** (yield 87%). HRMS, calculated for C₁₁₆H₁₅₄N₃₂O₂₄S₃: [M+H]⁺ 2476.1054, [M+3H]³⁺ 826.0404, [M+4H]⁴⁺ 619.7822, observed 826.0475, 619.1098.

Step 3: Compound **8** (15 mg) was weighed and dissolved in 500 µL of dichloromethane. 450 µL of trifluoroacetic acid and 50 µL of triisopropylsilane were added under ice bath conditions. After reacting at room temperature for 1 h, the reaction was blown dry with N₂ to obtain Compound **ATS-4.** HRMS, calculated for C₉₇H₁₄₀N₃₂O₂₄S₃: [M+H]⁺ 2233.9955, [M+3H]³⁺ 745.3372, [M+4H]⁴⁺ 559.2548, observed 745.3356, 559.2573.

### Preparation Example 7: Synthesis of Compound ATS-5

**Structure and synthesis method of Compound ATS-5 are as follows:**

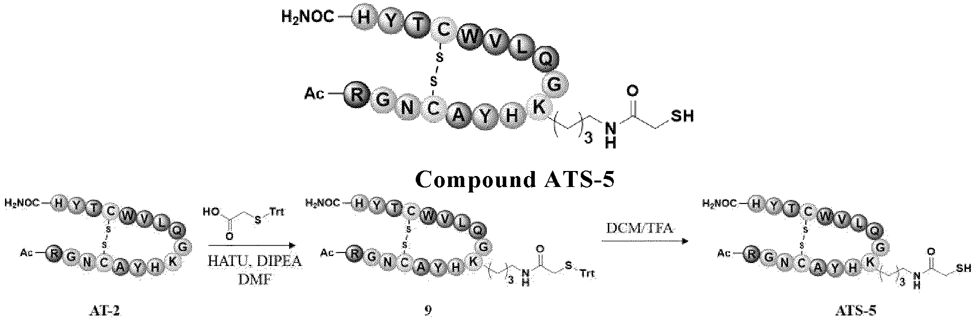

Step 1: 2-(triphenylmethylthio)acetic acid (15.7 mg, 0.047 mmol) was weighed and dissolved in DMF. HATU (7.14 mg, 0.0188 mmol), DIPEA (4.84 µL, 0.028 mmol) and Compound **AT-2** (19.5 mg, 0.0094 mmol) were added sequentially and the mixture was stirred at room temperature for 1 h. When the reaction was completed as monitored by LC-MS, the reaction was subjected to semi-preparative separation and purification, and lyophilized to obtain a white powder, i.e., Compound **9** (yield 87%). HRMS, calculated for C₁₁₃H₁₄₇N₂₉O₂₄S₃: [M+H]⁺ 2391.0414, [M+3H]³⁺ 797.6857, [M+4H]⁴⁺ 598.5162, observed 797.685, 598.5136.

Step 2: Compound **9** (15 mg) was weighed and dissolved in 500 µL of dichloromethane. 450 µL of trifluoroacetic acid and 50 µL of triisopropylsilane were added under ice bath conditions. After reacting at room temperature for 1 h, the reaction was blown dry with N₂ to obtain Compound **ATS-5.** HRMS, calculated for C₉₄H₁₃₃N₂₉O₂₄S₃: [M+H]⁺ 2148.9319, [M+3H]³⁺ 716.9825, [M+4H]⁴⁺ 537.9888, observed 716.9867, 537.9872.

### Preparation Example 8: Synthesis of Compound ATS-6

**Structure and synthesis method of Compound ATS-6 are as follows:**

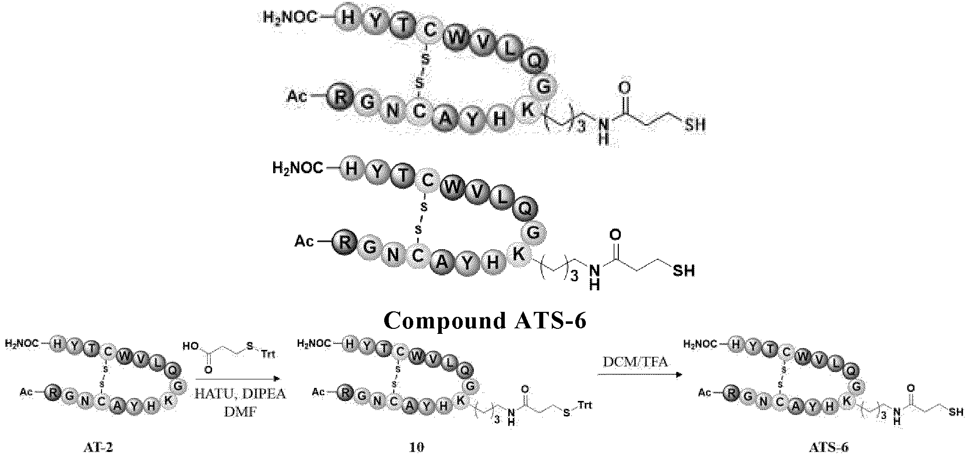

Step 1: 3-(triphenylmethylthio)propionic acid (16.36 mg, 0.047 mmol) was weighed and dissolved in DMF. HATU (7.14 mg, 0.0188 mmol), DIPEA (4.84 µL, 0.028 mmol) and Compound **AT-2** (19.5 mg, 0.0094 mmol) were added sequentially and the mixture was stirred at room temperature for 1 h. When the reaction was completed as monitored by LC-MS, the reaction was subjected to semi-preparative separation and purification, and lyophilized to obtain a white powder, i.e., Compound **10** (yield 89%). HRMS, calculated for C₁₁₄H₁₄₉N₂₉O₂₄S₃: [M+H]⁺ 2405.0571, [M+3H]³⁺ 802.3576, [M+4H]⁴⁺ 602.0201, observed 802.3512, 602.0258.

Step 2: Compound **10** (15 mg) was weighed and dissolved in 500 µL of dichloromethane. 450 µL of trifluoroacetic acid and 50 µL of triisopropylsilane were added under ice bath conditions. After reacting at room temperature for 1 h, the reaction was blown dry with N₂ to obtain Compound **ATS-6.** HRMS, calculated for C₉₅H₁₃₄N₂₉O₂₄S₃: [M+H]⁺ 2162.9475, [M+3H]³⁺ 721.6544, [M+4H]⁴⁺ 541.4928, observed 721.6523, 514.4953.

### Preparation Example 9: Synthesis of Compound ATS-7

**Structure and synthesis method of Compound ATS-7 are as follows:**

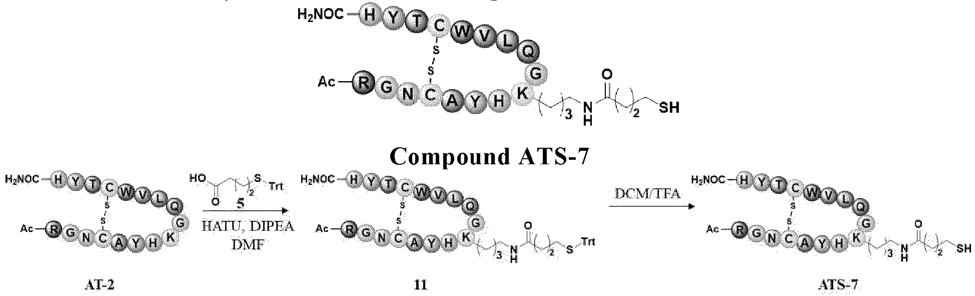

Step 1: Compound **5** (17 mg, 0.047 mmol) was weighed and dissolved in DMF. HATU (7.14 mg, 0.0188 mmol), DIPEA (4.84 µL, 0.028 mmol) and Compound **AT-2** (19.5 mg, 0.0094 mmol) were added sequentially and the mixture was stirred at room temperature for 1 h. When the reaction was completed as monitored by LC-MS, the reaction was subjected to semi-preparative separation and purification, and lyophilized to obtain a white powder, i.e., Compound **11** (yield 92%). HRMS, calculated for C₁₁₅H₁₅₁N₂₉O₂₄S₃: [M+H]⁺ 2419.0727, [M+3H]³⁺ 807.0294, [M+4H]⁴⁺ 605.5240, observed 807.0287, 605.5198.

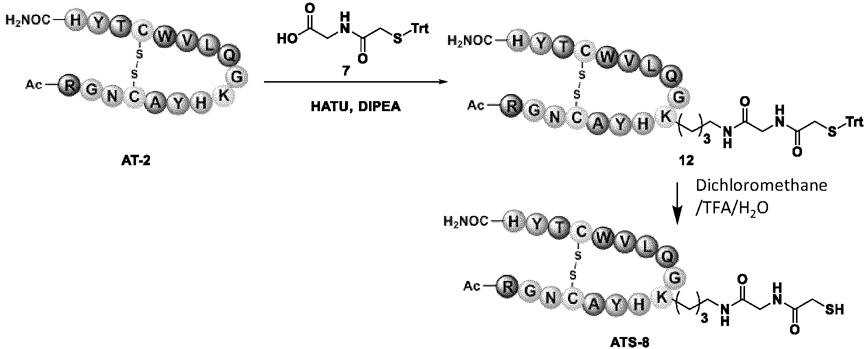

Step 2: Compound **11** (15 mg) was weighed and dissolved in 500 µL of dichloromethane. 450 µL of trifluoroacetic acid and 50 µL of triisopropylsilane were added under ice bath conditions. After reacting at room temperature for 1 h, the reaction was blown dry with N₂ to obtain Compound **ATS-7**. HRMS, calculated for C₉₆H₁₃₇N₂₉O₂₄S₃: [M+H]⁺ 2176.9632, [M+3H]³⁺ 726.3263, [M+4H]⁴⁺ 544.9967, observed 726.3248, 544.9913.

### Preparation Example 10: Synthesis of Compound ATS-8

**Structure and synthesis method of Compound ATS-8 are as follows:**

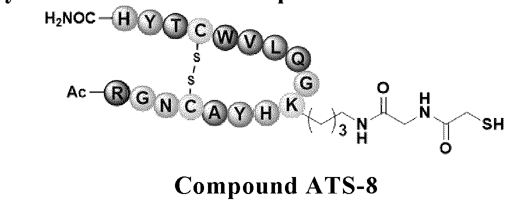

Step 1: Compound 7 (18.4 mg, 0.047 mmol) was weighed and dissolved in DMF. HATU (7.14 mg, 0.0188 mmol), DIPEA (4.84 µL, 0.028 mmol) and Compound **AT-2** (19.5 mg, 0.0094 mmol) were added sequentially and the mixture was stirred at room temperature for 1 h. When the reaction was completed as monitored by LC-MS, the reaction was subjected to semi-preparative separation and purification, and lyophilized to obtain a white powder, i.e., Compound **12** (yield 87%). HRMS, calculated for C₁₁₅H₁₅₀N₃₀O₂₅S₃: [M+H]⁺ 2448.0619, [M+3H]³⁺ 816.6928, [M+4H]⁴⁺ 612.7716, observed 816.6915, 612.7742.

Step 2: Compound **12** (15 mg) was weighed and dissolved in 500 µL of dichloromethane. 450 µL of trifluoroacetic acid and 50 µL of triisopropylsilane were added under ice bath conditions. After reacting at room temperature for 1 h, the reaction was blown dry with N₂ to obtain Compound **ATS-8.** HRMS, calculated for C₉₆H₁₃₆N₃₀O₂₅S₃: [M+H]⁺ 2205.9533, [M+3H]³⁺ 735.9897, [M+4H]⁴⁺ 552.2442, observed 735.9910, 552.2418.

### Preparation Example 11: Synthesis of Compound ATS-9

**Structure and synthesis method of Compound ATS-9 are as follows:**

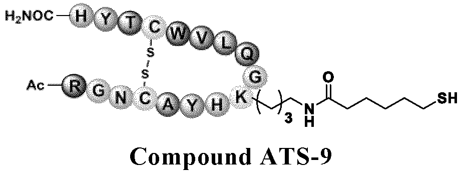

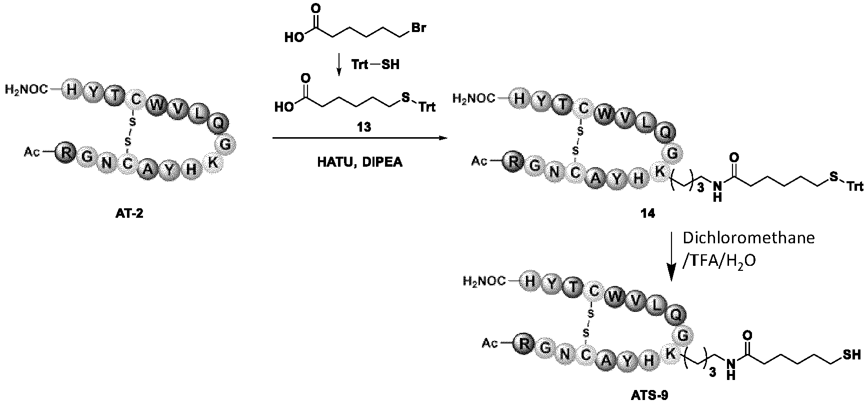

Step 1: NaH (34.5 mg, 1.44 mmol) was weighed and dissolved in DMF (5 mL). Under N₂ protection and ice bath conditions, triphenylmethyl mercaptan (200 mg, 0.72 mmol) was added at 0 °C. After stirring in ice bath for 30 min, 6-bromohexanoic acid (70 mg, 0.36 mmol) was added. After the temperature slowly rose to room temperature, the mixture was stirred overnight to react, and separated and purified by silica gel column to obtain compound **13** (yield 82%).

Step 2: Compound **13** (18.3 mg, 0.047 mmol) was weighed and dissolved in DMF. HATU (7.14 mg, 0.0188 mmol), DIPEA (4.84 µL, 0.028 mmol) and Compound **AT-2** (19.5 mg, 0.0094 mmol) were added sequentially and the mixture was stirred at room temperature for 1 h. When the reaction was completed as monitored by LC-MS, the reaction was subjected to semi-preparative separation and purification, and lyophilized to obtain a white powder, i.e., Compound **14** (yield 87%). HRMS, calculated for C₁₁₇H₁₅₅N₂₉O₂₄S₃: [M+H]⁺ 2447.1040, [M+3H]³⁺ 816.3732, [M+4H]⁴⁺ 612.5319, observed 816.3798, 612.5326.

Step 3: Compound **14** (15 mg) was weighed and dissolved in 500 µL of dichloromethane. 450 µL of trifluoroacetic acid and 50 µL of triisopropylsilane were added under ice bath conditions. After reacting at room temperature for 1 h, the reaction was blown dry with N₂ to obtain Compound **ATS-9.** HRMS, calculated for C₉₈H₁₄₁N₂₉O₂₄S₃: [M+H]⁺ 2204.9945, [M+3H]³⁺ 735.6700, [M+4H]⁴⁺ 552.0044, observed 735.6712, 552.0019.

### Preparation Example 12: Synthesis of Compound ATS-10

**Structure and synthesis method of Compound ATS-10 are as follows:**

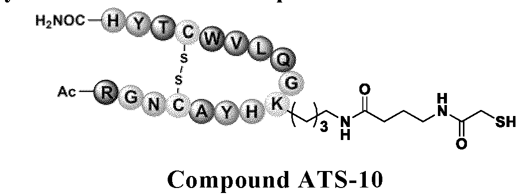

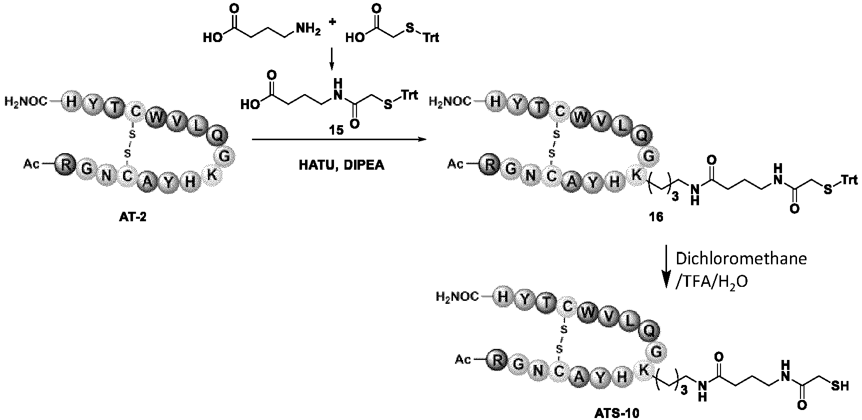

Step 1: 2-(triphenylmethylthio)acetic acid (334 mg, 1 mmol) was weighed and dissolved in 5 mL of DMF. N-hydroxysuccinimide (138 mg, 1.2 mmol) and 1-(3-dimethylaminopropyl)-3-ethylcarbodiimide hydrochloride (230 mg, 1.2 mmol) were added and the mixture was stirred at room temperature overnight. Then 4-aminobutyric acid (103 mg, 1 mmol) was added, and the mixture was reacted at room temperature for 2 h to obtain Compound **15** (yield 78%). HRMS, calculated for C₂₅H₂₅NO₃S: [M+H]⁺ 420.1633, observed 420.1649.

Step 2: Compound **15** (19.7 mg, 0.047 mmol) was weighed and dissolved in DMF. HATU (7.14 mg, 0.0188 mmol), DIPEA (4.84 µL, 0.028 mmol) and Compound **AT-2** (19.5 mg, 0.0094 mmol) were added sequentially and the mixture was stirred at room temperature for 1 h. When the reaction was completed as monitored by LC-MS, the reaction was subjected to semi-preparative separation and purification, and lyophilized to obtain a white powder, i.e., Compound **16** (yield 82%). HRMS, calculated for C₁₁₇H₁₅₄N₃₀O₂₅S₃: [M+H]⁺ 2476.0942, [M+3H]³⁺ 826.0366, [M+4H]⁴⁺ 619.7794, observed 826.0342, 619.7758.

Step 3: Compound **16** (15 mg) was weighed and dissolved in 500 µL of dichloromethane. 450 µL of trifluoroacetic acid and 50 µL of triisopropylsilane were added under ice bath conditions. After reacting at room temperature for 1 h, the reaction was blown dry with N₂ to obtain Compound **ATS-10**. HRMS, calculated for C₉₈H₁₄₀N₃₀O₂₅S₃: [M+H]⁺ 2233.9846, [M+3H]³⁺ 745.3334, [M+4H]⁴⁺ 559.2520, observed 745.3316, 559.2561.

### III: Synthesis of Drug Linker

### Preparation Example 13: Synthesis of Compound D-1

**Structure and synthesis method of Compound D-1 are as follows:**

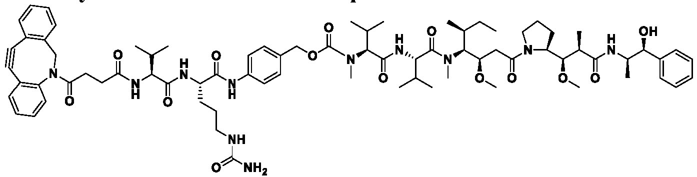

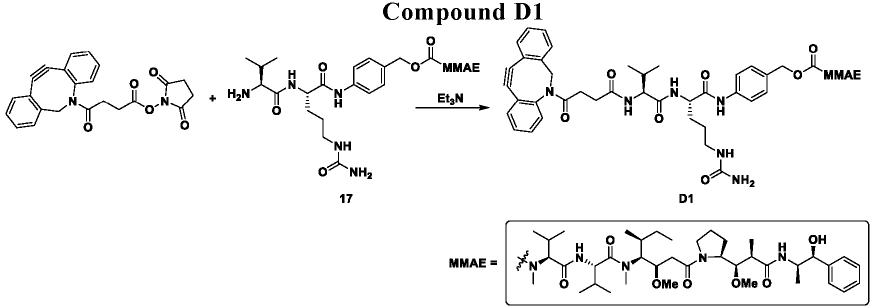

Compound **17** (10 mg, 8.9 µmol) was weighed and dissolved in 200 mL of DMF. DBCO-CONHS (4.3 mg, 10.7 µmol) and triethylamine (3.7 mL, 26.7 µmol) were added and the mixture was stirred at room temperature for 2 h. When the reaction was completed as monitored by LC-MS, the reaction was subjected to semi-preparative separation and purification, and lyophilized to obtain white powder i.e., compound **D1** (yield 81%). HRMS, calculated for C₇₇H₁₀₇N₁₁O₁₄: [M+H]⁺ 1410.8077, observed 1410.8052.

### Preparation Example 14: Synthesis of Compound D-2

**Structure and synthesis method of Compound D-2 are as follows:**

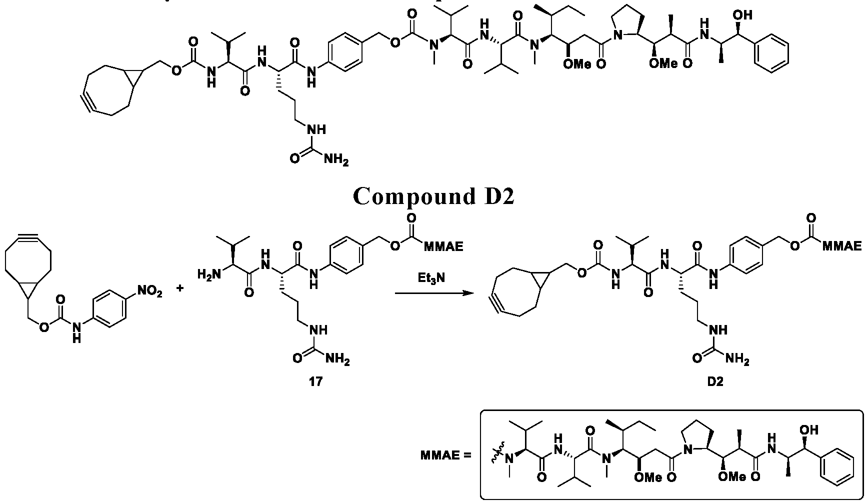

Compound **17** (10 mg, 8.9 µmol) was weighed and dissolved in 200 µL of DMF. BCN-O-PNP (3.5 mg, 10.7 µmol) and triethylamine (3.7 µL, 26.7 µmol) were added and the mixture was stirred at room temperature for 3 h. When the reaction was completed as monitored by LC-MS, the reaction was subjected to semi-preparative separation and purification, and lyophilized to obtain Compound **D2** (yield 76%). HRMS, calculated for C₆₉H₁₀₆N₁₀O₁₄: [M+H]⁺ 1299.7968, observed 1299.8016.

### Preparation Example 15: Synthesis of Compound D-3

**Structure and synthesis method of Compound D-3 are as follows:**

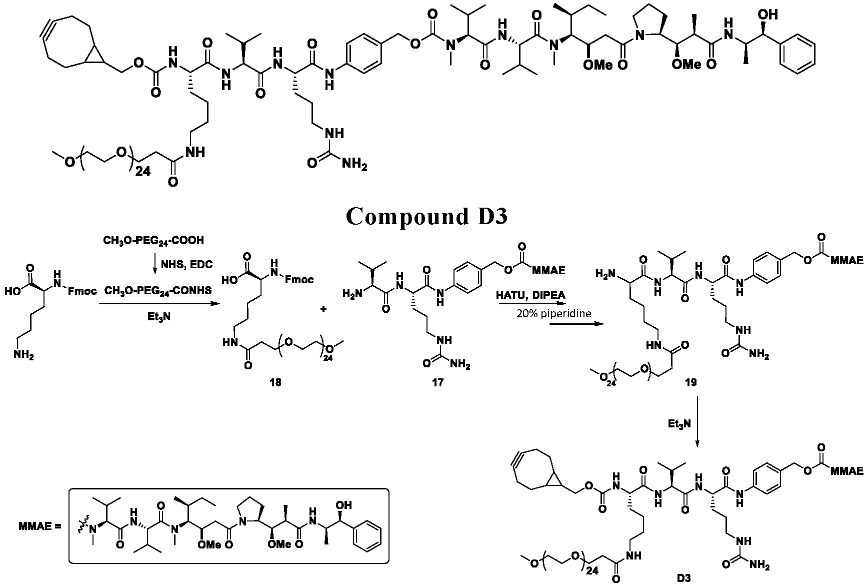

Step 1: CH₃O-PEG₂₄-COOH (58 mg, 0.05 mmol) was weighed and dissolved in acetonitrile. NHS (6.8 mg, 0.06 mmol) and EDC (7.9 µL, 0.06 mmol) were added. After reacting at room temperature overnight, a solution of Fmoc-Lys(NH₂)-OH (18.4 mg, 0.05 mmol) in acetonitrile was added and the mixture was stirred at room temperature for 2 h. When the reaction was completed as monitored by LC-MS, the reaction was subjected to semi-preparative separation and purification, and lyophilized to obtain a white powder, i.e., providing Compound **18** (yield 78%). HRMS, calculated for C₇₃H₁₂₆N₂O₃₀: [M+H]⁺ 756.4275, observed 756.4218.

Step 2: Compound **18** (13.5 mg, 8.9 µmol) was weighed and dissolved in DMF. HATU (6.8 mg, 17.8 µmol), DIPEA (4.6 µL, 27.6 µmol), and Compound **17** (10 mg, 8.9 µmol) were added and the mixture was reacted at room temperature for 2 h. When the reaction was completed as monitored by LC-MS, the reaction was subjected to semi-preparative separation and purification, and lyophilized to obtain a white powder i.e., Compound **19** (yield 86%). HRMS, calculated for C₁₁₆H₂₀₈N₁₂O₃₉: [M+H]⁺ 2394.4741, [M+3H]³⁺ 798.8299, observed 798.8312.

Step 3: Compound **19** (10 mg, 4.2 µmol) was weighed and dissolved in DMF. BCN-O-PNP (1.63 mg, 5 µmol) and triethylamine (1.7 µL, 12.6 µmol) were added and the mixture was stirred at room temperature for 2 h. When the reaction was completed as monitored by LC-MS, the reaction was subjected to semi-preparative separation and purification, and lyophilized to obtain Compound **D3** (yield 76%). HRMS, calculated for C₁₂₇H₂₂₀N₁₂O₄₁: [M+H]⁺ 2570.5577, [M+3H]³⁺ 857.5244, observed 857.5267.

### Preparation Example 16: Synthesis of Compound D-4

**Structure and synthesis method of Compound D-4 are as follows:**

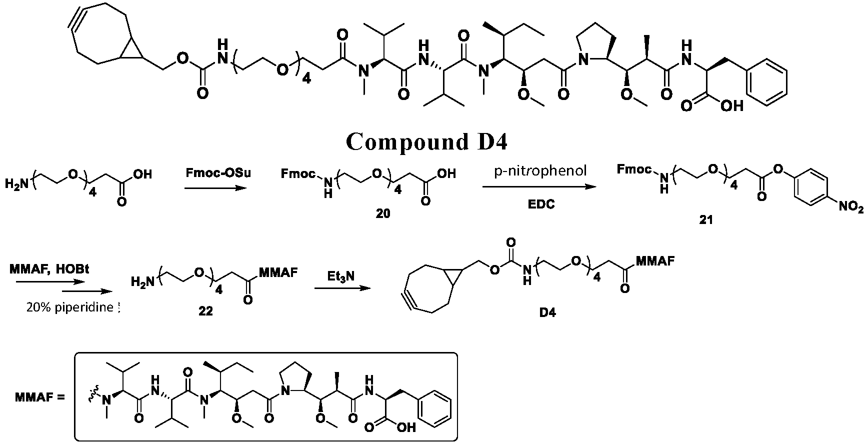

Step 1: H₂N-PEG₄-COOH (26.5 mg, 0.1 mmol) was dissolved in DMF, and 9-fluorenylmethyl-N-succinimidyl carbonate (67.4 mg, 0.2 mmol) and triethylamine (41.6 µL, 0.3 mmol) were added. After reacting at room temperature for 3 h, the reaction system was monitored by LC-MS, which showed that the reaction was complete. The reaction was subjected to semi-preparative separation and purification, and then lyophilized to obtain Compound **20.** HRMS, calculated for C₂₆H₃₃NO₈: [M+H]⁺ 488.2284, observed 488.2243 _{∘}

Step 2: Compound **20** (17 mg, 0.035 mmol) was weighed and dissolved in DMF. p-nitrophenol (9.7 mg, 0.07 mmol) and EDC (12.7 µL, 0.07 mmol) were added. After reacting at room temperature for 1 h, the reaction system was monitored by LC-MS, which showed that the reaction was complete. The reaction was subjected to semi-preparative separation and purification and, and then lyophilized to obtain Compound **21**. HRMS, calculated for C₃₂H₃₆N₂O₁₀: [M+H]⁺ 609.2448 observed 609.2252_{∘}

Step 3: Compound **21** (10 mg, 0.016 mmol) was weighed and dissolved in DMF. MMAF (12 mg, 0.02mmol), HOBt (0.54 mg, 3.2 µmol) and pyridine (36 µL, 0.45 mmol) were added and the mixture was reacted at room temperature for 1 h. When the reaction was basically completed as monitored by LC-MS, 20% piperidine was added and the mixture was reacted for 15 min. The reaction was subjected to semi-preparative separation and purification, and then lyophilized to obtain Compound **22**. HRMS, calculated for C₅₀H₈₆N₆O₁₃: [M+H]⁺ 979.6331 observed 979.6328_{∘}

Step 4: Compound **22** (10 mg, 0.01 mmol) was weighed and dissolved in DMF. BCN-O-PNB (9.45 mg, 0.03 mmol) and triethylamine (8.3 µL, 0.06 mmol) were added and the mixture was reacted at room temperature for 4 h. When the reaction was basically completed as monitored LC-MS, the reaction was subjected to semi-preparative separation and purification, and lyophilized to obtain Compound **D4.** HRMS, calculated for C₆₁H₉₈N₆O₁₅: [M+H]⁺ 1155.7168, [M+2H]²⁺ 578.3623, observed 578.3619_{∘}

### Preparation Example 17: Synthesis of Compound D-5

**Structure and synthesis method of Compound D-5 are as follows:**

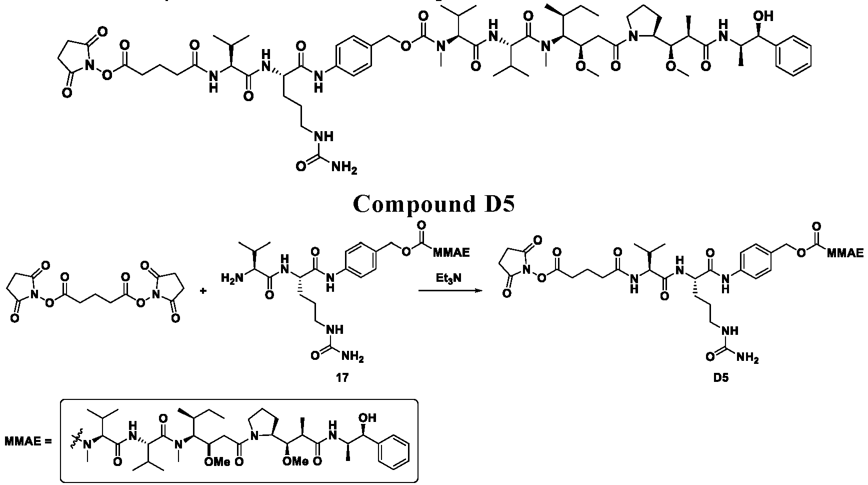

Compound **17** (10 mg, 9.8 µmol) was weighed and dissolved in DMF (500 µL). Di(N-succinimidyl) glutarate (DSG, 15.6 mg, 0.048 mmol) and triethylamine (4 µL, 0.029 mmol) were added and the mixture was reacted at room temperature for 2 h. When the reaction was completed as monitored by LC-MS, the reaction was subjected to semi-preparative separation and purification, and lyophilized to obtain a white powder **D5** (yield 91%). HRMS, calculated for C₆₇H₁₀₃N₁₁O₁₇: [M+H]⁺1334.7612, observed 1334.7655.

### Preparation Example 18: Synthesis of Compound D-6

**Structure and synthesis method of Compound D-6 are as follows:**

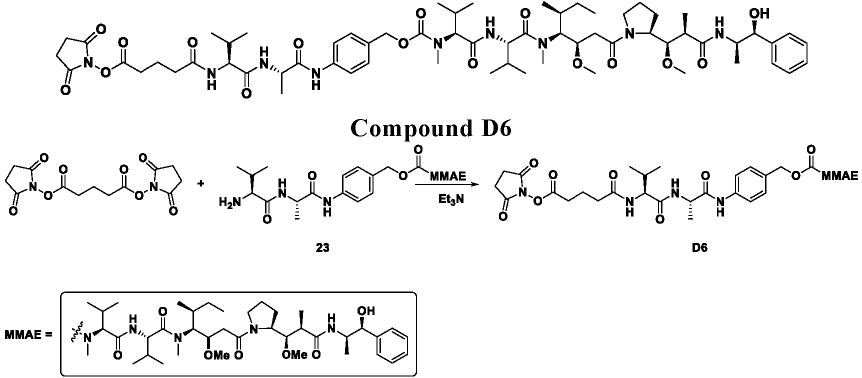

Compound **23** (10.1 mg, 9.8 µmol) was weighed and dissolved in DMF (500 µL). Di(N-succinimidyl) glutarate (DSG, 15.6 mg, 0.048 mmol) and triethylamine (4 µL, 0.029 mmol) were added and the mixture was reacted at room temperature for 2 h. When the reaction was completed as monitored by LC-MS, the reaction was subjected to semi-preparative separation and purification, and lyophilized to obtain a white powder **D6** (yield 85%). HRMS, calculated for C₆₄H₉₇N₉O₁₆: [M+H]⁺1248.7132, observed 1248.7154.

### Preparation Example 19: Synthesis of Compound D-7

**Structure and synthesis method of Compound D-7 are as follows:**

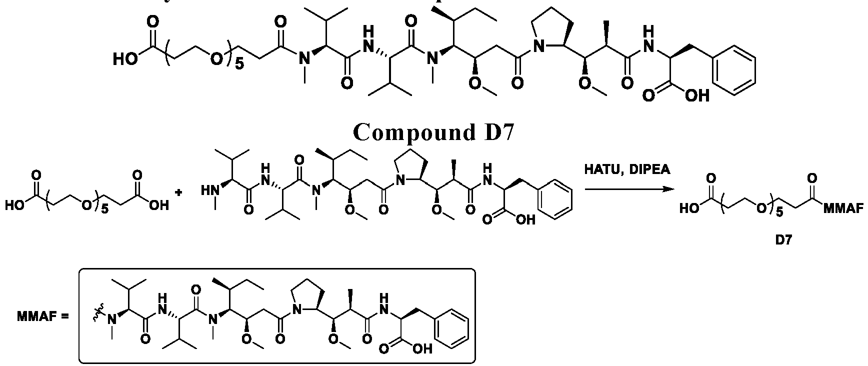

HOOC-PEG₅-COOH (23 mg, 0.068 mmol) was weighed and dissolved in DMF (500 µL). HATU (26 mg, 0.068 mmol), DIPEA (23.5 µL, 0.136 mmol) and Drug toxin MMAF (10 mg, 0.0136 mmol) were added and the mixture was stirred at room temperature for 1 h. The reaction was subjected to semi-preparative separation and purification, and lyophilized to obtain a white powder D7 (yield 82%). HRMS, calculated for C₅₃H₈₉N₅O₁₆: [M+H]⁺1052.6383, observed 1052.6354.

### Preparation Example 20: Synthesis of Compound D-8

**Structure and synthesis method of Compound D-8 are as follows:**

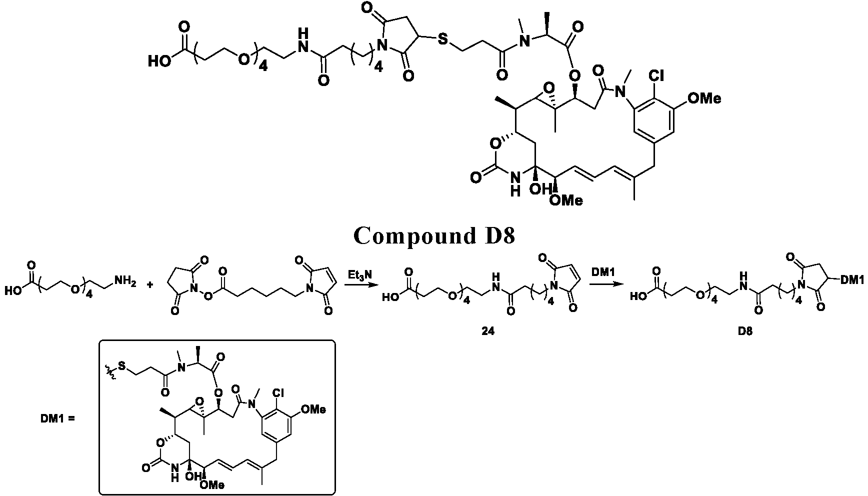

Step 1: HOOC-PEG₄-NH₂ (26.5 mg, 0.1 mmol) was weighed and dissolved in DMF (400 µL). N-Succinimidyl 6-maleimidohexanoate (30.8 mg, 0.1 mmol) and triethylamine (41.6 µL, 0.3 mmol) were added and the mixture was stirred at room temperature for 1 h. When the reaction was completed as monitored by LC-MS, the reaction was subjected to semi-preparative separation and purification, and lyophilized to obtain a white powder i.e., Compound **24** (yield 86%). HRMS, calculated for C₂₀H₃₂N₂O₁₀: [M+H]⁺461.2135, observed 461.2169.

Step 2: Compound **24** (10 mg, 0.022 mmol) was weighed and dissolved in PB 7.4 (500 µL). Cytotoxin DM1 (16 mg, 0.022 mmol) was added and the mixture was stirred at room temperature for 1 h. When the reaction was completed as monitored by LC-MS, the reaction was subjected to semi-preparative separation and purification, and lyophilized to obtain Compound **D8** (yield 87%). HRMS, calculated for C₅₅H₈₀ClN₅O₂₀S: [M+H]⁺1198.4884, observed 1198.4798.

### IV: Preparation of Cleavable Fragment Directed By Affinity Fragment

### Example 1: Synthesis of Compound ATC-1

**Structure and synthesis method of Compound ATC-1 are as follows:**

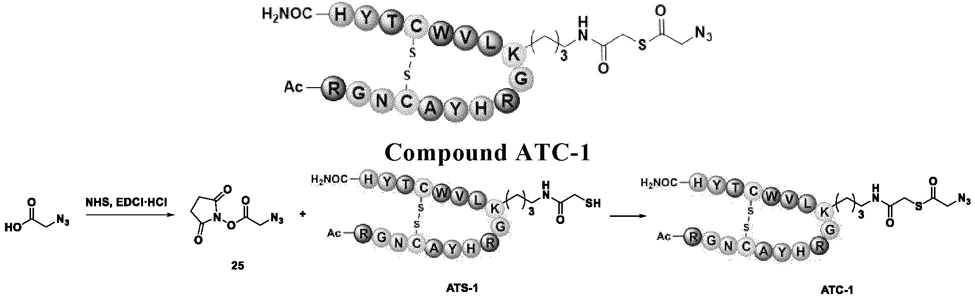

Step 1: Azidoacetic acid (250mg, 2.5 mmol) was weighed and dissolved in THF (5 mL). N-hydroxysuccinimide (288 mg, 2.5 mmol) was added. After stirring at 0 °C for 10 min, dicyclohexylcarbodiimide (516 mg, 2.5 mmol) was added, and the reaction solution was stirred at 0 °C for 4 h. After removing DCU by filtration, the filtrate was collected, and diethyl ether (20 mL) was added for recrystallization, and the mixture was allowed to stand at 4 °C overnight. After filtration, the filter cake was washed with THF (40 mL) and dried in vacuo to obtain a white powder, i.e., Compound **25** (yield 82%). ¹H NMR (600 MHz, DMSO-*d*₆) δ 4.71 (s, 2H), 2.84 (s, 4H). ¹³C NMR (151 MHz, DMSO) δ 169.91, 165.51, 47.50, 25.50.

Step 2: Compound **ATS-1** (7 mg, 3.2 µmol) was weighed and dissolved in a mixed solution of PB 7.4/DMF=1/1. Compound **25** (0.77 mg, 3.86 µmol) was added and the mixture was stirred at room temperature for 15min. When the reaction was completed as monitored by LC-MS, the reaction was subjected to semi-preparative separation and purification, and lyophilized to obtain a white powder i.e., Compound **ATC-1** (yield 71%). HRMS, calculated for C₉₇H₁₃₈N₃₄O₂₄S₃: [M+H]⁺ 2259.9861, [M+3H]³⁺ 754.0006, [M+4H]⁴⁺ 565.7524, observed 753.9815, 565.7080.

### Example 2: Synthesis of Compound ATC-2

**Structure and synthesis method of Compound ATC-2 are as follows:**

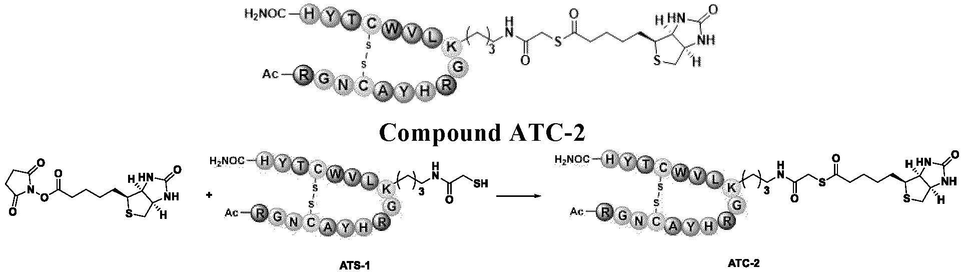

Compound **ATS-1** (7 mg, 3.2 µmol) was weighed and dissolved in a mixed solution of PB 7.4/DMF=1/1. Biotin-N-succinimidyl ester (1.32 mg, 3.86 µmol) was added and the mixture was stirred at room temperature for 15min. When the reaction was completed as monitored by LC-MS, the reaction was subjected to semi-preparative separation and purification, and lyophilized to obtain Compound **ATC-2** (yield 77%). HRMS, calculated for C₁₀₅H₁₅₁N₃₃O₂₅S₄: [M+H]⁺ 2403.0520, [M+3H]³⁺ 801.6892, [M+4H]⁴⁺601.5188, observed 801.6801, 601.5139.

### Example 3: Synthesis of Compound ATC-3

**Structure and synthesis method of Compound ATC-3 are as follows:**

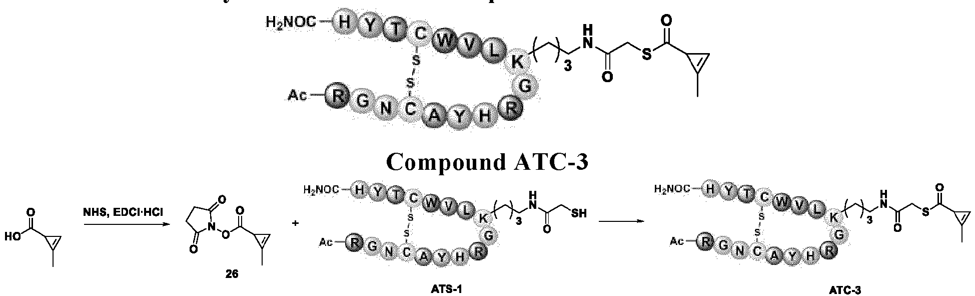

Step 1: 2-Methyl-2-cyclopropenecarboxylic acid (0.4 mg, 4 µmol) was weighed and dissolved in DMF (50 µL). N-hydroxysuccinimide (0.69 mg, 6 µmol) and 1-(3-dimethylaminopropyl)-3-ethylcarbodiimide hydrochloride (1.15 mg, 6 µmol) were added and the mixture was reacted at room temperature overnight to obtain crude product of Compound **26.**

Step 2: Compound **ATS-1** (7 mg, 3.2 µmol) was weighed and dissolved in DMF (50 µL), and added into the reaction solution obtained in step 1. PB 7.4 buffer (200 µL) was then added and the mixture was stirred at room temperature for 30 min. When the reaction was completed as monitored by LC-MS, the reaction was subjected to semi-preparative separation and purification, and lyophilized to obtain a white powder i.e., Compound **ATC-3** (yield 67%). HRMS, calculated for C₁₀₀H₁₄₁N₃₁O₂₄S₃: [M+H]⁺ 2257.0006, [M+3H]³⁺ 753.0054, [M+4H]⁴⁺ 565.0060, observed 753.0013, 565.0059.

### Example 4: Synthesis of Compound ATC-4

**Structure and synthesis method of Compound ATC-4 are as follows:**

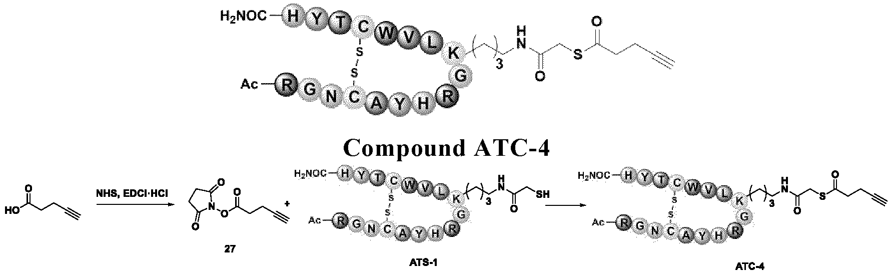

Step 1: Pentynoic acid (0.4 mg, 4 µmol) was weighed and dissolved in DMF (50 µL). N-hydroxysuccinimide (0.69 mg, 6 µmol) and 1-(3-dimethylaminopropyl)-3-ethylcarbodiimide hydrochloride (1.15 mg, 6 µmol) were added and the mixture was reacted at room temperature overnight to obtain crude product of Compound **27.**

Step 2: Compound **ATS-1** (7 mg, 3.2 µmol) was weighed and dissolved in DMF (50 µL), and added into the reaction solution obtained in step 1. PB 7.4 buffer (200 µL) was then added and the mixture was stirred at room temperature for 30 min. When the reaction was completed as monitored by LC-MS, the reaction was subjected to semi-preparative separation and purification, and lyophilized to obtain a white powder i.e., Compound **ATC-4** (yield 59%). HRMS, calculated for C₁₀₀H₁₄₁N₃₁O₂₄S₃: [M+H]⁺ 2257.0006, [M+3H]³⁺ 753.0054, [M+4H]⁴⁺ 565.0060, observed 753.0051, 565.0086.

### Example 5: Synthesis of Compound ATC-5

**Structure and synthesis method of Compound ATC-5 are as follows:**

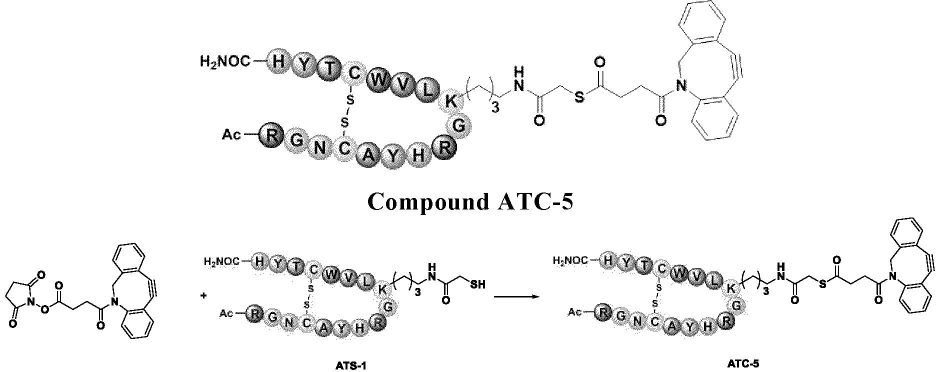

Compound **ATS-1** (7 mg, 3.2 µmol) was weighed and dissolved in a mixed solution of PB 7.4/DMF=1/1. DBCO-NHS (1.55 mg, 3.86 µmol) was added and the mixture was stirred at room temperature for 15min. When the reaction was completed as monitored by LC-MS, the reaction was subjected to semi-preparative separation and purification, and lyophilized to obtain Compound **ATC-6** (yield 68%). HRMS, calculated for C₁₁₄H₁₅₀N₃₂O₂₅S₃: [M+H]⁺ 2464.0690, [M+3H]³⁺ 822.0282, [M+4H]⁴⁺ 616.7731, observed 822.0264, 616.7715.

### Example 6: Synthesis of Compound ATC-6

**Structure and synthesis method of Compound ATC-6 are as follows:**

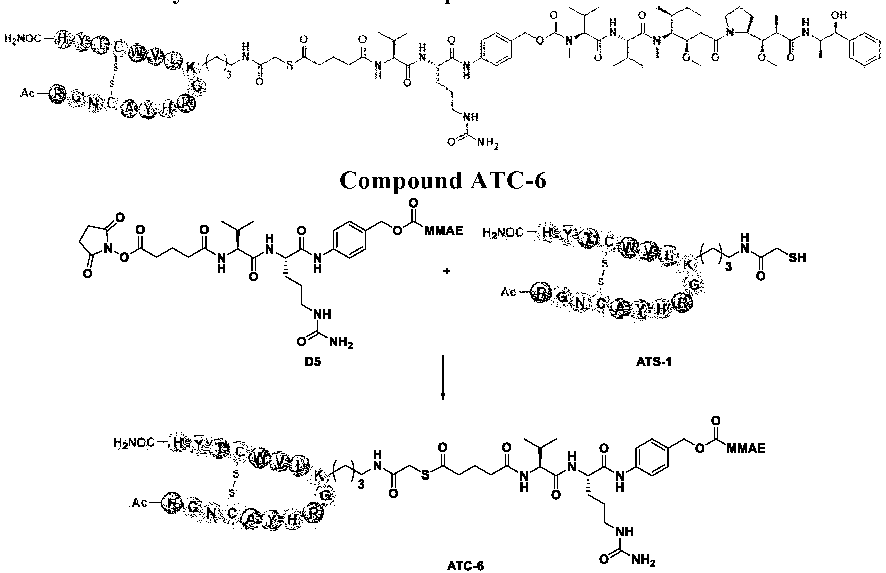

Compound **D5** (13.3 mg, 0.01 mmol) was weighed and dissolved in a mixed solution of DMF/PB 7.4 =1/1. Compound **ATS-1** was added. After reacting at room temperature for 30min, the reaction was subjected to semi-preparative separation and purification, and lyophilized to obtain a white powder i.e., Compound **ATC-6** (yield 76%). HRMS, calculated for C₁₅₈H₂₃₅N₄₁O₃₇S₃: [M+H]⁺3395.7007, [M+3H]³⁺1132.5721, [M+4H]⁴⁺ 849.6810, observed 1133.5587, 849.6596.

### Example 7: Synthesis of Compound ATC-7

**Structure and synthesis method of Compound ATC-7 are as follows:**

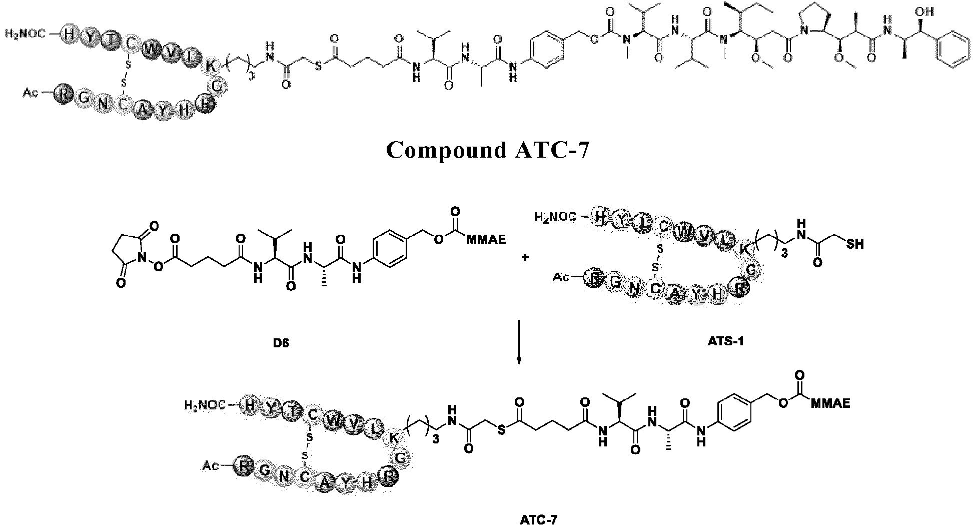

Compound **D6** (12.5 mg, 0.01 mol) was weighed and dissolved in a mixed solution of DMF/PB 7.4=1/1. Compound **ATS-1** was added. After reacting at room temperature for 30min, the reaction was subjected to semi-preparative separation and purification, and lyophilized to obtain Compound **ATC-7** (yield 81%). HRMS, calculated for C₁₅₅H₂₂₉N₃₉O₃₆S₃: [M+H]⁺3309.6526, [M+3H]³⁺1103.8894, [M+4H]⁴⁺ 828.1690, observed 1103.8900, 828.1674.

### Example 8: Synthesis of Compound ATC-8

**Structure and synthesis method of Compound ATC-8 are as follows:**

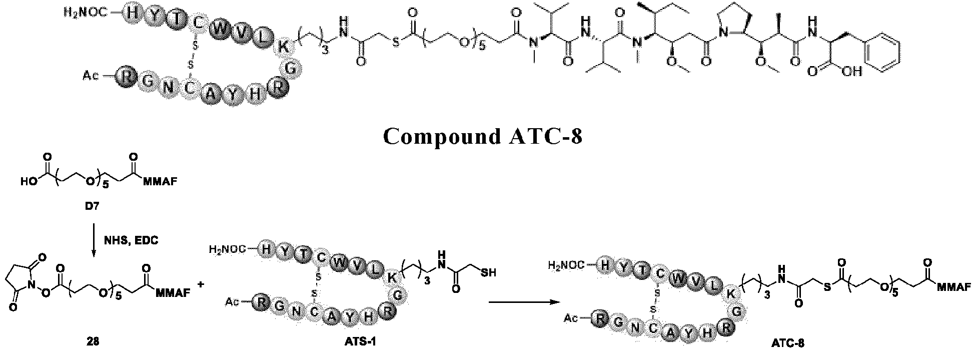

Compound **D7** (10.5 mg, 0.01 mmol) was weighed and dissolved in DMF. NHS (1.4 mg, 0.012 mmol) and EDC (2.1 µL, 0.012 mmol) were added and the mixture was reacted at room temperature overnight. A solution of compound **ATS-1** in DMF was added to the reaction solution, and an equal volume of 0.2M PB 7.4 buffer was added at the same time. After reacting at room temperature for 2 h, the reaction was subjected to semi-preparative separation and purification, and lyophilized to obtain Compound **ATC-8** (yield 77%). HRMS, calculated for C₁₄₈H₂₂₄N₃₆O₃₈S₃: [M+H]⁺3210.5941, [M+3H]³⁺1070.8699, [M+4H]⁴⁺ 803.4044, observed 1070.4196, 803.9044.

### Example 9: Synthesis of Compound ATC-9

**Structure and synthesis method of Compound ATC-9 are as follows:**

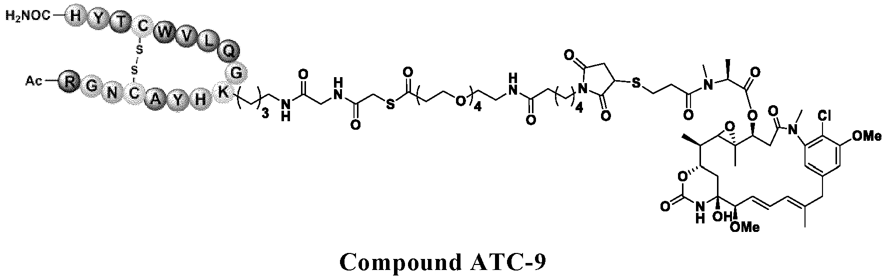

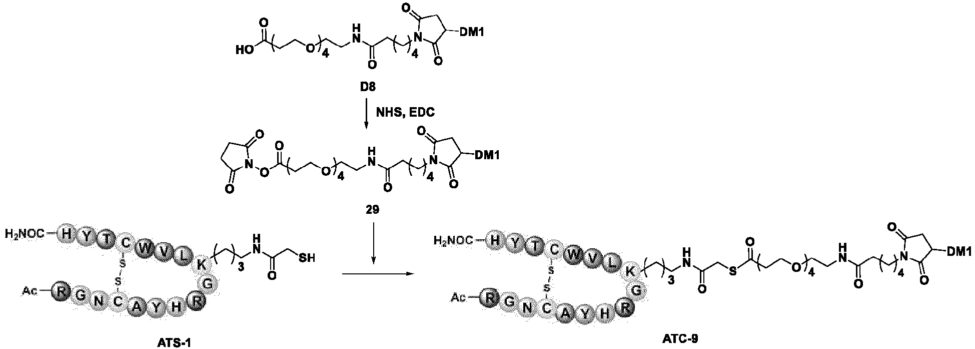

**D8** (12 mg, 0.01 mmol) was weighed and dissolved in DMF. NHS (1.4 mg, 0.012 mmol) and EDC (2.1 µL, 0.012 mmol) were added. After reacting at room temperature overnight, a solution of **ATS-1** in DMF was added, and an equal volume of 0.2M PB 7.4 buffer was then added. After reacting at room temperature for 2 h, the reaction was subjected to semi-preparative separation and purification, and lyophilized to obtain a white powder i.e., Compound **ATC-9** (yield 72%). HRMS, calculated for C₁₅₀H₂₁₅ClN₃₆O₄₂S₄: [M+H]⁺3356.4445, [M+3H]³⁺1119.4867, [M+4H]⁴⁺839.8669, observed 1119.4882, 839.8637.

### Example 10: Synthesis of Compound ATC-10

**Structure and synthesis method of Compound ATC-10 are as follows:**

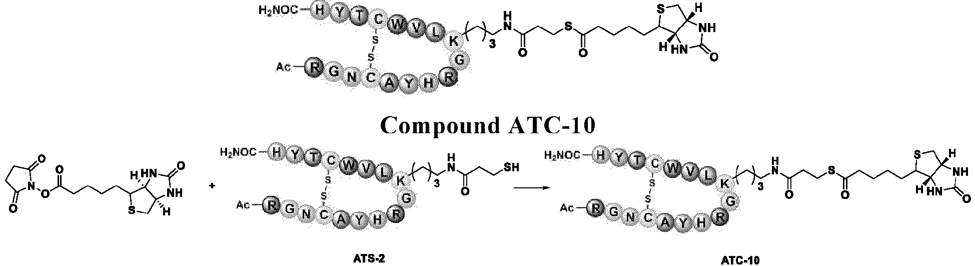

Compound **ATS-2** (7 mg, 3.2 µmol) was weighed and dissolved in a mixed solution of PB 7.4/DMF=1/1. Biotin-N-succinimidyl ester (1.32 mg, 3.86 □µmol) was added and the mixture was stirred at room temperature for 15min. When the reaction was completed as monitored by LC-MS, the reaction was subjected to semi-preparative separation and purification, and lyophilized to obtain Compound **ATC-10** (yield 79%). HRMS, calculated for C₁₀₆H₁₅₃N₃₃O₂₅S₄: [M+H]⁺ 2417.0676, [M+3H]³⁺806.3611, [M+4H]⁴⁺ 605.0228, observed 806.3648, 605.0256.

### Example 11: Synthesis of Compound ATC-11

**Structure and synthesis method of Compound ATC-11 are as follows:**

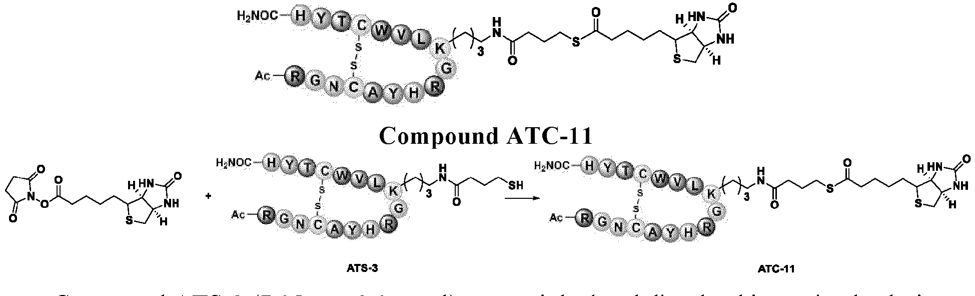

Compound **ATS-3** (7.05 mg, 3.2 µmol) was weighed and dissolved in a mixed solution of PB 7.4/DMF=1/1. Biotin-N-succinimidyl ester (1.32 mg, 3.86 µmol) was added and the mixture was stirred at room temperature for 15min. When the reaction was completed as monitored by LC-MS, the reaction was subjected to semi-preparative separation and purification, and lyophilized to obtain Compound ATC-11 (yield 67%). HRMS, calculated for C₁₀₇H₁₅₅N₃₃O₂₅S₄: [M+H]⁺2431.0833, [M+3H]³⁺811.0330, [M+4H]⁴⁺608.5267, observed 811.0325, 608.5286.

### Example 12: Synthesis of Compound ATC-12

**Structure and synthesis method of Compound ATC-12 are as follows:**

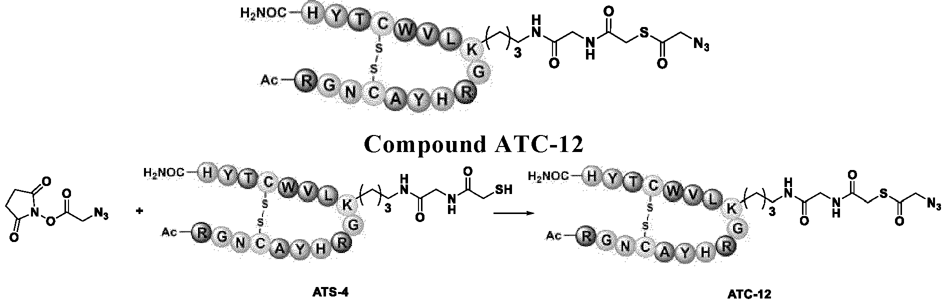

Compound **ATS-4** (7.05 mg, 3.2 µmol) was weighed and dissolved in a mixed solution of PB 7.0/DMF=1/1. Compound **25** (0.77 mg, 3.86 µmol) was added and the mixture was stirred at room temperature for 15min. When the reaction was completed as monitored by LC-MS, the reaction was subjected to semi-preparative separation and purification, and lyophilized to obtain a white powder i.e., Compound **ATC-12** (yield 89%). HRMS, calculated for C₉₉H₁₄₁N₃₅O₂₅S₃: [M+H]⁺ 2317.0078, [M+3H]³⁺ 773.0078, [M+4H]⁴⁺ 580.0078, observed 773.0123, 580.0071.

### Example 13: Synthesis of Compound ATC-13

**Structure and synthesis method of Compound ATC-13 are as follows:**

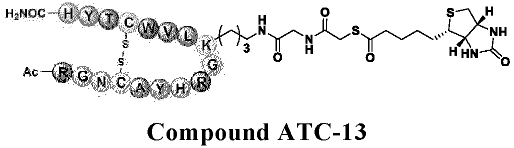

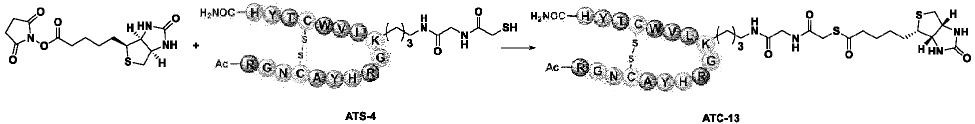

Compound **ATS-4** (7.05 mg, 3.2 µmol) was weighed and dissolved in a mixed solution of PB 7.4/DMF=1/1. Biotin-N-succinimidyl ester (1.32 mg, 3.86 µmol) was added and the mixture was stirred at room temperature for 15min. When the reaction was completed as monitored by LC-MS, the reaction was subjected to semi-preparative separation and purification, and lyophilized to obtain Compound **ATC-13** (yield 77%). HRMS, calculated for C₁₀₇H₁₅₄N₃₄O₂₆S₄: [M+H]⁺2460.0734, [M+3H]³⁺820.6963, [M+4H]⁴⁺ 615.7742, observed 820.7012, 615.7726.

### Example 14: Synthesis of Compound ATC-14

**Structure and synthesis method of Compound ATC-14 are as follows:**

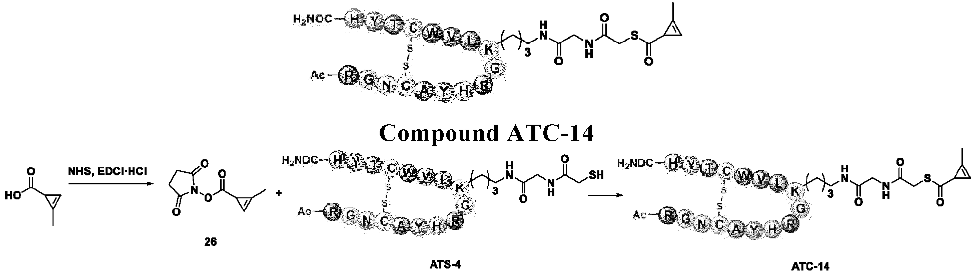

Step 1: 2-Methyl-2-cyclopropenecarboxylic acid (0.4 mg, 4 µmol) was weighed and dissolved in DMF (50 µL). N-hydroxysuccinimide (0.69 mg, 6 µmol) and 1-(3-dimethylaminopropyl)-3-ethylcarbodiimide hydrochloride (1.15 mg, 6 µ□mol) were added and the mixture was reacted at room temperature overnight to obtain crude product of Compound **26.**

Step 2: Compound **ATS-4** (7.05 mg, 3.2 µmol) was weighed and dissolved in DMF (50 µL), and added into the reaction solution obtained in step 1. PB 7.4 buffer (200 □µL) was then added and the mixture was stirred at room temperature for 30 min. When the reaction was completed as monitored by LC-MS, the reaction was subjected to semi-preparative separation and purification, and lyophilized to obtain a white powder i.e., Compound **ATC-14** (yield 59%). HRMS, calculated for C₁₀₂H₁₄₄N₃₂O2₅S₃: [M+H]⁺ 2314.0221, [M+3H]³⁺ 772.0126, [M+4H]⁴⁺ 579.2614, observed 772.0148, 579.2658.

### Example 15: Synthesis of Compound ATC-15

**Structure and synthesis method of Compound ATC-15 are as follows:**

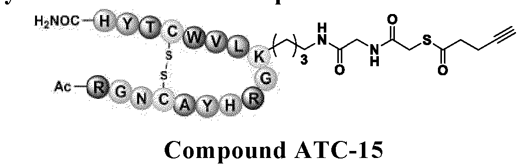

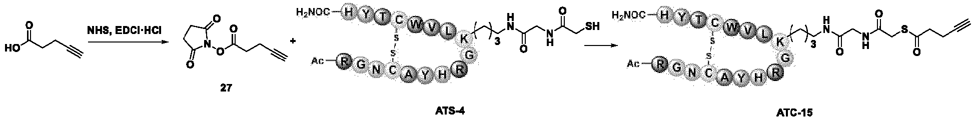

Step 1: Pentynoic acid (0.4 mg, 4 µmol) was weighed and dissolved in DMF (50 µL). N-hydroxysuccinimide (0.69 mg, 6 µmol) and 1-(3-dimethylaminopropyl)-3-ethylcarbodiimide hydrochloride (1.15 mg, 6 µmol) were added and the mixture was reacted at room temperature overnight to obtain crude product of Compound **27.**

Step 2: Compound **ATS-4** (7.05 mg, 3.2 µmol) was weighed and dissolved in DMF (50 µL), and added into the reaction solution obtained in step 1. PB 7.4 buffer (200 µL) was then added and the mixture was stirred at room temperature for 30 min. When the reaction was completed as monitored by LC-MS, the reaction was subjected to semi-preparative separation and purification, and lyophilized to obtain a white powder i.e., Compound **ATC-4** (yield 66%). HRMS, calculated for C₁₀₂H₁₄₄N₃₂O2₅S₃: [M+H]⁺ 2314.0221, [M+3H]³⁺ 772.0126, [M+4H]⁴⁺ 579.2614, observed 772.0148, 579.2594.

### Example 16: Synthesis of Compound ATC-16

**Structure and synthesis method of Compound ATC-16 are as follows:**

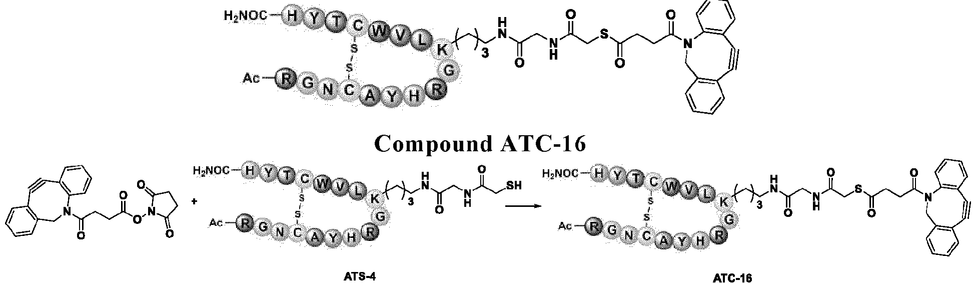

Compound **ATS-4** (7.05 mg, 3.2 µmol) was weighed and dissolved in a mixed solutionof PB 7.4/DMF=1/1. DBCO-NHS (1.55 mg, 3.86 µmol) was added and the mixture was stirred at room temperature for 15min. When the reaction was completed as monitored by LC-MS, the reaction was subjected to semi-preparative separation and purification, and lyophilized to obtain Compound **ATC-16** (yield 68%). HRMS, calculated for C₁₁₆H₁₅₃N₃₃O₂₆S₃: [M+H]⁺ 2521.0905, [M+3H]³⁺ 841.0354, [M+4H]⁴⁺ 631.0285, observed 841.0389, 631.0268.

### Example 17: Synthesis of Compound ATC-17

**Structure and synthesis method of Compound ATC-17 are as follows:**

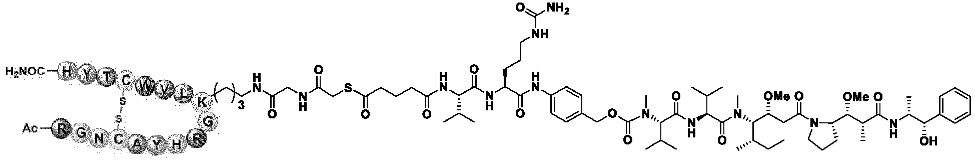

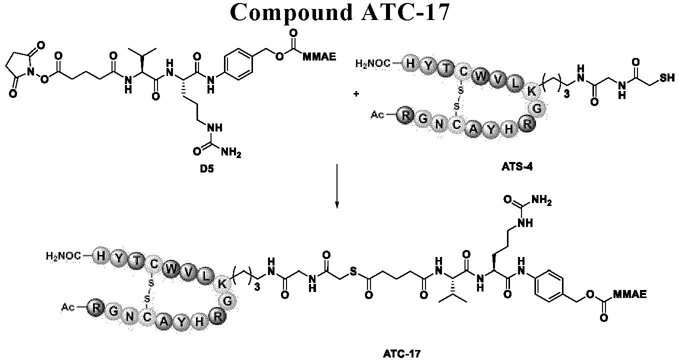

Compound **D5** (13.3 mg, 0.01 mol) was weighed and dissolved in a mixed solution of DMF/PB 7.4=1/1. Compound **ATS-4** was added. After reacting at room temperature for 30min, the reaction was subjected to semi-preparative separation and purification, and lyophilized to obtain a white powder i.e., Compound ATC-17 (yield 73%). HRMS, calculated for C₁₅₉H₂₃₆N₄₂O₃₈S₃: [M+H]⁺ 3438.7066, [M+3H]³⁺ 1146.9074, [M+4H]⁴⁺ 860.4325, observed 860.4365.

### Example 18: Synthesis of Compound ATC-18

**Structure and synthesis method of Compound ATC-18 are as follows:**

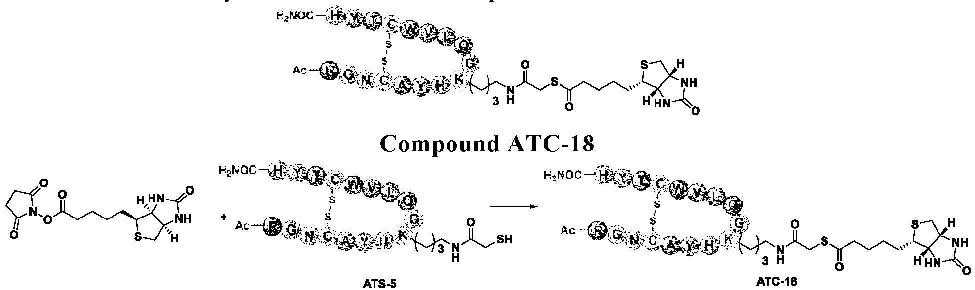

Compound **ATS-5** (6.9 mg, 3.2 µmol) was weighed and dissolved in a mixed solution of PB 7.4/DMF=1/1. Biotin-N-succinimidyl ester (1.32 mg, 3.86 µmol) was added and the mixture was stirred at room temperature for 20min. When the reaction was completed as monitored by LC-MS, the reaction was subjected to semi-preparative separation and purification, and lyophilized to obtain Compound **ATC-2** (yield 77%). HRMS, calculated for C₁₀₄H₁₄₇N₃₁O₂₆S₄: [M+H]⁺2375.0094, [M+3H]³⁺792.3417, [M+4H]⁴⁺ 594.5082, observed 792.3395, 594.5079.

### Example 19: Synthesis of Compound ATC-19

**Structure and synthesis method of Compound ATC-19 are as follows:**

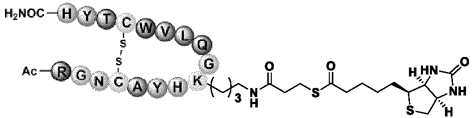

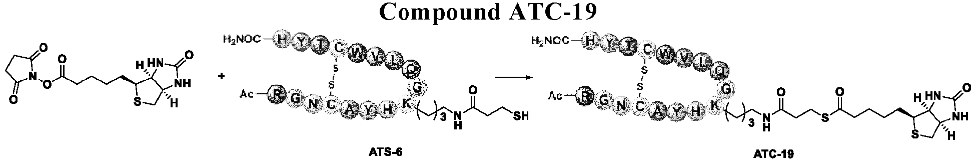

Compound **ATS-6** (6.9 mg, 3.2 µmol) was weighed and dissolved in a mixed solution of PB 7.4/DMF=1/1. Biotin-N-succinimidyl ester (1.32 mg, 3.86 µmol) was added and the mixture was stirred at room temperature for 15min. When the reaction was completed as monitored by LC-MS, the reaction was subjected to semi-preparative separation and purification, and lyophilized to obtain Compound **ATC-19** (yield 76%). HRMS, calculated for C₁₀₅H₁₄₉N₃₁O₂₆S₄: [M+H]⁺2389.0251, [M+3H]³⁺797.0136, [M+4H]⁴⁺ 598.0121, observed 797.0178, 598.0135.

### Example 20: Synthesis of Compound ATC-20

**Structure and synthesis method of Compound ATC-20 are as follows:**

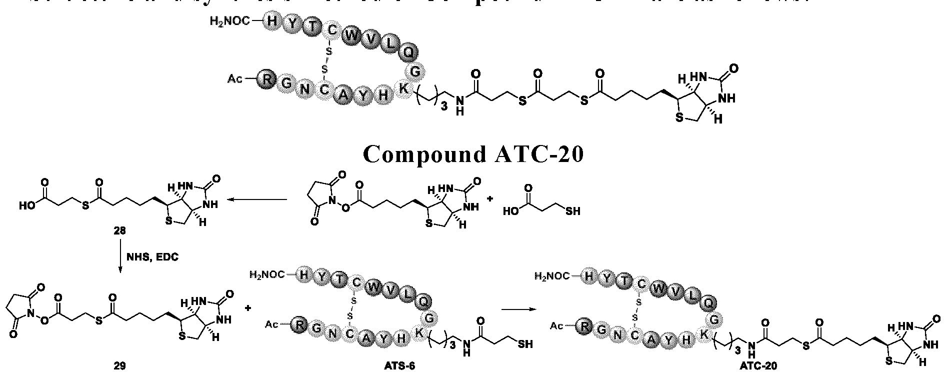

Step 1: Mercaptopropionic acid (26.5 mg, 0.25 mmol) was weighed and dissolved in a mixed solution of PB 7.4/DMF=1/1. Biotin-N-succinimidyl ester (17 mg, 0.05 mmol) was added and the mixture was stirred at room temperature overnight. the reaction was completed as monitored by LC-MS, the reaction was subjected to semi-preparative separation and purification, and lyophilized to obtain Compound **28** (yield 53%).

Step 2: Compound **28** (1.33 mg, 4 µmol) was weighed and dissolved in DMF (50 µL). N-hydroxysuccinimide (0.69 mg, 6 µmol) and 1-(3-dimethylaminopropyl)-3-ethylcarbodiimide hydrochloride (1.15 mg, 6 µmol) were added and the mixture was reacted at room temperature overnight to obtain crude product of Compound **29.**

Step 3: Compound **ATS-6** (6.9 mg, 3.2 µmol) was weighed and dissolved in DMF (50 □µL), and added into the reaction solution obtained in step 2. PB 7.4 buffer (200 µL) was then added and the mixture was stirred at room temperature for 1 h. When the reaction was completed as monitored by LC-MS, the reaction was subjected to semi-preparative separation and purification, and lyophilized to obtain a white powder i.e., Compound **ATC-20** (yield 75%). HRMS, calculated for C₁₀₈H₁₅₃N₃₁O₂₇S₅: [M+H]⁺ 2477.0234, [M+3H]³⁺ 826.3463, [M+4H]⁴⁺ 620.0117, observed 826.3457, 619.9915.

### Example 21: Synthesis of Compound ATC-21

**Structure and synthesis method of Compound ATC-21 are as follows:**

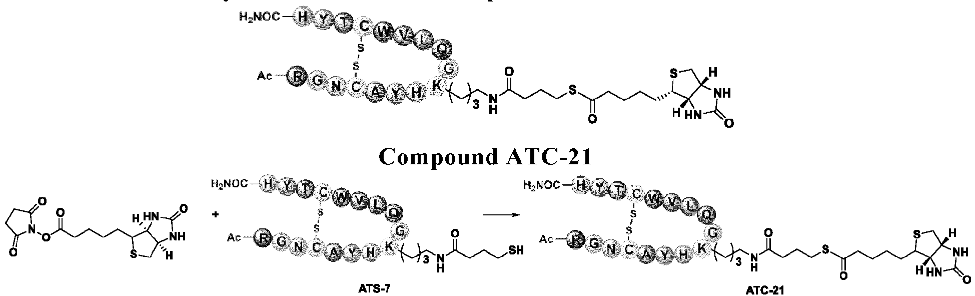

Compound **ATS-7** (7 mg, 3.2 µmol) was weighed and dissolved in a mixed solution of PB 7.4/DMF=1/1. Biotin-N-succinimidyl ester (1.32 mg, 3.86 µmol) was added and the mixture was stirred at room temperature for 15min. When the reaction was completed as monitored by LC-MS, the reaction was subjected to semi-preparative separation and purification, and lyophilized to obtain Compound **ATC-21** (yield 74%). HRMS, calculated for C₁₀₆H₁₅₁N₃₁O₂₆S₄: [M+H]⁺2403.0407, [M+3H]³⁺801.6854, [M+4H]⁴⁺ 601.5160, observed 801.6794, 601.5172.

### Example 22: Synthesis of Compound ATC-22

**Structure and synthesis method of Compound ATC-22 are as follows:**

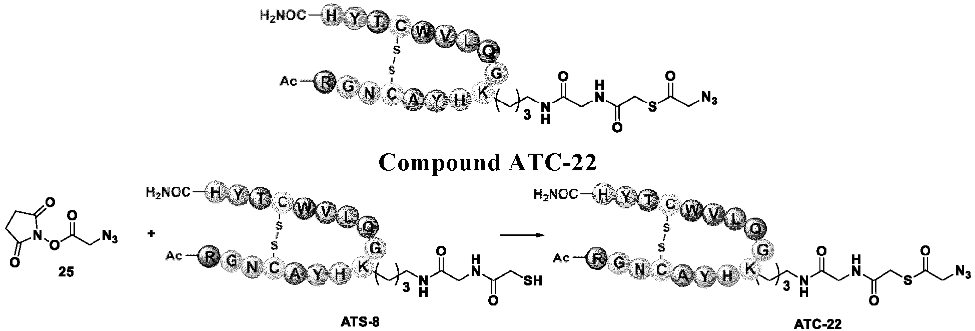

Compound **ATS-8** (7 mg, 3.2 µmol) was weighed and dissolved in a mixed solution of PB 7.0/DMF=1/1. Compound **25** (0.77 mg, 3.86 µmol) was added and the mixture was stirred at room temperature for 15min. When the reaction was completed as monitored by LC-MS, the reaction was subjected to semi-preparative separation and purification, and lyophilized to obtain a white powder i.e., Compound **ATC-22** (yield 91%). HRMS, calculated for C₉₈H₁₃₇N₃₃O₂₆S₃: [M+H]⁺2288.9653, [M+3H]³⁺763.6603, [M+4H]⁴⁺ 572.9971, observed 763.6584, 571.9829.

### Example 23: Synthesis of Compound ATC-23

**Structure and synthesis method of Compound ATC-23 are as follows:**

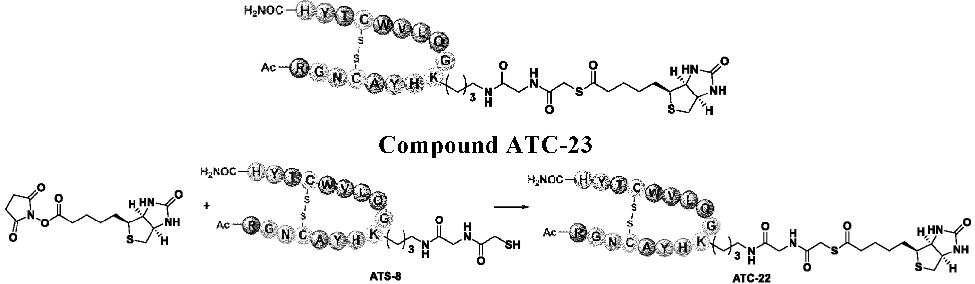

Compound **ATS-8** (7 mg, 3.2 µmol) was weighed and dissolved in a mixed solution of PB 7.4/DMF=1/1. Biotin-N-succinimidyl ester (1.32 mg, 3.86 µmol) was added and the mixture was stirred at room temperature for 15min. When the reaction was completed as monitored by LC-MS, the reaction was subjected to semi-preparative separation and purification, and lyophilized to obtain Compound **ATC-23** (yield 81%). HRMS, calculated for C₁₀₆H₁₅₀N₃₂O₂₇S₄: [M+H]⁺2432.0309, [M+3H]³⁺811.3488, [M+4H]⁴⁺608.7636, observed 811.2514, 608.7618.

### Example 24: Synthesis of Compound ATC-24

**Structure and synthesis method of Compound ATC-24 are as follows:**

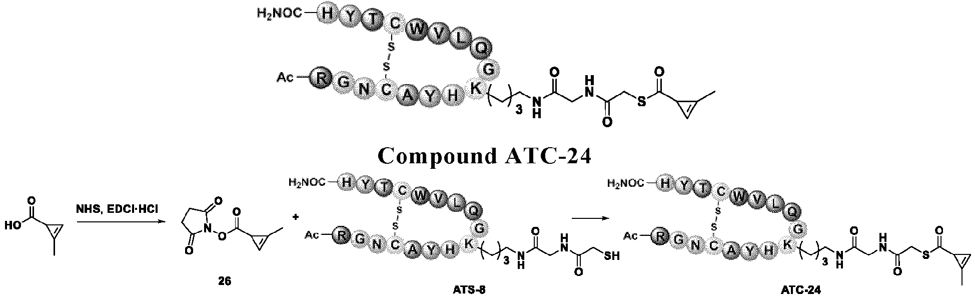

Step 1: 2-Methyl-2-cyclopropenecarboxylic acid (0.4 mg, 4 µmol) was weighed and dissolved in DMF (50 µL). N-hydroxysuccinimide (0.69 mg, 6 µmol) and 1-(3-dimethylaminopropyl)-3-ethylcarbodiimide hydrochloride (1.15 mg, 6 µmol) were added and the mixture was reacted at room temperature overnight to obtain crude product of Compound **26.**

Step 2: Compound **ATS-8** (7 mg, 3.2 µmol) was weighed and dissolved in DMF (50 µL), and added into the reaction solution obtained in step 1. PB 7.4 buffer (200 µL) was then added and the mixture was stirred at room temperature for 30 min. When the reaction was completed as monitored by LC-MS, the reaction was subjected to semi-preparative separation and purification, and lyophilized to obtain a white powder i.e., Compound **ATC-24** (yield 64%). HRMS, calculated for C₁₀₁H₁₄₀N₃₀O₂₆S₃: [M+H]⁺ 2285.9795, [M+3H]³⁺ 762.6650, [M+4H]⁴⁺ 572.2507, observed 762.6612, 572.2547.

### Example 25: Synthesis of Compound ATC-25

**Structure and synthesis method of Compound ATC-25 are as follows:**

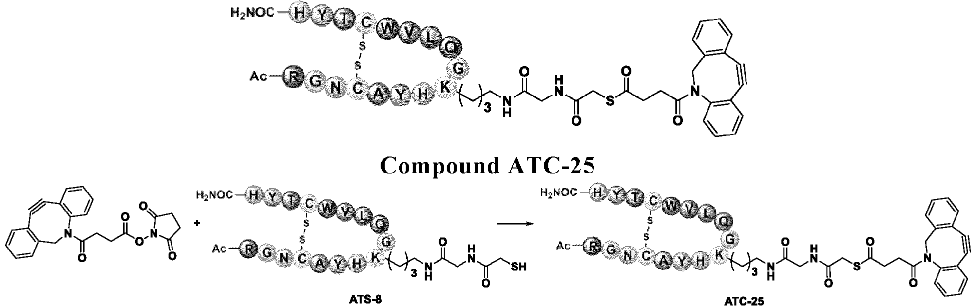

Compound **ATS-8** (7 mg, 3.2 µmol) was weighed and dissolved in a mixed solution of PB 7.4/DMF=1/1. DBCO-NHS (1.55 mg, 3.86 µmol) was added and the mixture was stirred at room temperature for 15min. When the reaction was completed as monitored by LC-MS, the reaction was subjected to semi-preparative separation and purification, and lyophilized to obtain Compound **ATC-25** (yield 65%). HRMS, calculated for C₁₁₅H₁₄₉N₃₁O₂₇S₃: [M+H]⁺2493.0479, [M+3H]³⁺831.6878, [M+4H]⁴⁺624.0178, observed 831.6894, 624.0189.

### Example 26: Synthesis of Compound ATC-26

**Structure and synthesis method of Compound ATC-26 are as follows:**

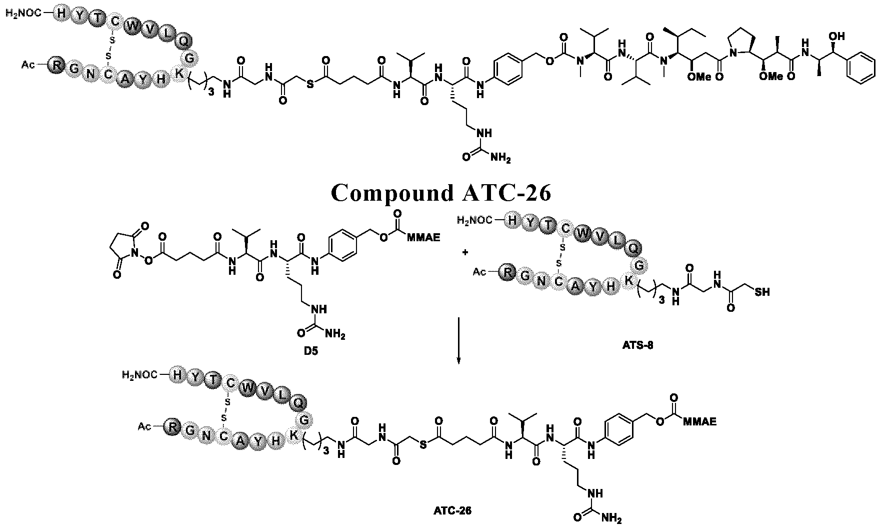

Compound **D5** (13.3 mg, 0.01 mol) was weighed and dissolved in a mixed solution of DMF/PB 7.4=1/1. Compound **ATS-8** was added. After reacting at room temperature for 30min, the reaction was subjected to semi-preparative separation and purification, and lyophilized to obtain a white powder i.e., Compound **ATC-26** (yield 83%). HRMS, calculated for C₁₅₉H₂₃₄N₄₀O₃₉S₃: [M+H]⁺ 3424.6797, [M+3H]³⁺1142.2318, [M+4H]⁴⁺856.9258, observed 856.9314.

### Example 27: Synthesis of Compound ATC-27

**Structure and synthesis method of Compound ATC-27 are as follows:**

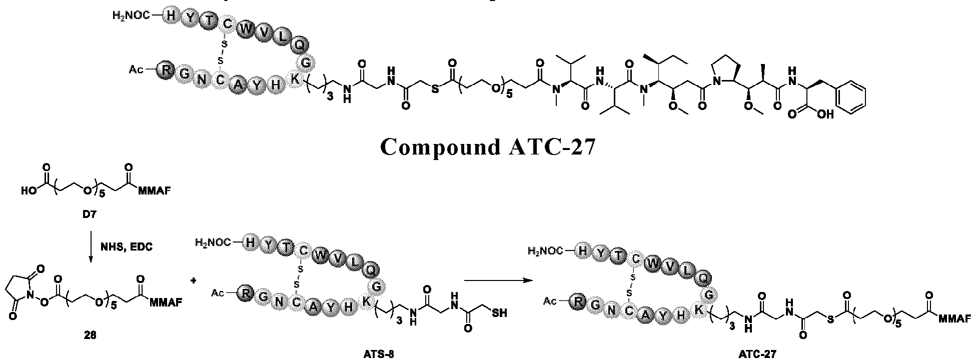

**D7** (10.5 mg, 0.01 mmol) was weighed and dissolved in DMF. NHS (1.4 mg, 0.012 mmol) and EDC (2.1 µL, 0.012 mmol) were added and the mixture was reacted at room temperature overnight. A solution of compound **ATS-8** in DMF was added to the reaction solution, and an equal volume of 0.2M PB 7.4 buffer was added at the same time. After reacting at room temperature for 2 h, the reaction was subjected to semi-preparative separation and purification, and lyophilized to obtain Compound **ATC-27** (yield 70%). HRMS, calculated for C₁₄₉H₂₂₃N₃₅O₄₀S₃: [M+H]⁺3239.5732, [M+3H]³⁺1080.5296, [M+4H]⁴⁺810.6492, observed 810.6554.

### Example 28: Synthesis of Compound ATC-28

**Structure and synthesis method of Compound ATC-28 are as follows:**

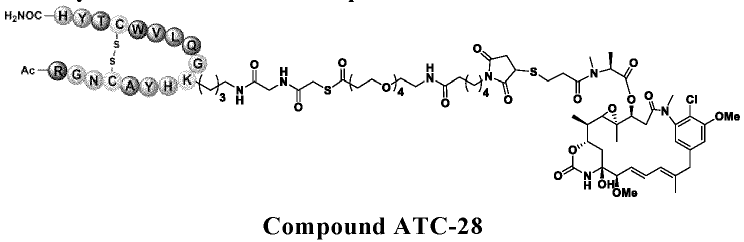

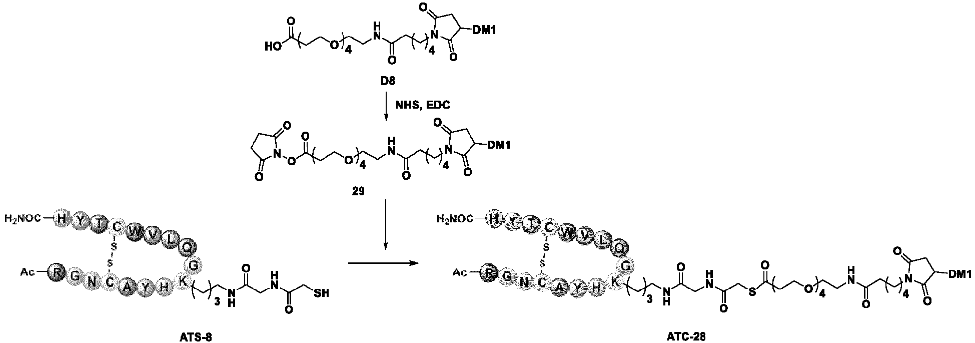

**D8** (12 mg, 0.01 mmol) was weighed and dissolved in DMF. NHS (1.4 mg, 0.012 mmol) and EDC (2.1 µL, 0.012 mmol) were added. After reacting at room temperature overnight, a solution of **ATS-8** in DMF was added, and an equal volume of 0.2M PB 7.4 buffer was then added. After reacting at room temperature for 2 h, the reaction was subjected to semi-preparative separation and purification, and lyophilized to obtain a white powder i.e., Compound **ATC-28** (yield 73%). HRMS, calculated for C₁₄₉H₂₁₀ClN₃₅O₄₃S₄: [M+H]⁺3341.3971, [M+3H]³⁺1114.4709, [M+4H]⁴⁺836.1051, observed 836.1095.

### Example 29: Synthesis of Compound ATC-29

**Structure and synthesis method of Compound ATC-29 are as follows:**

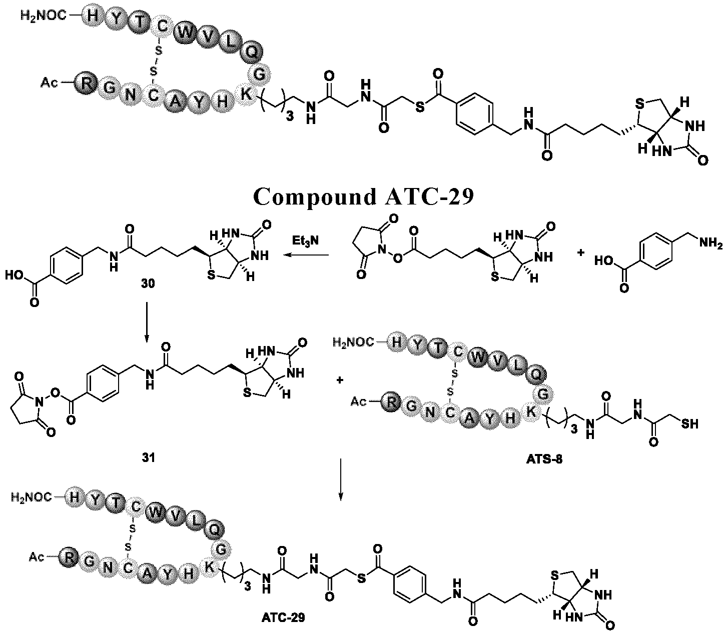

Step 1: 4-(aminomethyl)benzoic acid (9 mg, 0.06 mmol) was weighed and dissolved in DMF. Biotin-N-succinimidyl ester (17 mg, 0.05 mmol) and triethylamine (20 µL, 0.15 mmol) were added and the mixture was stirred at room temperature overnight. When the reaction was completed as monitored by LC-MS, the reaction was subjected to semi-preparative separation and purification, and lyophilized to obtain Compound **30** (yield 91%).

Step 2: Compound **30** (1.33 mg, 4 µmol) was weighed and dissolved in DMF (50 µL). N-hydroxysuccinimide (0.69 mg, 6 µmol) and 1-(3-dimethylaminopropyl)-3-ethylcarbodiimide hydrochloride (1.15 mg, 6 µmol) were added and the mixture was reacted at room temperature overnight to obtain crude product of Compound **31.**

Step 3: Compound **ATS-8** (7 mg, 3.2 µmol) was weighed and dissolved in DMF (50 µL), and added into the reaction solution obtained in step 2. PB 7.4 buffer (200 µL) was then added and the mixture was stirred at room temperature for 1h. When the reaction was completed as monitored by LC-MS, the reaction was subjected to semi-preparative separation and purification, and lyophilized to obtain a white powder i.e., Compound **ATC-29** (yield 74%). HRMS, calculated for C₁₁₄H₁₅₇N₃₃O₂₈S₄: [M+H]⁺ 2565.0837, [M+3H]³⁺ 855.6998, [M+4H]⁴⁺ 642.0268, observed 855.7043, 642.0276.

### Example 30: Synthesis of Compound ATC-30

**Structure and synthesis method of Compound ATC-30 are as follows:**

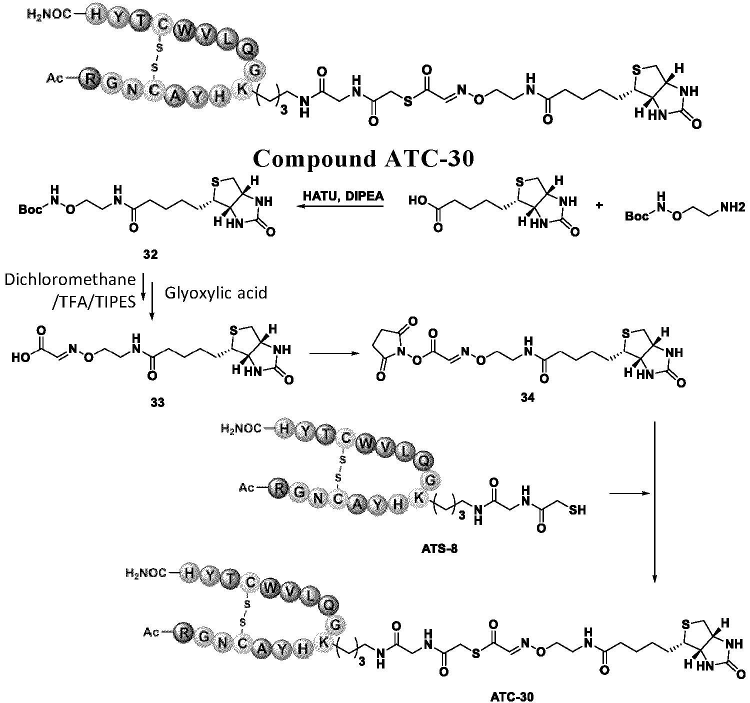

Step 1: Biotin (50 mg, 0.2 mmol) was weighed and dissolved in DMF. HATU (152 mg, 0.4 mmol), DIPEA (103 µL, 0.6 mmol) and tert-butyl (2-aminoethoxy)carbamate (35 mg, 0.2 mmol) were added and the mixture was reacted at room temperature for 2 h. When the reaction was completed as monitored by LC-MS, the reaction was subjected to semi-preparative separation and purification, and lyophilized to obtain Compound **32** (yield 85%). HRMS, calculated for C₁₇H₃₀N₄O₅S: [M+H]⁺403.2015, observed 403.2114.

Step 2: Compound **32** (20 mg, 0.05 mmol) was weighed and dissolved in 500 µL of dichloromethane. 450 µL of trifluoroacetic acid and 50 µL of triisopropylsilane were added under ice bath conditions. After reacting at room temperature for 1 h, the reaction was blown dry with N₂ for use.

Step 3: 500 µL of a mixed solution of acetonitrile/water/acetic acid = 9/9/2 was added to the crude product obtained in step 2. Glyoxylic acid (2.8 µL, 0.05 mmol) was added, and the mixture was reacted at room temperature for 15 min. The reaction was subjected to semi-preparative separation and purification, and lyophilized to obtain compound **33** (yield 92%). HRMS, calculated for C₁₄H₂₂N₄O₅S: [M+H]⁺359.1389, observed 359.1425.

Step 4: Compound **33** (1.43 mg, 4 µmol) was weighed and dissolved in DMF (50 µL). N-hydroxysuccinimide (0.69 mg, 6 µmol) and 1-(3-dimethylaminopropyl)-3-ethylcarbodiimide hydrochloride (1.15 mg, 6 µmol) were added and the mixture was reacted at room temperature overnight to obtain crude product of Compound **34.**

Step 5: Compound **ATS-8** (7 mg, 3.2 µmol) was weighed and dissolved in DMF (50 µL), and added into the reaction solution obtained in step 2. PB 7.4 buffer (200 µL) was then added and the mixture was stirred at room temperature for 1h. When the reaction was completed as monitored by LC-MS, the reaction was subjected to semi-preparative separation and purification, and lyophilized to obtain a white powder i.e., Compound **ATC-30** (yield 63%). HRMS, calculated for C₁₁₀H₁₅₆N₃₄O₂₉S₄: [M+H]⁺ 2546.0738, [M+3H]³⁺ 849.3631, [M+4H]⁴⁺ 637.2743, observed 849.3654, 637.2842.

### Example 31: Synthesis of Compound ATC-31

**Structure and synthesis method of Compound ATC-31 are as follows:**

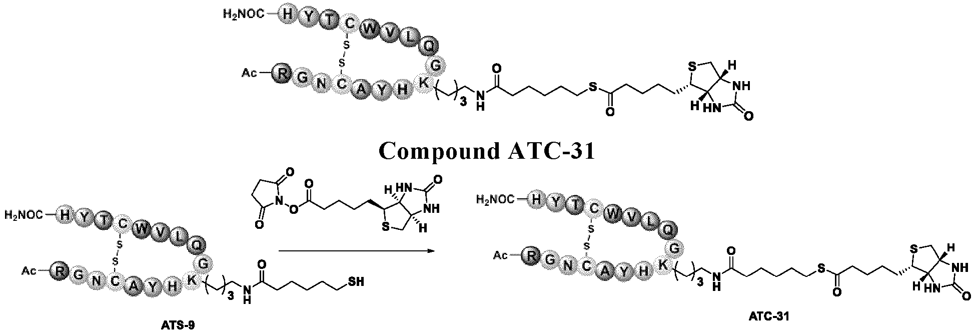

Compound **ATS-9** (7.05 mg, 3.2 µmol) was weighed and dissolved in a mixed solution of PB 7.4/DMF=1/1. Biotin-N-succinimidyl ester (1.32 mg, 3.86 µmol) was added and the mixture was stirred at room temperature for 15min. When the reaction was completed as monitored by LC-MS, the reaction was subjected to semi-preparative separation and purification, and lyophilized to obtain Compound **ATC-31** (yield 78%). HRMS, calculated for C₁₁₂H₁₆₀N₃₂O₂₈S₅: [M+H]⁺2562.0761, [M+3H]³⁺854.6972, [M+4H]⁴⁺ 641.2749, observed 854.7015, 641.2796.

### Example 32: Synthesis of Compound ATC-32

**Structure and synthesis method of Compound ATC-32 are as follows:**

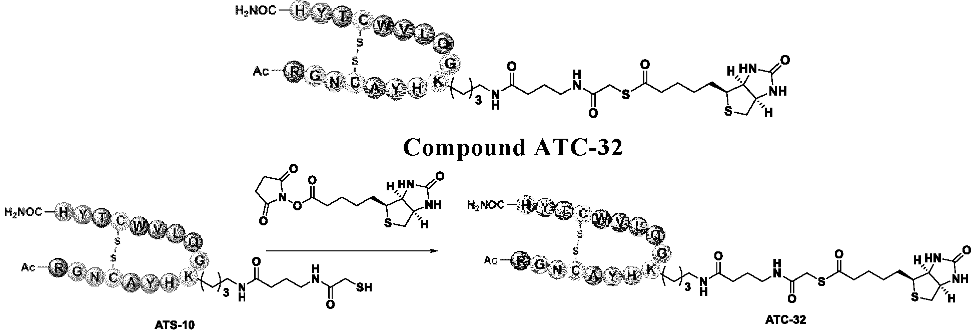

Compound **ATS-10** (7.2 mg, 3.2 µmol) was weighed and dissolved in a mixed solution of PB 7.4/DMF=1/1. Biotin-N-succinimidyl ester (1.32 mg, 3.86 µmol) was added and the mixture was stirred at room temperature for 15min. When the reaction was completed as monitored by LC-MS, the reaction was subjected to semi-preparative separation and purification, and lyophilized to obtain Compound **ATC-32** (yield 78%). HRMS, calculated for C₁₁₂H₁₅₉N₃₃O₂₉S₅: [M+H]⁺2591.0663, [M+3H]³⁺864.3606, [M+4H]⁴⁺ 648.5224, observed 864.3587, 648.5246.

### Example 33: Synthesis of Compound ATC-33

**Structure and synthesis method of Compound ATC-33 are as follows:**

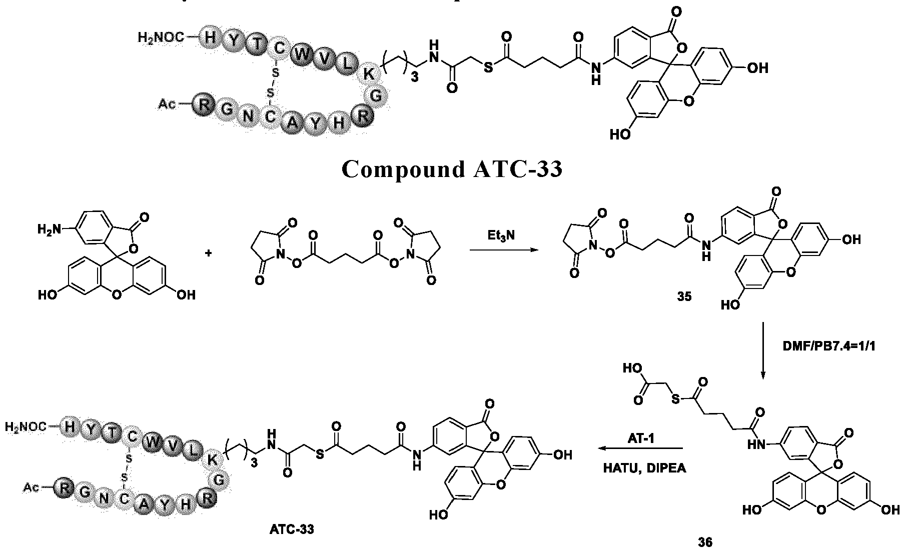

Step 1: Aminofluorescein (10.4 mg, 0.03 mmol) was weighed and dissolved in DMF (500 µL). Di(N-succinimidyl) glutarate (DSG, 39 mg, 0.12 mmol) and triethylamine (12.5 µL, 0.09 mmol) were added and the mixture was reacted at room temperature and away from light for 2 h. When the reaction was completed as monitored by LC-MS, Compound **35** was obtained. Mercaptoacetic acid (6.3 µL, 0.09 mmol) and PB 7.4 buffer (500 µL) were added to the reaction solution, and the reaction was carried out at room temperature in the dark for 2 h. When the reaction was completed as monitored by LC-MS, the reaction was subjected to semi-preparative separation and purification, and lyophilized to obtain a yellow powder (yield 84%). HRMS, calculated for C₂₇H₂₂NO₉S: [M+H]⁺536.1015, observed 536.1028.

Step 2: Compound **36** (5.1 mg, 9.51 µmol) was weighed and dissolved in DMF (300 µL). HATU (2.7 mg, 7.14 µmol), DIPEA (1.84 µL, 10.7 µmol), and **AT-1** (10 mg, 4.76 µmol) were added and the mixture was reacted at room temperature for 2 h. When the reaction was completed as monitored by LC-MS, the reaction was subjected to semi-preparative separation and purification, and lyophilized to obtain Compound **ATC-33.** HRMS, calculated for C₁₂₀H₁₅₄N₃₂O₃₀S₃: [M+H]⁺ 2620.0749, [M+3H]³⁺874.0302, [M+4H]⁴⁺ 655.7746, observed 874.0562, 655.7789.

### Example 34: Synthesis of Compound ATC-34

**Structure and synthesis method of Compound ATC-34 are as follows:**

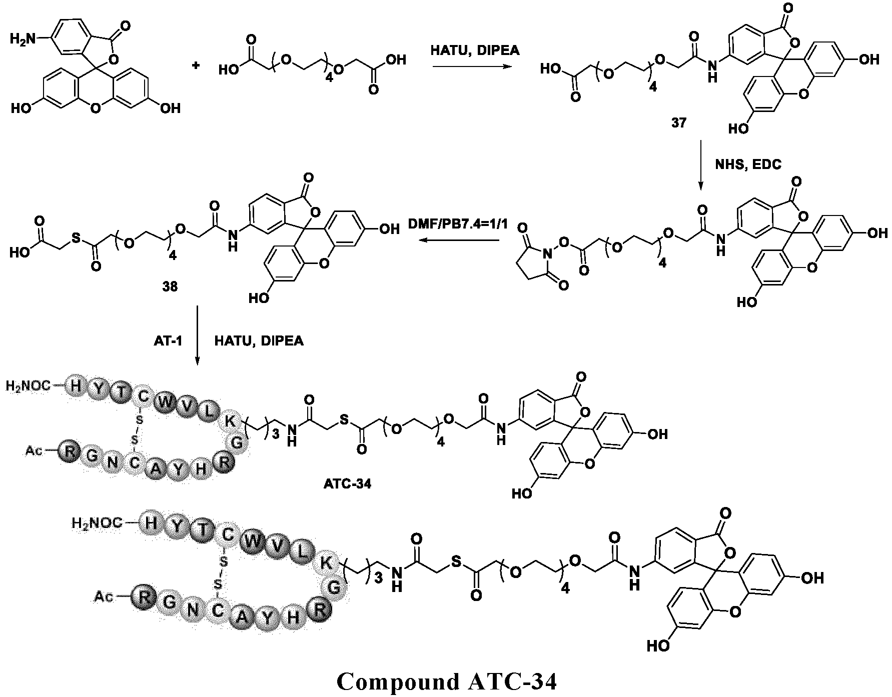

Step 1: Aminofluorescein (17 mg, 49 µmol) was weighed and dissolved in DMF (400 µL ). HATU (28 mg, 73.5 µmol), DIPEA (18.9 µL, 110 µmol), carboxylic acid-polyethylene glycol-carboxylic acid (17.4 mg, 98 µmol) were added and the mixture was reacted at room temperature and away from light for 1 h. When the reaction was completed as monitored by LC-MS, the reaction was subjected to semi-preparative separation and purification, and lyophilized to obtain Compound **37** (yield 92%). HRMS, calculated for C₂₆H₂₁NO₁₀: [M+H]⁺ 508.1244, observed 508.1312.

Step 2: Compound **37** (15.2 mg, 0.03mmol) was weighed and dissolved in DMF (300 µL). N-hydroxysuccinimide (NHS, 4.1 mg, 0.036 mmol) and EDCI (6.4 µL, 0.036 mmol) were added and the reaction was carried out at room temperature in the dark overnight. When the reaction was completed as monitored by LC-MS, mercaptoacetic acid (6.3 µL, 0.09 mmol) and PB 7.4 buffer (300 µL) were added to the reaction solution, and the reaction was carried out at room temperature in the dark for 2 h. When the reaction was completed as monitored by LC-MS, the reaction was subjected to semi-preparative separation and purification, and lyophilized to obtain a yellow powder (yield 81%). HRMS, calculated for C₂₈H₂₃NO₁₁S: [M+H]⁺582.1070, observed 582.1235.

Step 3: Compound **38** (5.5 mg, 9.51 µmol) was weighed and dissolved in DMF (200 µL). HATU (2.7 mg, 7.14 µmol), DIPEA (1.84 µL, 10.7 µmol), and **AT-1** (10 mg, 4.76 µmol) were added and the mixture was reacted at room temperature for 2 h. When.the reaction was completed as monitored by LC-MS, the reaction was subjected to semi-preparative separation and purification, and lyophilized to obtain Compound **ATC-34** (yield 84%). HRMS, calculated for C₁₂₁H₁₅₆N₃₂O₃₂S₃: [M+H]⁺ 2666.0804, [M+3H]³⁺889.3653, [M+4H]⁴⁺ 667.2760, observed 889.3815, 667.2715.

### Example 35: Synthesis of Compound ATC-35

**Structure and synthesis method of Compound ATC-35 are as follows:**

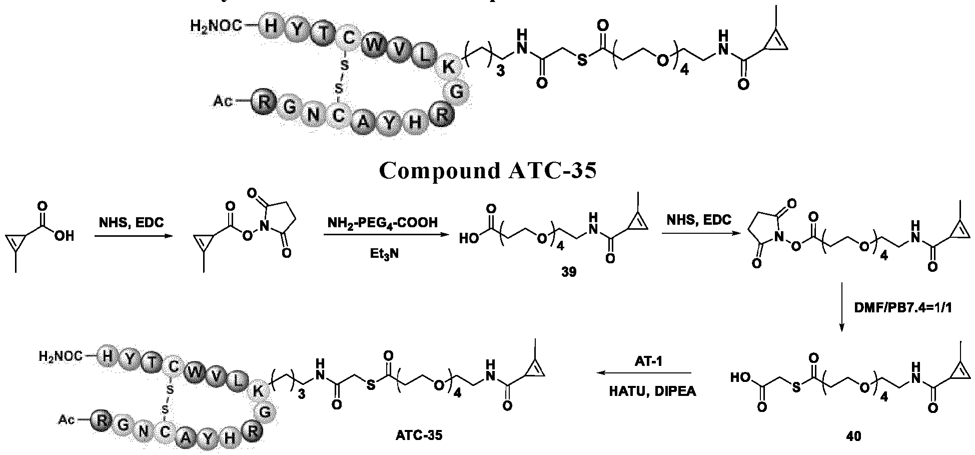

Step 1: 2-Methyl-2-cyclopropenecarboxylic acid (20 mg, 0.2 mmol) was weighed and dissolved in DMF (400 µL). NHS (27.6 mg, 0.24 mmol), and EDCI (42.4 µL, 0.24 mmol) were added and the mixture was reacted at room temperature overnight. 3-[2-[2-[2-(2-aminoethoxy)ethoxy]ethoxy]ethoxy]propanoic acid (53 mg, 0.2 mmol) and triethylamine (83 µL, 0.6 mmol) were added to the reaction solution. When the reaction was completed as monitored by LC-MS, the reaction was subjected to semi-preparative separation and purification, and lyophilized to obtain Compound **39** (yield 78%). HRMS, calculated for C₁₆H₂₇NO₇: [M+H]⁺ 346.1866, observed 346.1902.

Step 2: Compound **39** (10.4 mg, 0.03mmol) was weighed and dissolved in DMF (300 µL). N-hydroxysuccinimide (NHS, 4.1 mg, 0.036 mmol) and EDCI (6.4 µL, 0.036 mmol) were added. After reacting at room temperature overnight, mercaptoacetic acid (6.3 µL, 0.09 mmol) and PB 7.4 buffer (300 µL) were added to the reaction solution, and the reaction was carried out at room temperature for 1 h. When the reaction was completed as monitored by LC-MS, the reaction was subjected to semi-preparative separation and purification, and lyophilized to obtain a white powder (yield 84%). HRMS, calculated for C₁₈H₂₉NO₈S: [M+H]⁺420.1692, observed 420.1589.

Step 3: Compound **40** (4 mg, 9.51 µmol) was weighed and dissolved in DMF (200 µL). HATU (2.7 mg, 7.14 µmol), DIPEA (1.84 µL, 10.7 µmol), and **AT-1** (10 mg, 4.76 µmol) were added and the mixture was reacted at room temperature for 2 h. When the reaction was completed as monitored by LC-MS, the reaction was subjected to semi-preparative separation and purification, and lyophilized to obtain Compound **ATC-35** (yield 90%). HRMS, calculated for C₁₁₁H₁₆₂N₃₂O₂₉S₃: [M+H]⁺ 2531.1426, [M+3H]³⁺ 844.3861, [M+4H]⁴⁺ 633.5415, observed 844.3845, 633.5436.

### Example 36: Synthesis of Compound ATC-36

**Structure and synthesis method of Compound ATC-36 are as follows:**

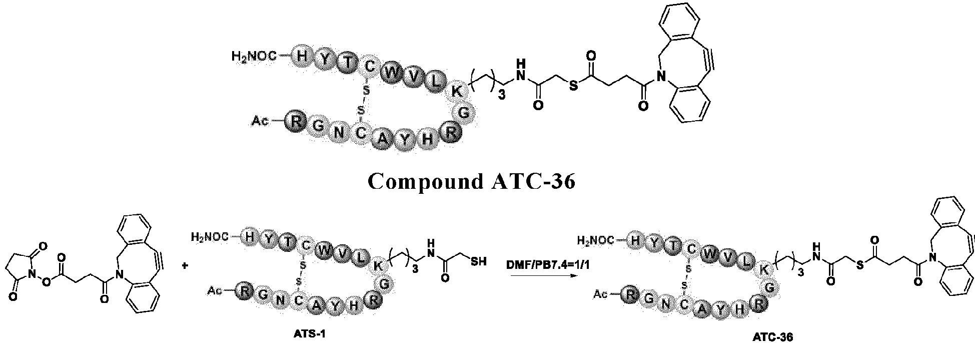

Compound **ATS-1** (6.7 mg, 3.2 µmol) was weighed and dissolved in a mixed solution of PB 7.4/DMF=1/1. DBCO-NHS (1.55 mg, 3.86 µmol) was added and the mixture was stirred at room temperature for 30 min. When the reaction was completed as monitored by LC-MS, the reaction was subjected to semi-preparative separation and purification, and lyophilized to obtain Compound ATC-36 (yield 62%). HRMS, calculated for C₁₁₄H₁₅₀N₃₂O₂₅S₃: [M+H]⁺ 2464.0690, [M+3H]³⁺822.0282, [M+4H]⁴⁺616.7731, observed 822.0315, 616.7712.

### Example 37: Synthesis of Compound ATC-37

**Structure and synthesis method of Compound ATC-37 are as follows:**

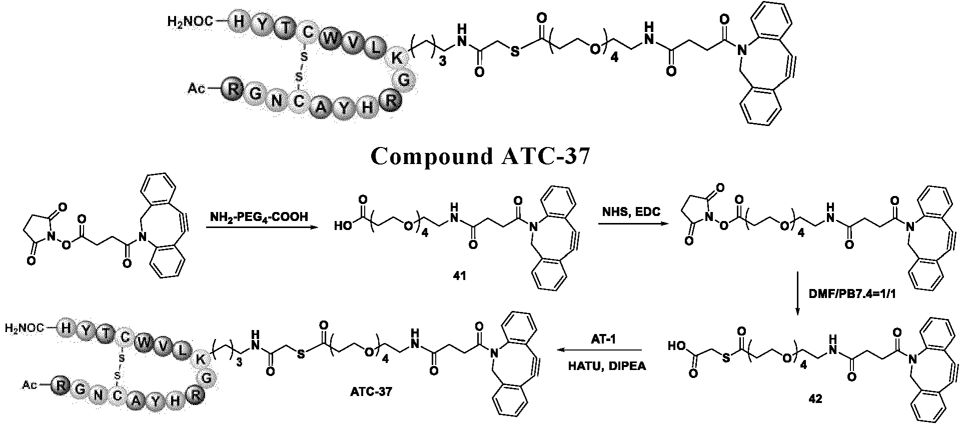

Step 1: DBCO-NHS (40 mg, 0.1 mmol) was weighed and dissolved in DMF (400 µL). NHS (13.8 mg, 0.12 mmol), and EDCI (21.2 µL, 0.12 mmol) were added and the mixture was reacted at room temperature overnight. 3-[2-[2-[2-(2-aminoethoxy)ethoxy]ethoxy]ethoxy]propanoic acid (26.5 mg, 0.1 mmol) and triethylamine (41.5 µL, 0.3 mmol) were added to the reaction solution. When the reaction was completed as monitored by LC-MS, the reaction was subjected to semi-preparative separation and purification, and lyophilized to obtain Compound **41** (yield 81%). HRMS, calculated for C₃₀H₃₆N₂O₈: [M+H]⁺ 553.2550, observed 553.2548.

Step 2: Compound **41** (16.5 mg, 0.03mmol) was weighed and dissolved in DMF (400 µL). N-hydroxysuccinimide (NHS, 4.1 mg, 0.036 mmol) and EDCI (6.4 µL, 0.036 mmol) were added. After reacting at room temperature overnight, mercaptoacetic acid (6.3 µL, 0.09 mmol) and PB 7.4 buffer (400 µL) were added to the reaction solution, and the reaction was carried out at room temperature for 2 h. When the reaction was completed as monitored by LC-MS, the reaction was subjected to semi-preparative separation and purification, and lyophilized to obtain a white powder, i.e, Compound **42** (yield 87%). HRMS, calculated for C₃₂H₃₈N₂O₉S: [M+H]⁺627.2376, observed 627.2315.

Step 3: Compound **42** (6 mg, 9.51 µmol) was weighed and dissolved in DMF (200 µL). HATU (2.7 mg, 7.14 µmol), DIPEA (1.84 µL, 10.7 µmol), and **AT-1** (10 mg, 4.76 µmol) were added and the mixture was reacted at room temperature for 2 h. When the reaction was completed as monitored by LC-MS, the reaction was subjected to semi-preparative separation and purification, and lyophilized to obtain Compound **ATC-37** (yield 92%). HRMS, calculated for C₁₂₅H₁₇₁N₃₃O₃₀S₃: [M+H]⁺ 2711.2110, [M+3H]³⁺ 904.4089, [M+4H]⁴⁺ 678.5586, observed 904.4123, 678.5591.

### Example 38: Synthesis of Compound ATC-38

**Structure and synthesis method of Compound ATC-38 are as follows:**

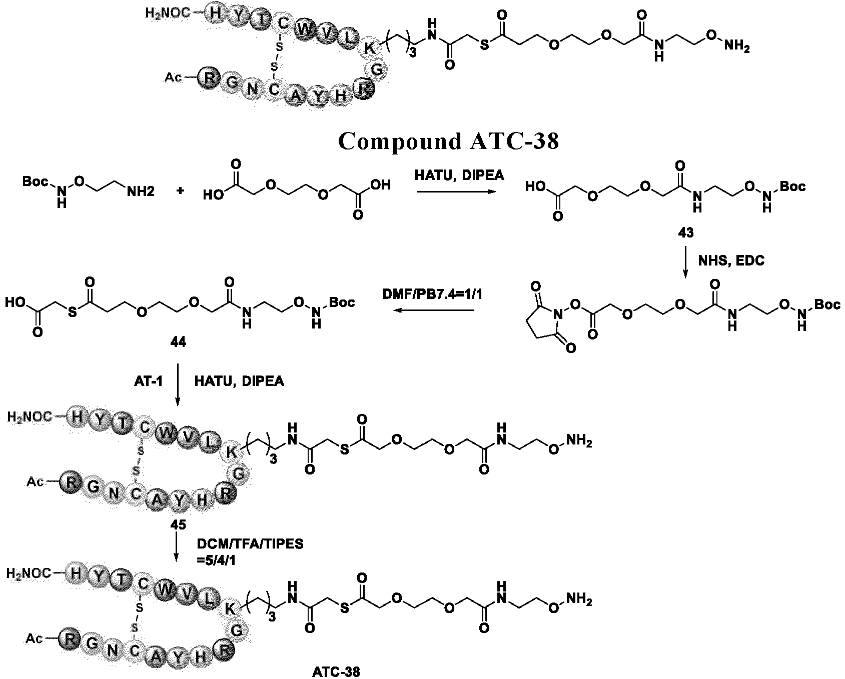

Step 1: Tert-butyl 2-aminoethoxycarbamate (17.6 mg, 0.1 mmol) was weighed and dissolved in DMF (400 µL). HATU (76 mg, 0.2 mmol), DIPEA (51.5 µL, 0.3 mmol) and carboxylic acid-polyethylene glycol-carboxylic acid (53.4 mg, 0.3 mmol) were added and the mixture was reacted at room temperature for 2 h. When the reaction was completed as monitored by LC-MS, the reaction was subjected to semi-preparative separation and purification, and lyophilized to obtain Compound 43 (yield 88%). HRMS, calculated for C₁₃H₂₄N₂O₈: [M+H]⁺ 337.1611, observed 337.1587.

Step 2: Compound **43** (20 mg, 0.06 mmol) was weighed and dissolved in DMF (400 µL). N-hydroxysuccinimide (NHS, 8.2 mg, 0.072 mmol) and EDCI (12.8 µL, 0.072 mmol) were added and the reaction was carried out at room temperature in the dark overnight. When the reaction was completed as monitored by LC-MS, mercaptoacetic acid (12.6 µL, 0.18 mmol) and PB 7.4 buffer (400 µL) were added to the reaction solution, and the reaction was carried out at room temperature in the dark for 2 h. When the reaction was completed as monitored by LC-MS, the reaction was subjected to semi-preparative separation and purification, and lyophilized to obtain a white powder, i.e., Compound **44** (yield 83%). HRMS, calculated for C₁₆H₂₈N₂O₉S: [M+H]⁺425.1594, observed 425.1608.

Step 3: Compound **44** (4 mg, 9.51 µmol) was weighed and dissolved in DMF (200 µL). HATU (2.7 mg, 7.14 µmol), DIPEA (1.84 µL, 10.7 µmol), and **AT-1** (10 mg, 4.76 µmol) were added and the mixture was reacted at room temperature for 2 h. When the reaction was completed as monitored by LC-MS, the reaction was subjected to semi-preparative separation and purification, and lyophilized to obtain Compound **45** (yield 90%). HRMS, calculated for C₁₀₈H₁₅₉N₃₃O₃₀S₃: [M+H]⁺2495.1171, [M+3H]³⁺832.3776, [M+4H]⁴⁺ 624.5351, observed 832.3810, 624.5305.

Step 4: Compound **45** (9 mg, 3.6 µmol) was weighed and added with 200 µL of dichloromethane, 180 µL of trifluoroacetic acid, and 20 µL of isopropyl silane, and the mixture was reacted at 4 °C for 30 min. The solvent was blown dry with N₂ to obtain compound **ATC-38** (yield 95%). HRMS, calculated for C₁₀₃H₁₅₁N₃₃O₂₈S₃: [M+H]⁺2395.0646, [M+3H]³⁺799.0267, [M+4H]⁴⁺599.5220, observed 799.0248, 599.5278.

### Example 39: Synthesis of Compound ATC-39

**Structure and synthesis method of Compound ATC-39 are as follows:**

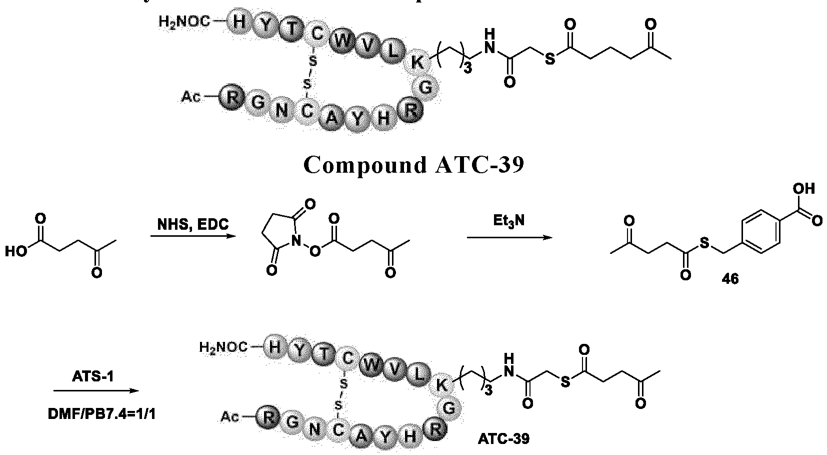

Step 1: Levulinic acid (23.2 mg, 0.2 mmol) was weighed and dissolved in DMF (400 µL). N-hydroxysuccinimide (NHS, 27.6 mg, 0.24 mmol) and EDCI (42.4 µL, 0.24 mmol) were added. After reacting at room temperature overnight, 4-mercaptopropylacetic acid (67.2 mg, 0.4 mmol) and triethylamine (83 µL, 0.6 mmol) were added, and the reaction was carried out at room temperature for 2 h. When the reaction was completed as monitored by LC-MS, the reaction was subjected to semi-preparative separation and purification, and lyophilized to obtain a white powder, i.e., Compound **46** (yield 73%). HRMS, calculated for C₁₄H₁₆O₄S: [M+H]⁺281.0848, observed 281.0923.

Step 2: Compound **ATS1** (6.7 mg, 3 µmol) was weighed and dissolved in a mixed solution of PB 7.4/DMF=1/1. Compound **46** (2.7 mg, 9.6 µmol) was added and the mixture was stirred at room temperature for 4h. When the reaction was completed as monitored by LC-MS, the reaction was subjected to semi-preparative separation and purification, and lyophilized to obtain Compound **ATC-39** (yield 74%). HRMS, calculated for C₁₀₁H₁₄₅N₃₁O₂₅S₃: [M+H]⁺ 2290.0346, [M+3H]³⁺764.0167, [M+4H]⁴⁺573.2645, observed 764.0205, 573.0618.

### Example 40: Synthesis of Compound ATC-40

**Structure and synthesis method of Compound ATC-40 are as follows:**

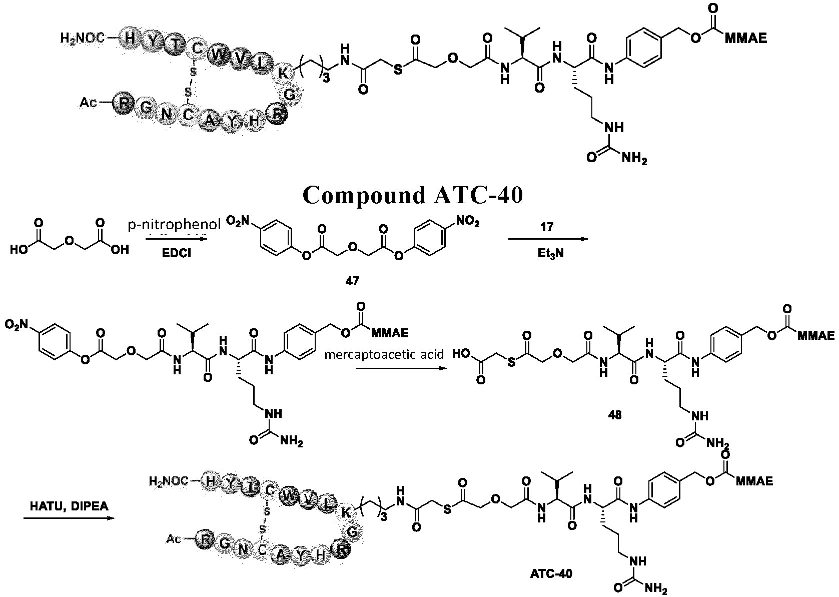

Step 1: Diglycolic acid (26.8 mg, 0.2 mmol) was weighed and dissolved in DMF (400 µL). *p*-Nitrophenol (66.7 mg, 0.48 mmol) and EDCI (84.8 µL, 0.48 mmol) were added. After reacting at room temperature overnight, ether was added for precipitation to obtain product **47** (yield 69%).

Step 2: Compound **47** (11.3 mg, 30 µmol) was dissolved in DMF (200 µL). Compound **17** (11.2 mg, 10 µmol) and triethylamine (6.94 µL, 50 µmol) were added. After reacting at room temperature for 20 min, mercaptoacetic acid (2.1 µL, 30 µmol) and PB 7.4 buffer were added, and the reaction was carried out at room temperature for 1 h. The reaction was subjected to semi-preparative separation and purification to obtain Compound **48** (yield 76%). HRMS, calculated for C₆₄H₁₀₀N₁₀O₁₇S : [M+H]⁺ 1313.7067, observed 1313.7054.

Step 3: Compound **48** (6.2 mg, 4.76 µmol) was weighed and dissolved in DMF (200 µL). HATU (2.7 mg, 7.14 µmol), DIPEA (1.84 µL, 10.7 µmol), and **AT-1** (10 mg, 4.76 µmol) were added and the mixture was reacted at room temperature for 2 h. When the reaction was completed as monitored by LC-MS, the reaction was subjected to semi-preparative separation and purification, and lyophilized to obtain Compound **ATC-40** (yield 91%). HRMS, calculated for C₁₅₇H₂₃₃N₄₁O₃₈S₃: [M+H]⁺ 3397.6800, [M+3H]³⁺1133.2319, [M+4H]⁴⁺ 850.1759, observed 1133.2291, 850.1782.

### Example 41: Synthesis of Compound ATC-41

**Structure and synthesis method of Compound ATC-41 are as follows:**

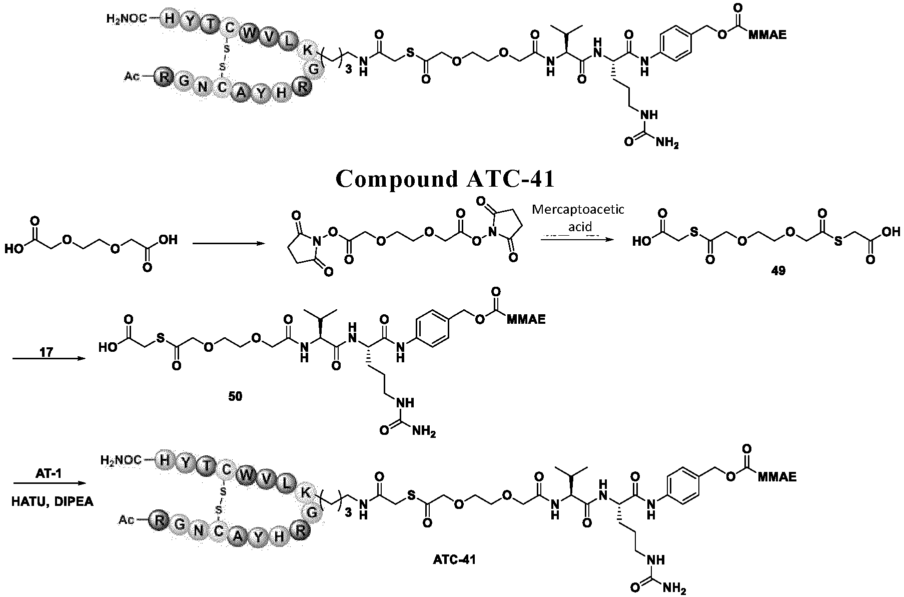

Step 1: Carboxylic acid-polyethylene glycol-carboxylic acid (17.8 mg, 0.1 mmol) was weighed and dissolved in DMF (400 µL). NHS (66.7 mg, 0.24 mmol) and EDCI (42.4 µL, 0.24 mmol) were added. After reacting at room temperature overnight, mercaptoacetic acid (20.8 µL, 0.3 mmol) and PB 7.4 buffer (400 µL) were added, and the reaction was carried out at room temperature for 1 h. The reaction was subjected to semi-preparative separation and purification to obtain Compound **49** (yield 79%). HRMS, calculated for C₁₀H₁₄O₈S₂ : [M+H]⁺ 327.0208, observed 327.0197.

Step 2: Compound **49** (9.8 mg, 30 µmol) was weighed and dissolved in DMF (200 µL). Compound **17** (11.2 mg, 10 µmol) and triethylamine (125 µL, 0.9 mmol) were added. After reacting at room temperature for 2 h, LC-MS monitoring showed that the reaction was complete. The reaction was subjected to semi-preparative separation and purification to obtain Compound **50** (yield 88%). HRMS, calculated for C₆₆H₁₀₄N₁₀O₁₈S : [M+H]⁺ 1357.7329, [M+2H]²⁺679.3704, observed 679.3759.

Step 3: Compound **50** (6.5 mg, 4.76 µmol) was weighed and dissolved in DMF (200 µL). HATU (2.7 mg, 7.14 µmol), DIPEA (1.84 µL, 10.7 µmol), and **AT-1** (10 mg, 4.76 µmol) were added and the mixture was reacted at room temperature for 2 h. When the reaction was completed as monitored by LC-MS, the reaction was subjected to semi-preparative separation and purification, and lyophilized to obtain Compound **ATC-41** (yield 85%). HRMS, calculated for C₁₅₉H₂₃₇N₄₁O₃₉S₃: [M+H]⁺ 3441.7062, [M+4H]⁴⁺861.1824, [M+5H]⁵⁺ 689.1475, observed 861.1859, 689.1512.

### Example 42: Synthesis of Compound ATC-42

**Structure and synthesis method of Compound ATC-42 are as follows:**

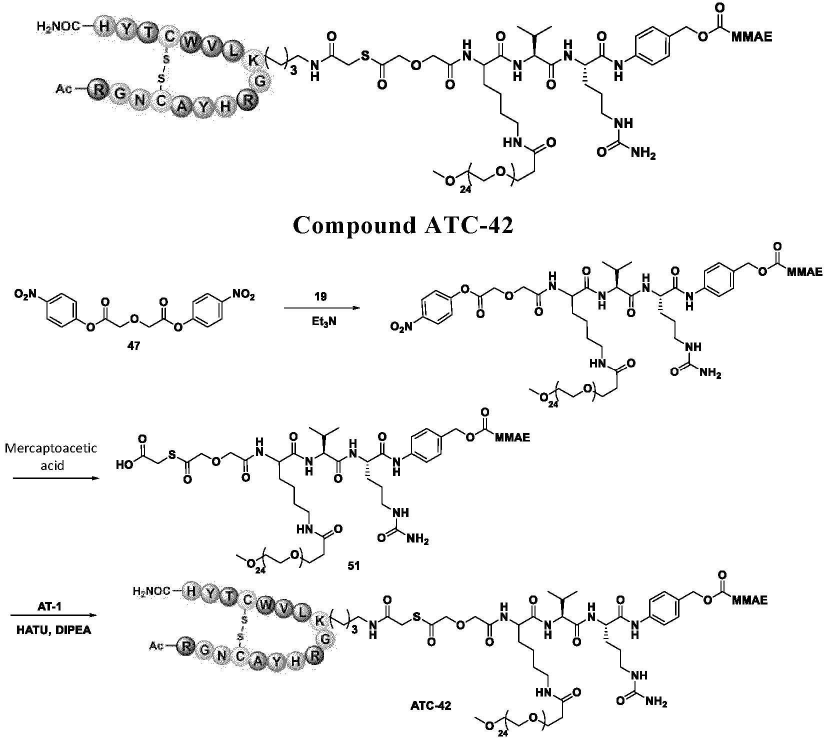

Step 1: Compound **47** (11.3 mg, 30 µmol) was dissolved in DMF (200 µL). Compound **19** (23.9 mg, 10 µmol) and triethylamine (6.94 µL, 50 µmol) were added. After reacting at room temperature for 20 min, mercaptoacetic acid (2.1 µL, 30 µmol) and PB 7.4 buffer were added, and the reaction was carried out at room temperature for 1 h. The reaction was subjected to semi-preparative separation and purification to obtain Compound **51** (yield 81%). HRMS, calculated for C₁₂₂H₂₁₄N₁₂O₄₄S : [M+H]⁺ 2584.4676, [M+3H]³⁺862.1611, [M+4H]⁴⁺ 646.8728, observed 862.1596, 646.8756.

Step 2: Compound **51** (6.15 mg, 2.38 µmol) was weighed and dissolved in DMF (200 µL). HATU (1.35 mg, 4.76 µmol), DIPEA (1.23 µL, 7.14 µmol), and **AT-1** (5 mg, 2.38 _{Il}mol) were added and the mixture was reacted at room temperature for 2 h. When the reaction was completed as monitored by LC-MS, the reaction was subjected to semi-preparative separation and purification, and lyophilized to obtain Compound **ATC-42** (yield 84%). HRMS, calculated for C₂₁₅H₃₄₇N₄₃O₆₅S₃: [M+H]⁺ 4668.4409, [M+4H]⁴⁺1167.8661, [M+5H]⁵⁺ 934.4944, [M+6H]⁶⁺778.9133 observed 934.5015, 778.9215.

### Example 43: Synthesis of Compound ATC-43

**Structure and synthesis method of Compound ATC-43 are as follows:**

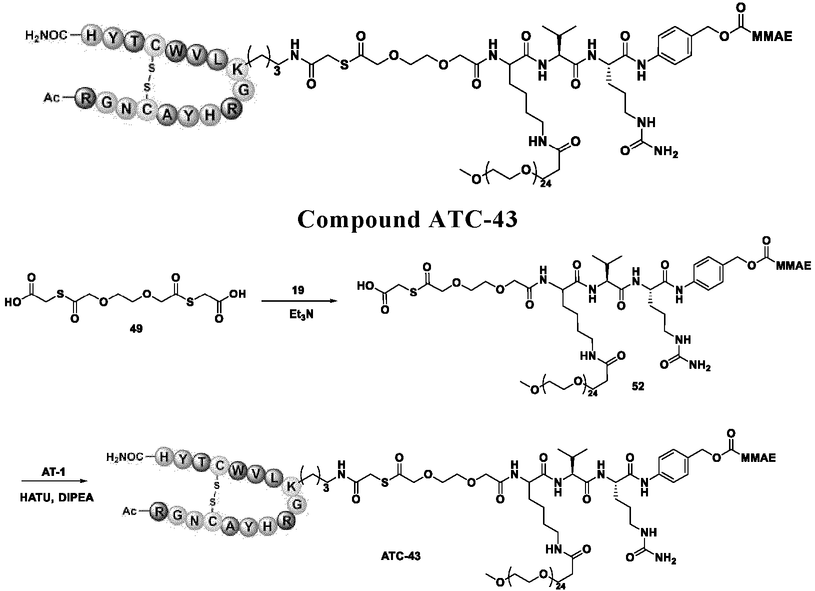

Step 1: Compound **49** (4.9 mg, 15 µmol) was weighed and dissolved in DMF (200 µL). Compound **19** (12 mg, 5 µmol) and triethylamine (62.5 µL, 0.45 mmol) were added. After reacting at room temperature for 3 h, LC-MS monitoring showed that the reaction was complete. The reaction was subjected to semi-preparative separation and purification to obtain Compound **52** (yield 85%). HRMS, calculated for C₁₂₄H₂₁₈N₁₂O₄₅S : [M+H]⁺ 2628.4938, [M+3H]³⁺876.8365, [M+4H]⁴⁺657.8793, observed 876.8395, 657.8852.

Step 2: Compound **52** (6.25 mg, 2.38 µmol) was weighed and dissolved in DMF (200 µL). HATU (1.8 mg, 4.76 µmol), DIPEA (1.23 µL, 7.14 µmol), and **AT-1** (5 mg, 2.38 µmol) were added and the mixture was reacted at room temperature for 1 h. When the reaction was completed as monitored by LC-MS, the reaction was subjected to semi-preparative separation and purification, and lyophilized to obtain Compound **ATC-43** (yield 87%). HRMS, calculated for C₂₁₇H₃₅₁N₄₃O₆₆S₃: [M+H]⁺4712.4671, [M+5H]⁵⁺943.2997, [M+6H]⁶⁺786.2510 observed 943.3005, 786.2519.

### Example 44: Synthesis of Compound ATC-44

**Structure and synthesis method of Compound ATC-44 are as follows:**

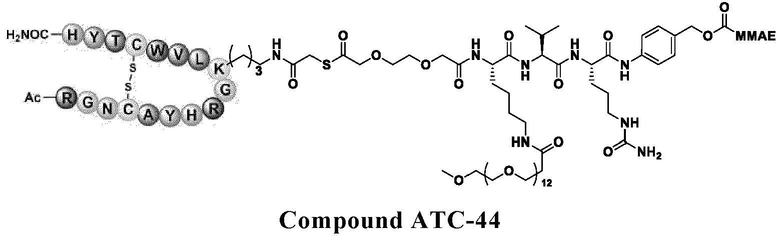

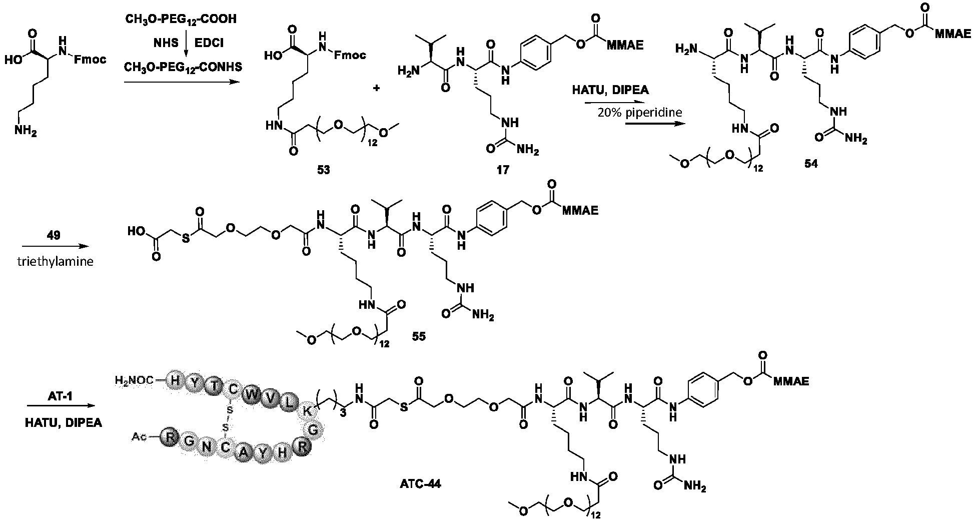

Step 1: CH₃O-PEG₁₂-COOH (17 mg, 0.027 mmol) was weighed and dissolved in acetonitrile. NHS (3.71 mg, 0.032 mmol) and EDCI (5.7 µL, 0.032 mmol) were added. After reacting at room temperature overnight, Fmoc-Lys(NH₂)-OH (10 mg, 0.027 mmol) in a mixed solution of acetonitrile/water=1/1 was added and the mixture was stirred at room temperature for 2 h. When the reaction was completed as monitored by LC-MS, the reaction was subjected to semi-preparative separation and purification, and lyophilized to obtain a white powder **53** (yield 78%). HRMS, calculated for C₄₉H₇₈N₂O₁₈: [M+H]⁺ 983.5328, observed 983.5318.

Step 2: Compound **53** (8.74 mg, 8.9 µmol) was weighed and dissolved in DMF. HATU (6.8 mg, 17.8 µmol), DIPEA (4.6 µL, 27.6 µmol), and Compound **17** (10 mg, 8.9 µmol) were added and the mixture was reacted at room temperature for 2 h. When the reaction was completed as monitored by LC-MS, 20% piperidine was added and the mixture was reacted at room temperature for 15 min. The reaction was subjected to semi-preparative separation and purification, and lyophilized to obtain a white powder **54** (yield 78%). HRMS, calculated for C₉₂H₁₆₀N₁₂O₂₇: [M+H]⁺ 1866.1594, [M+3H]³⁺ 622.7250, observed 622.7289.

Step 3: Compound **54** (9.33 mg, 5 µmol) was weighed and dissolved in DMF (200 µL). Compound **49** (4.9 mg, 15 µmol) and triethylamine (20.8 µL, 0.15mmol) were added. After reacting at room temperature for 2 h, LC-MS monitoring showed that the reaction was complete. The reaction was subjected to semi-preparative separation and purification to obtain Compound **55** (yield 87%). HRMS, calculated for C₁₀₀H₁₇₀N₁₂O₃₃S : [M+H]⁺ 2100.1792, [M+3H]³⁺700.7316, [M+4H]⁴⁺525.8007, observed 700.7359, 525.8095.

Step 4: Compound **52** (5 mg, 2.38 µmol) was weighed and dissolved in DMF (200 µL). HATU (1.8 mg, 4.76 µmol), DIPEA (1.23 µL, 7.14 µmol), and **AT-1** (5 mg, 2.38 µmol) were added and the mixture was reacted at room temperature for 1 h. When the reaction was completed as monitored by LC-MS, the reaction was subjected to semi-preparative separation and purification, and lyophilized to obtain Compound **ATC-44** (yield 86%). HRMS, calculated for C₁₉₃H₃₀₃N₄₃O₅₄S₃: [M+H]⁺ 4184.1526, [M+5H]⁵⁺837.6368, [M+6H]⁶⁺ 698.1986, observed 837.6389, 698.2015.

### Example 45: Synthesis of Compound ATC-45

**Structure and synthesis method of Compound ATC-45 are as follows:**

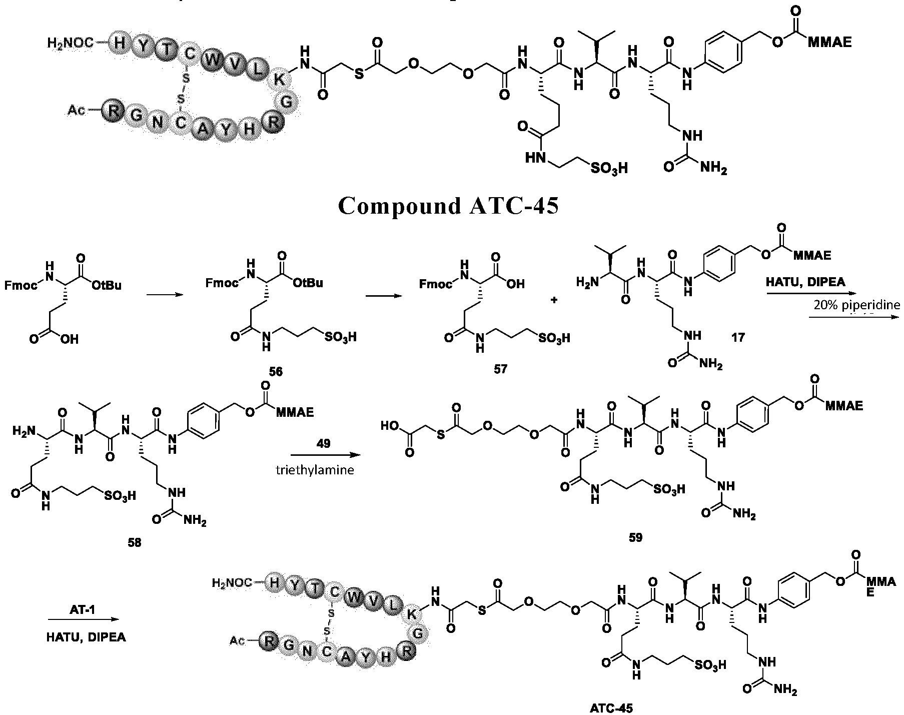

Step 1: Fmoc-L-glutamic acid 1-tert-butyl ester (20 mg, 0.047 mmol) was weighed and dissolved in DMF. HATU (35.8 mg, 0.094 mmol), DIPEA (24.2 µL, 0.14 mmol) and 3-amino-1-propanesulfonic acid (6.5 mg, 0.047 mmol) were added and the mixture was reacted at room temperature for 2 h. When the reaction was completed as monitored by LC-MS, the reaction was subjected to semi-preparative separation and purification, and lyophilized to obtain a white powder **56** (yield 85%). HRMS, calculated for C₂₇H₃₄N₂O₈S: [M+H]+ 547.2114, observed 547.2156.

Step 2: Compound **56** (4.9 mg, 8.9 µmol) was weighed and added with dichloromethane 200 µL, trifluoroacetic acid 180 µL and isopropylsilane 20 µL, and the mixture was reacted at room temperature for 1h. The solvent was blown dry with N₂ to obtain Compound 57, which was dissolved in DMF and added with HATU (6.8 mg, 17.8 µmol), DIPEA (4.6 µL, 27.6 µmol), and Compound **17** (10 mg, 8.9 µmol) and the mixture was reacted at room temperature for 2 h. When the reaction was completed as monitored by LC-MS, 20% piperidine was added and the mixture was reacted at room temperature for 15 min. The reaction was subjected to semi-preparative separation and purification, and lyophilized to obtain a white powder **58** (yield 81%). HRMS, calculated for C₆₆H₁₀₈N₁₂O₁₇S: [M+H]⁺ 1373.7754, [M+2H]²⁺ 687.3916, observed 687.3988.

Step 3: Compound **58** (7.2 mg, 10 µmol) was weighed and dissolved in DMF (200 µL). Compound **49** (9.8 mg, 30 µmol) and triethylamine (41.6 µL, 0.30 mmol) were added. After reacting at room temperature for 2 h, LC-MS monitoring showed that the reaction was complete. The reaction was subjected to semi-preparative separation and purification to obtain Compound **59** (yield 82%). HRMS, calculated for C₇₄H₁₁₈N₁₂O₂₃S₂ : [M+H]⁺ 1607.7952, [M+2H]²⁺804.4015, observed 804.4110.

Step 4: Compound **59** (3.8 mg, 2.38 µmol) was weighed and dissolved in DMF (200 µL). HATU (1.8 mg, 4.76 µmol), DIPEA (1.23 µL, 7.14 µmol), and **AT-1** (5 mg, 2.38 µmol) were added and the mixture was reacted at room temperature for 1 h. When the reaction was completed as monitored by LC-MS, the reaction was subjected to semi-preparative separation and purification, and lyophilized to obtain Compound **ATC-45** (yield 90%). HRMS, calculated for C₁₆₇H₂₅₁N₄₃O₄₄S₄: [M+H]⁺ 3691.7686, [M+5H]⁵⁺739.1600, observed 739.1642.

### Example 46: Synthesis of Compound ATC-46

**Structure and synthesis method of Compound ATC-46 are as follows:**

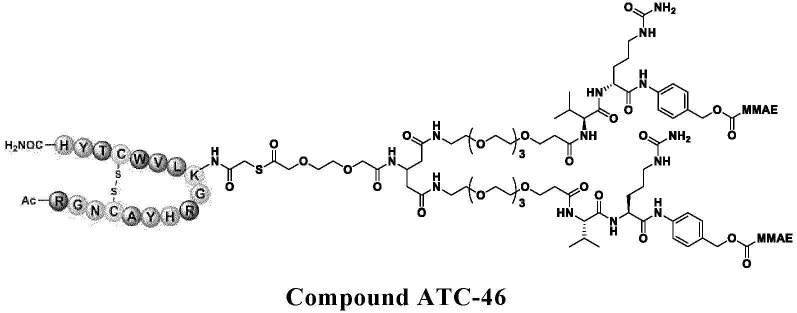

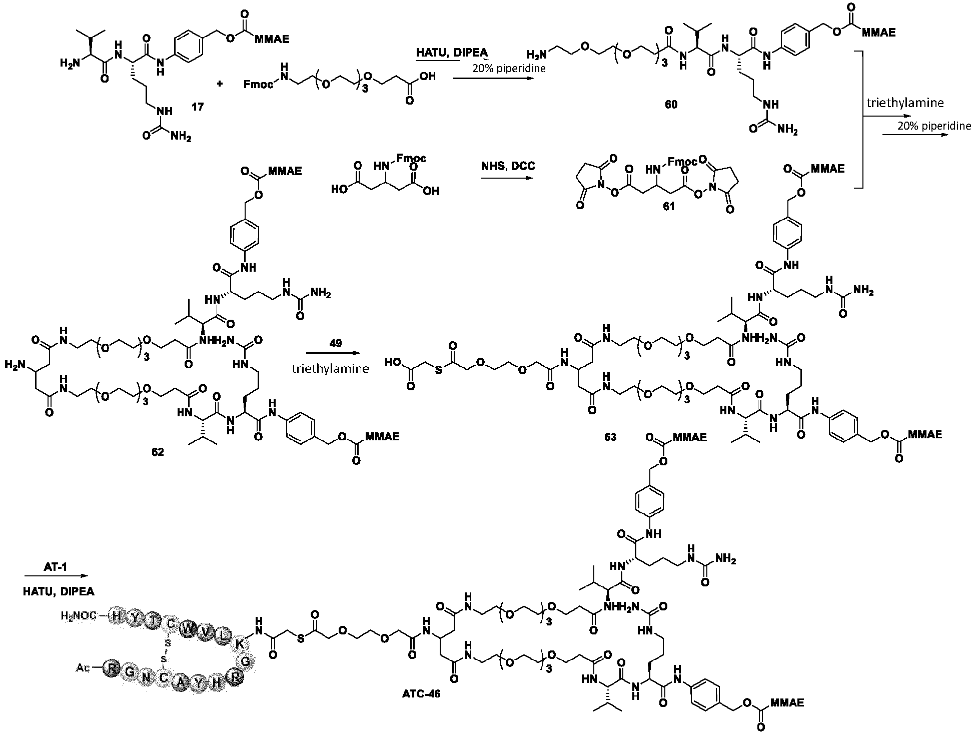

Step 1: Fmoc-PEG₄-COOH (8.8 mg, 17.8 µmol) was weighed and added with HATU (13.6 mg, 35.6 µmol), DIPEA (9.2 µL, 55.2 µmol), and Compound **17** (20 mg, 17.8 µmol). The mixture was reacted at room temperature for 2 h. When the reaction was completed as monitored by LC-MS, 20% piperidine was added and the mixture was reacted for 15 min, the reaction was subjected to semi-preparative separation and purification, and lyophilized to obtain a white powder **60** (yield 87%). HRMS, calculated for C₆₉H₁₁₅N₁₁O₁₇: [M+H]⁺ 1370.8550, observed 1370.8547.

Step 2: 3-((((9H-fluoren-9-yl)methoxy)carbonyl)amino)pentanedioic acid (10 mg, 27.1 µmol) was weighed and dissolved in DMF (300 µL). NHS (3.8 mg, 32.5 µmol) and DCC (6.7 mg, 32.5 µmol) was added and the mixture was reacted at 4 °C overnight to obtain crude product of compound 61. 80 µL of the reaction solution was taken and added with 80 µL of DMF solution of compound 60 (10 mg, 7.3 µmol) and triethylamine (3 µL, 21.9 µmol), and the mixture was reacted at room temperature for 2 h. When the reaction was completed as monitored by LC-MS, 40 µL of piperidine was added and the mixture was reacted at room temperature for 15 min. The reaction was subjected to semi-preparative separation and purification, and lyophilized to obtain compound **62** (yield 90%). HRMS, calculated for C₁₄₃H₂₃₅N₂₃O₃₆ : [M+H]⁺ 2851.7343, [M+3H]³⁺ 951.2500, observed 951.2495.

Step 3: Compound **62** (7.12 mg, 2.5 µmol) was weighed and dissolved in DMF(200 µL). Compound **49** (1.7 mg, 5 µmol) and triethylamine (16.6 µL, 0.12 mmol) were added. After reacting at room temperature for 5 h, LC-MS monitoring showed that the reaction was complete. The reaction was subjected to semi-preparative separation and purification to obtain Compound **63** (yield 70%). HRMS, calculated for C₁₅₁H₂₄₅N₂₃O₄₂S : [M+H]⁺ 3085.7541, [M+3H]³⁺ 1029.2566, [M+4H]⁴⁺ 772.1944, observed 1029.1856, 772.2015.

Step 4: Compound **63** (5 mg, 1.62 µmol) was weighed and dissolved in DMF (200 µL). HATU (1.2 mg, 3.24 µmol), DIPEA (0.84 µL, 4.86 µmol), and **AT-1** (5 mg, 2.38 µmol) were added and the mixture was reacted at room temperature for 2 h. When the reaction was completed as monitored by LC-MS, the reaction was subjected to semi-preparative separation and purification, and lyophilized to obtain Compound **ATC-46** (yield 88%). HRMS, calculated for C₂₄₄H₃₇₈N₅₄O₆₃S₃: [M+H]⁺ 5169.7275, [M+5H]⁵⁺ 1034.7517, observed 1034.7451.

### V: Application Examples of Cleavable Fragment Directed By Affinity Fragment

### Example 47: Preparation and characterization of biotinylated antibody Ab-1

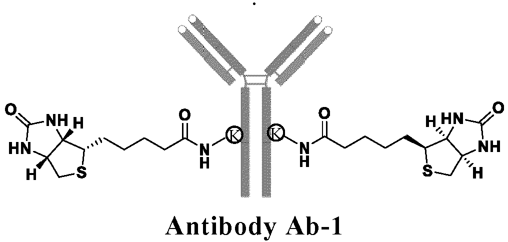

### 47-1. Preparation of Antibody Ab-1:

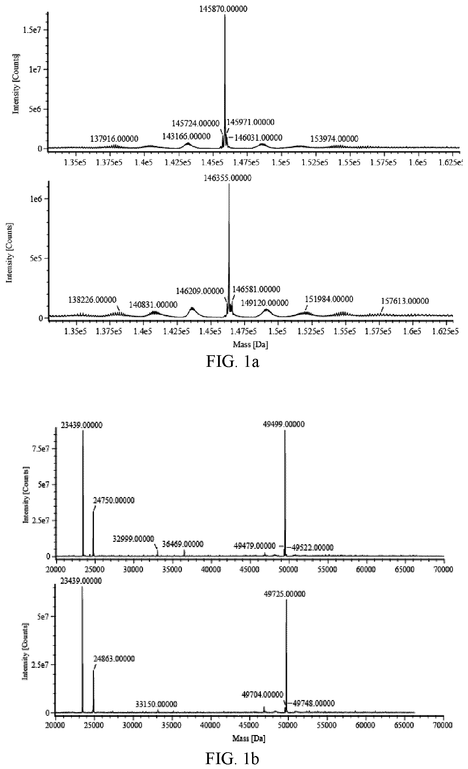

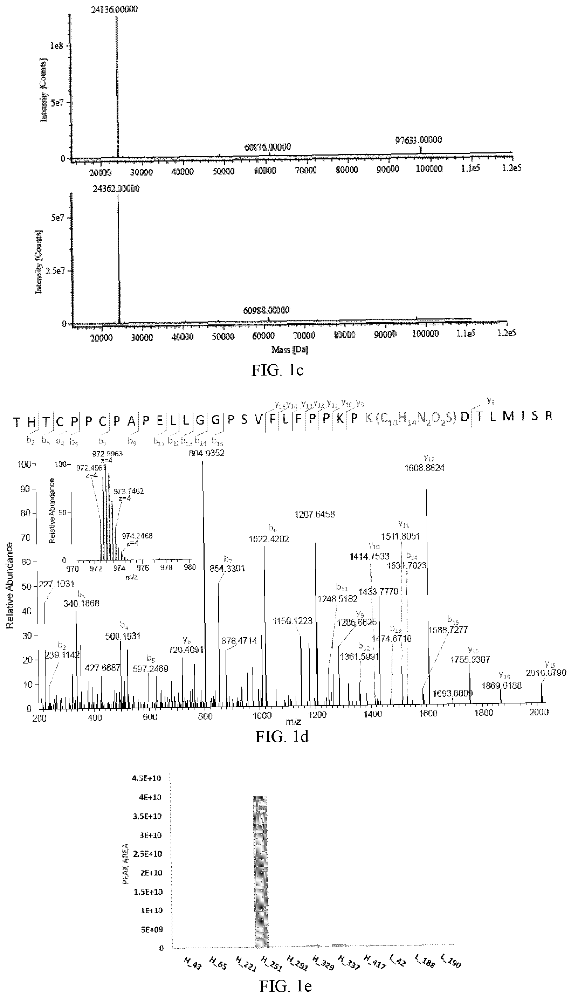

Trastuzumab (5 mg/mL) and compound **ATC-2** (10 eq, 0.334 mM) were added to PIPES buffer solution (50 mM, pH 7.4, containing 20% DMF) and the mixture was reacted at 37 °C for 2 h. The reaction was complete as monitored by LC-MS. The reaction was purified by protein A, concentrated by ultrafiltration tube, and then replaced with 1 × PBS buffer to obtain biotinylated antibody **Ab-1.** The mass was measured by ESI-TOFMS. The peak of the raw material trastuzumab was observed at 145870. Regarding the reaction product, the peak of the product coupled with two biotins was observed at 146355 (FIG. 1a).

### 47-2. Confirmation of heavy chain selectivity of antibody Ab-1:

Antibody **Ab-1** was diluted to 5 mg/mL with 50 mM PB 7.4 buffer, added with TCEP (20 eq, final concentration: 0.668 mM), and incubated at 37 °C for 20 min. The mass was measured by ESI-TOFMS. The same light chain peak was observed for the raw material and the product. The peak of the heavy chain of the raw material was observed at 49499. Regarding the product, the peak of the heavy chain introduced with biotin tag was observed at 49725 (FIG. 1b).

### 47-3 Confirmation of Fc selectivity of antibody Ab-1:

Antibody **Ab-1** was diluted to 5 mg/mL with 50 mM PB 7.0 buffer, added with IdeS protease (final concentration: 0.5 U/ µL), and incubated at 37 °C overnight. The mass was measured by ESI-TOFMS. The same Fab peak was observed for the raw material and the product. The peak of Fc of the raw material was observed at 24136. Regarding the product, the peak of the Fc region introduced with biotin tag was observed at 24362 (FIG. 1c).

### 47-4. Confirmation of the site of antibody Ab-1:

### 47-4-1. Sample preparation

Antibody **Ab-1** (10 µg) was taken and diluted to 90 □µL with 50 mM ammonium bicarbonate buffer. The mixture was added with 1 □µL of PNGase F enzyme and incubated at 37 °C for 4 h. The mixture was added with DTT (5 mM) and incubated at 37 °C for 30 min, and then added with then iodoacetamide (11 mM) and reacted at room temperature in the dark for 30 min. Subsequently, the mixture was digested with 0.5 □µg trypsin at 37 ° C overnight. 2% Trifluoroacetic acid was used to terminate digestion, and the digested protein was desalinated and dried. Subsequently, further LC-MS/MS analysis was conducted.

### 47-4-2. LC-MS/MS device and analysis conditions

NanoHPLC: EASY-nLC 1200 UHPLC System (ThermoFisher Scientific)
Mass spectrometer: Q Exactive HF-X mass spectrometer (ThermoFisher Scientific)
Analysis column: C18 column (75 µm × 25 cm, 1.9 µm) (Dr. Maisch GmbH)
Mobile phase A: 0.1% formic acid in water
Mobile phase B: 0.1% formic in acetonitrile
Gradient condition (B%): 5% -90% (0-30 min)

Nanometer spray source was used for analysis. Complete peptide was detected in Orbitrap at a resolution of 60000. The scanning range of MS is 350-2000 m/z, the automatic gain control value is 1e⁶, and the maximum injection time is 45 ms. Peptides were selected for MS/MS using a normalized collision energy setting of 28; tandem mass spectra were acquired with a mass resolution of 30,000. The first 12 precursor ions with charges of 2 to 5 in each MS scan were selected for subsequent MS/MS scans with an automatic gain control value of 5e⁴ and a maximum injection time of 100 ms. Dynamic exclusion was set to 10 s.

### 47-4-3. Data acquisition

The raw data were analyzed using the protein identification software Mascot 2.3. The trastuzumab sequence was added to the peptide recognition database provided to the search engine. Trypsin was set as the digestion enzyme (C-terminal being cleaved to Lys and Arg residues), allowing up to two missed cleavages. Oxidation of methionine residue (+15.9949 Da), new modification to lysine (+226.0776 Da), and deamidation of asparagine (+0.9840 Da) were set as variable modifications. A search was performed for methyl carbamate on cysteine (+57.0215 Da) as a fixed modification. The mass tolerances for precursor ions and fragment ions were set to 10 ppm and 0.05 Da, respectively. All modified peptides were manually verified.

### 47-4-4. Result Analysis

From the LC-MS/MS analysis results, observed is the MS spectrum of a peptide fragment of trastuzumab digested with trypsin that contains the modified site on lysine and consists of 33 amino acid residues (THTCPPCPAPELLGGPSVFLFPPKPK(C₁₀H₁₄N₂O₂S)DTLMISR). The modification of the lysine residue at position 251 on the heavy chain was confirmed based on the CID spectrum. In addition, further analysis by Mascot 2.3 software showed that the modification of the lysine residue at position 251 occurred with high selectivity (FIG. 1e).

### Example 48: Preparation and characterization of azide antibody Ab-2

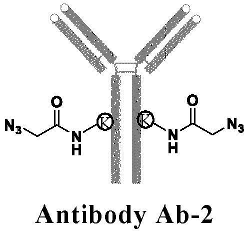

### 48-1. Preparation of Antibody Ab-2:

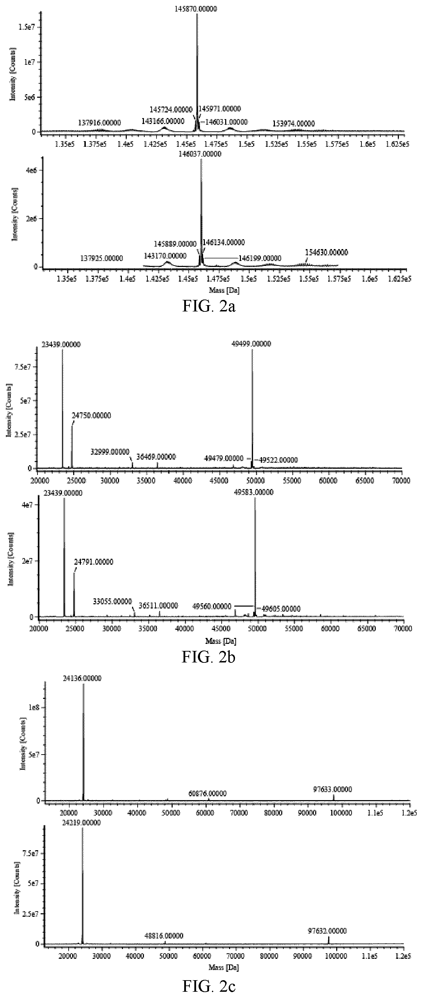

Trastuzumab (5 mg/mL) and compound **ATC-1** (5 eq, 0.168 mM) were added to NaOAc buffer solution (50 mM, pH 5.5, containing 20% DMF) and the mixture was incubated at 37 °C for 30 min. The reaction was complete as monitored by LC-MS. The reaction was subjected to concentration by ultrafiltration tube, and then replacement with 1 × PBS buffer to obtain biotinylated antibody **Ab-2.** The mass was measured by ESI-TOFMS. The peak of the raw material trastuzumab was observed at 145870. Regarding the reaction product, the peak of the product coupled with two azidoacetic acid was observed at 146037 (FIG. 2a).

### 48-2. Confirmation of heavy chain selectivity of antibody Ab-2:

Antibody Ab-2 was diluted to 5 mg/mL with 50 mM PB 7.4 buffer, added with TCEP (20 eq, final concentration: 0.668 mM), and incubated at 37 °C for 20 min. The mass was measured by ESI-TOFMS. The same light chain peak was observed for the raw material and the product. The peak of the heavy chain of the raw material was observed at 49499. Regarding the product, the peak of the heavy chain introduced with biotin tag was observed at 49583 (FIG. 2b).

### 48-3 Confirmation of Fc selectivity of antibody Ab-2:

Antibody **Ab-2** was diluted to 5 mg/mL with 50 mM PB 7.0 buffer, added with IdeS protease (final concentration: 0.5 U/ µL), and incubated at 37 °C overnight. The mass was measured by ESI-TOFMS. The same Fab peak was observed for the raw material and the product. The peak of Fc of the raw material was observed at 24136. Regarding the product, the peak of the Fc region introduced with azide group was observed at 24219 (FIG. 2c).

### Example 49: Preparation and characterization of FITC site-specific modified antibody Ab-3

### Antibody Ab-3

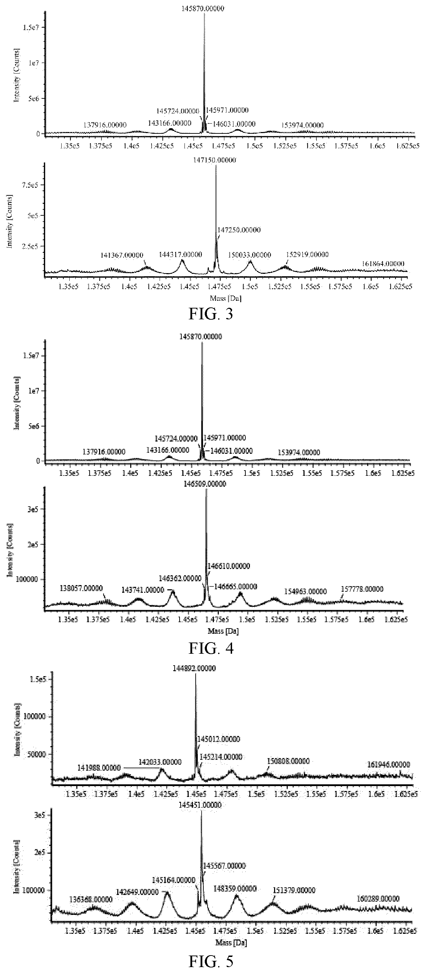

Trastuzumab (5 mg/mL) and compound **ATC-34** (10 eq, 0.334 mM) were added to PIPES buffer solution (50 mM, pH 7.4, containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain FITC site-specific modified antibody Ab-3. The mass was measured by ESI-TOFMS. The peak of the raw material trastuzumab was observed at 145870. Regarding the reaction product, the peak of the product coupled with two biofluorescein was observed at 147150 (FIG. 3).

### Example 50: Preparation and characterization of methylcyclopropene site-specific modified antibody Ab-4

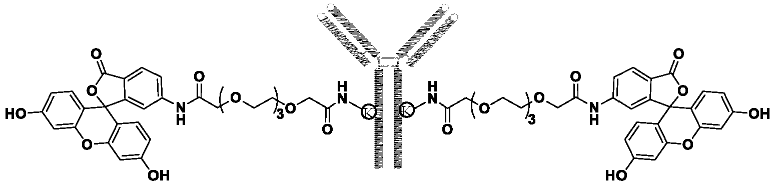

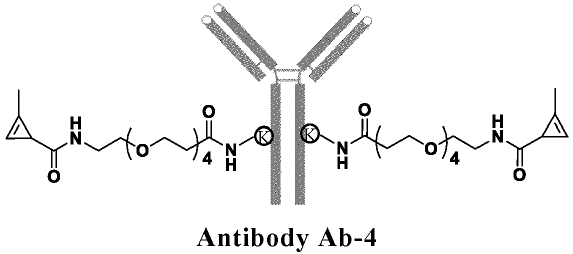

Trastuzumab (5 mg/mL) and compound **ATC-35** (10 eq, 0.334 mM) were added to PIPES buffer solution (50 mM, pH 7.4, containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain methylcyclopropene site-specific modified antibody **Ab-4.** The mass was measured by ESI-TOFMS. The peak of the raw material trastuzumab was observed at 145870. Regarding the reaction product, the peak of the product coupled with two methylcyclopropene was observed at 146524 (FIG. 4).

### Example 51: Preparation and characterization of DBCO site-specific modified antibody Ab-5

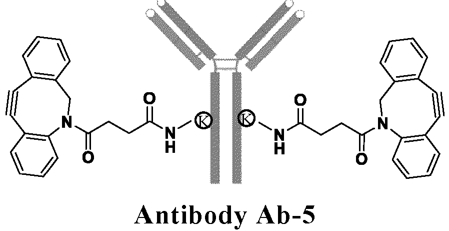

Rituximab (5 mg/mL) and compound **ATC-36** (10 eq, 0.334 mM) were added to PIPES buffer solution (50 mM, pH 7.4, containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain DBCO site-specific modified antibody **Ab-5.** The mass was measured by ESI-TOFMS. The peak of the raw material trastuzumab was observed at 144891. Regarding the reaction product, the peak of the product coupled with two DBCO was observed at 145465 (FIG. 5).

### Example 52: Preparation and characterization of DBCO site-specific modified antibody Ab-6

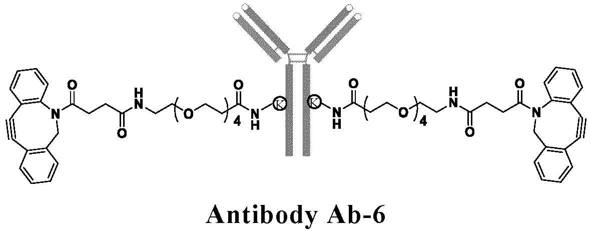

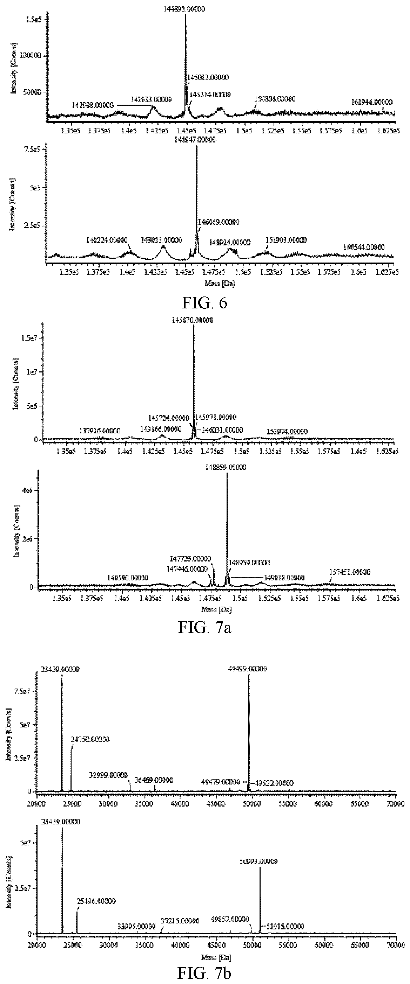

Rituximab (5 mg/mL) and compound **ATC-37** (10 eq, 0.334 mM) were added to PIPES buffer solution (50 mM, pH 7.4, containing 20% DMF) and the mixture was reacted at 37 °C for 2 h. The reaction was complete as monitored by LC-MS. The reaction was subjected to concentration by ultrafiltration tube, and then replacement with 1 × PBS buffer to obtain DBCO site-specific modified antibody **Ab-6.** The mass was measured by ESI-TOFMS. The peak of the raw material trastuzumab was observed at 144891. Regarding the reaction product, the peak of the product coupled with two DBCO was observed at 145947 (FIG. 6).

### Example 53: Preparation and characterization of ADC-1 using a two-step method

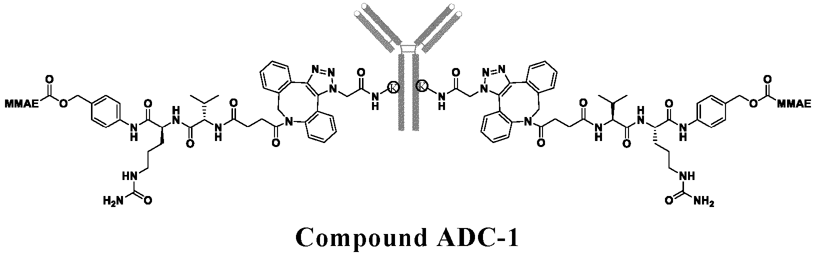

### 53-1. Preparation of Antibody ADC-1:

Azide antibody **Ab-2** (5 mg/mL) and Drug Linker **D1** (15 eq, 0.5 mM) were respectively added to PB buffer solution (50 mM, pH 7.4, containing 20% DMF) and the mixture was reacted at 37 °C overbnight. The reaction was complete as monitored by LC-MS. The reaction was subjected to purification using Protein A, concentration by ultrafiltration tube, and then replacement with 1 × PBS buffer to obtain ADC-1. The mass was measured by ESI-TOFMS. The peak of the raw material trastuzumab was observed at 145870. Regarding the reaction product, the peak of the product coupled with two drug linker **D1** was observed at 148859 (FIG. 7a).

### 53-2. Confirmation of heavy chain selectivity of ADC-1:

**ADC-1** was diluted to 5 mg/mL with 50 mM PB 7.4 buffer, added with TCEP (20 eq, final concentration: 0.668 mM), and incubated at 37 °C for 20 min. The mass was measured by ESI-TOFMS. The same light chain peak was observed for the raw material and the product. The peak of the heavy chain of the raw material was observed at 49499. Regarding the product, the peak of the heavy chain introduced with one drug linker **D1** was observed at 50992 (FIG. 7b).

### 53-3. Confirmation of Fc selectivity of ADC-1:

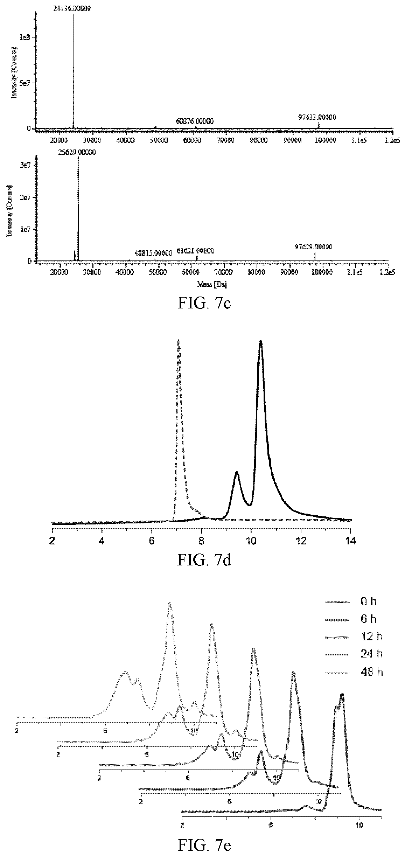

**ADC-1** was diluted to 5 mg/mL with 50 mM PB 7.0 buffer, added with IdeS protease (final concentration: 0.5 U/ µL), and incubated at 37 °C overnight. The mass was measured by ESI-TOFMS. The same Fab peak was observed for the raw material and the product. The peak of Fc of the raw material was observed at 24136. Regarding the product, the peak of the Fc region introduced with one drug linker was observed at 25629 (FIG. 7c).

### 53-4. HIC analysis (Hydrophobic Interaction Chromatography analysis) of ADC-1

**ADC-1** was diluted to 5 mg/mL with 1 × PBS and 5 µL was injected. The HIC column used was TSKgel, (4.6 × 10 cm, 2.5 µm); Mobile phase A: 0.1 M sodium phosphate, 1.5 M ammonium sulfate buffer, pH=7.0; mobile phase B: 0.1 M sodium phosphate buffer, pH=7.0, a flow rate of 1 mL/min, 0-15 min, a linear gradient of 0-100% for mobile phase B. Detection wavelength: 280 nm. The retention time of 9.391 min was considered to be an impurity peak introduced by the drug linker **D1** due to its insufficient purity, and the retention time of 10.354 min was considered to be a compound that has two drug linkers **D1** introduced into trastuzumab (FIG. 7d).

### 53-5. Aggregation stability analysis of ADC-1 (molecular-exclusion chromatography)

ADC-1 was diluted to 1 mg/mL with 1 × PBS and heated at 60 °C in a PCR instrument for 48 h. Samples were taken at 0 h, 6 h, 12 h, 24 h, and 48 h for SEC analysis. 15 µL of sample was taken at each time point. The SEC column used was a BioCore SEC-300 column (7.8×300 mm 5 µm). SEC analysis used a mobile phase of 150 mM sodium phosphate, pH = 7.4, a flow rate of 1 mL/min, an isocratic elution of 15 min, and a detection wavelength of 280 nm. The retention time of 5.4-8.0 min was considered to be the peak of aggregates, and the retention time of 8.907 min was considered to be the peak of monomers (FIG. 7e).

### Example 54: Preparation and characterization of ADC-2 using a two-step method

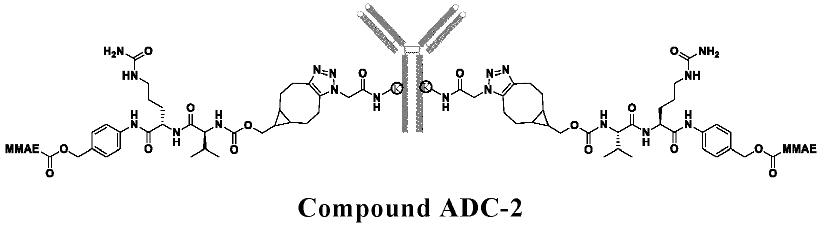

### 54-1. Preparation of Antibody ADC-2:

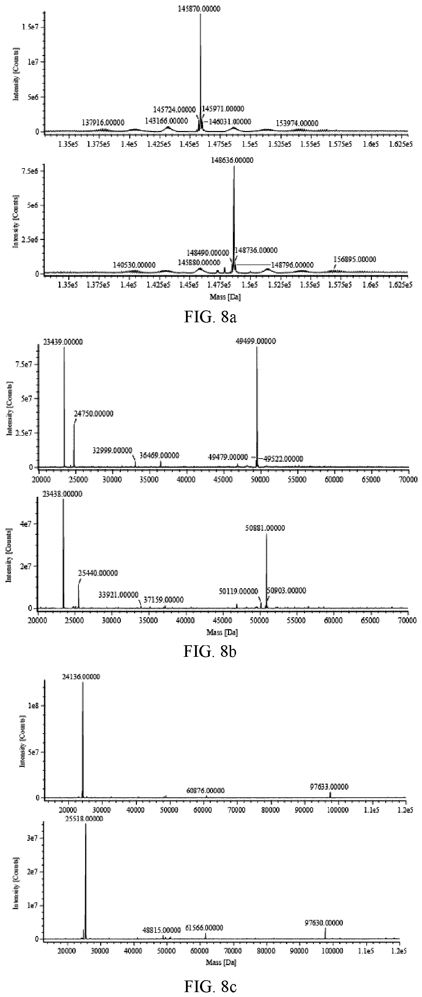

The preparation condition of ADC-2 was the same as that of ADC-1. The mass was measured by ESI-TOFMS. The peak of the raw material trastuzumab was observed at 145870. Regarding the reaction product, the peak of the product coupled with two drug linker **D2** was observed at 148636 (FIG. 8a).

### 54-2. Confirmation of heavy chain selectivity of ADC-2:

The condition of TCEP treatment was the same as those for ADC-1. The mass was measured by ESI-TOFMS. The same light chain peak was observed for the raw material and the product. The peak of the heavy chain of the raw material was observed at 49499. Regarding the product, the peak of the heavy chain introduced with one drug linker **D2** was observed at 50811 (FIG. 8b).

### 54-3. Confirmation of Fc selectivity of ADC-2:

The condition of IdeS protease treatment was the same as those for ADC-1. The mass was measured by ESI-TOFMS. The same Fab peak was observed for the raw material and the product. The peak of Fc of the raw material was observed at 24136. Regarding the product, the peak of the Fc region introduced with one drug linker was observed at 25518 (FIG. 8c).

### 54-4. HIC analysis (Hydrophobic Interaction Chromatography analysis) of ADC-2

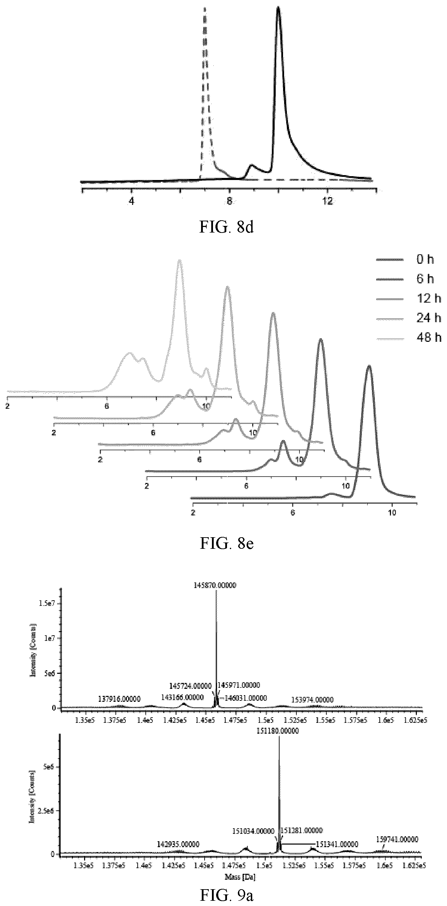

The HIC analysis operation was the same as that for **ADC-1.** The retention time of 9.071 min was considered to be the compound that has one drug linker **D2** introduced into trastuzumab, and the retention time of 10.170 min was considered to be the compound that has two drug linkers **D2** introduced into trastuzumab (FIG. 8d).

### 54-5. Aggregation stability analysis of ADC-2 (molecular-exclusion chromatography)

The aggregation stability analysis operation was the same as that for **ADC-1.** The retention time of 5.4-8.0 min was considered to be the peak of aggregates, and the retention time of 8.923 min was considered to be the peak of monomers (FIG. 8e).

### Example 55: Preparation and characterization of ADC-3 using a two-step method

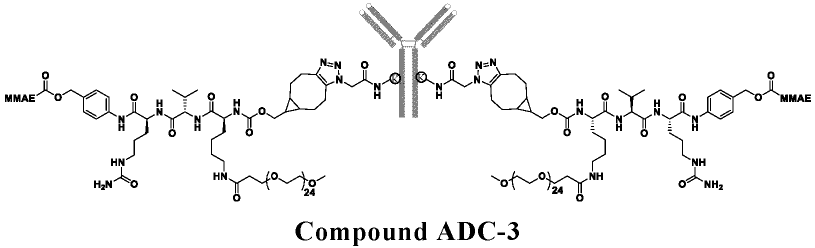

### 55-1. Preparation of Antibody ADC-3:

The preparation condition of ADC-3 was the same as that of ADC-1. The mass was measured by ESI-TOFMS. The peak of the raw material trastuzumab was observed at 145870. Regarding the reaction product, the peak of the product coupled with two drug linker **D3** was observed at 151180 (FIG. 9a).

### 55-2. Confirmation of heavy chain selectivity of ADC-3:

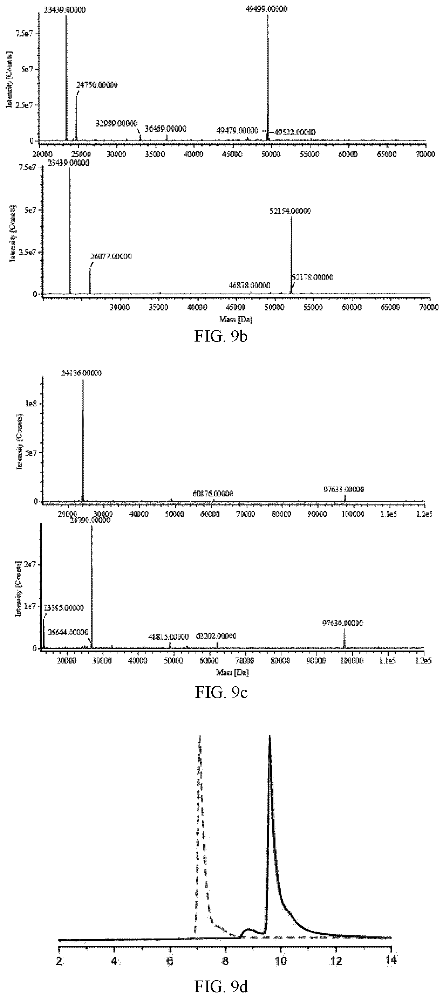

The condition of TCEP treatment was the same as those for ADC-1. The mass was measured by ESI-TOFMS. The same light chain peak was observed for the raw material and the product. The peak of the heavy chain of the raw material was observed at 49499. Regarding the product, the peak of the heavy chain introduced with one drug linker **D3** was observed at 52154 (FIG. 9b).

### 55-3. Confirmation of Fc selectivity of ADC-3:

The condition of IdeS protease treatment was the same as those for ADC-1. The mass was measured by ESI-TOFMS. The same Fab peak was observed for the raw material and the product. The peak of Fc of the raw material was observed at 24136. Regarding the product, the peak of the Fc region introduced with one drug linker **D3** was observed at 26790 (FIG. 9c).

### 55-4. HIC analysis (Hydrophobic Interaction Chromatography analysis) of ADC-3

The HIC analysis operation was the same as that for **ADC-1.** The retention time of 8.846 min was considered to be the compound that has one drug linker **D3** introduced into trastuzumab, and the retention time of 9.611 min was considered to be the compound that has two drug linker **D3** introduced into trastuzumab (FIG. 9d).

### 55-5. Aggregation stability analysis of ADC-3 (molecular-exclusion chromatography)

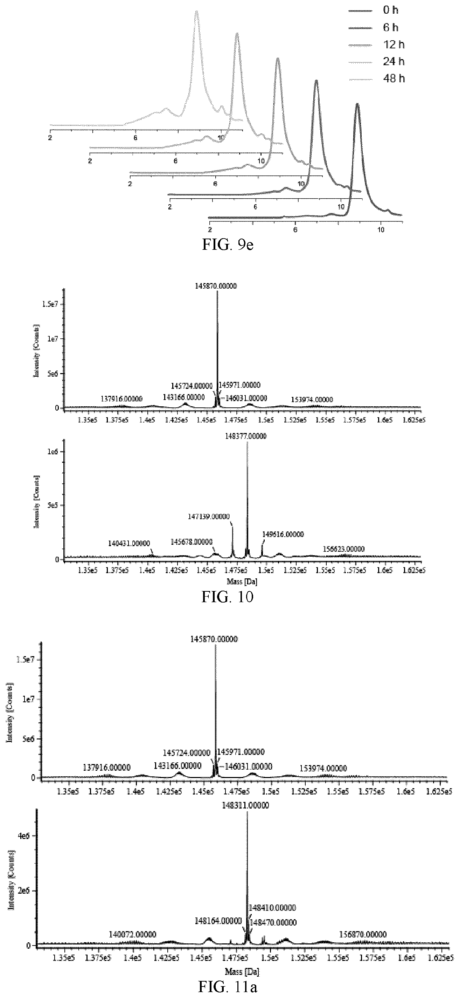

The aggregation stability analysis operation was the same as that for **ADC-1.** The retention time of 5.4-8.0 min was considered to be the peak of aggregates, and the retention time of 8.850 min was considered to be the peak of monomers (FIG. 9e).

### Example 56: Preparation and characterization of ADC-4 using a two-step method

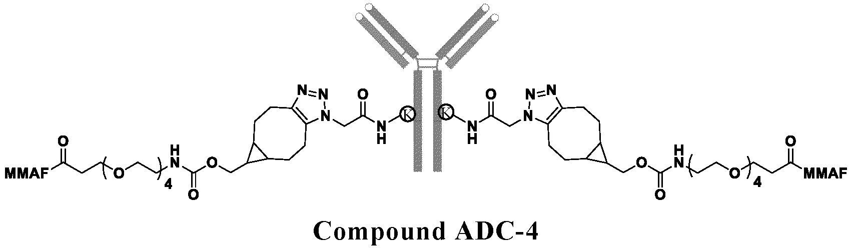

### Preparation of ADC-4:

The preparation condition of **ADC-4** was the same as that of **ADC-1.** The mass was measured by ESI-TOFMS. The peak of the raw material trastuzumab was observed at 145870. Regarding the reaction product, the peak of the product coupled with two drug linkers **D4** was observed at 148377 (FIG. 10).

### Example 57: Preparation and characterization of ADC-5 using a one-step method

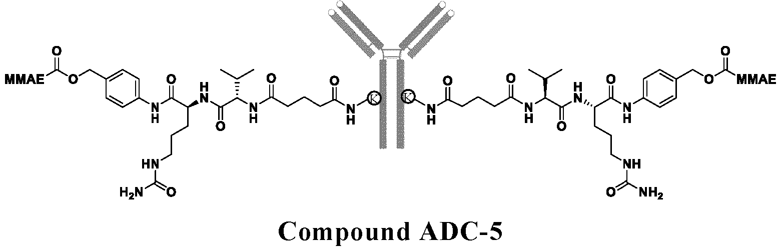

### 57-1. Preparation of Antibody ADC-5:

Trastuzumab (5 mg/mL) and compound **ATC-6** (10 eq, 0.334 mM) were added to PIPES buffer solution (50 mM, pH 7.4, containing 20% DMF) and the mixture was incubated at 37 °C for 3 h. The reaction was complete as monitored by LC-MS. The reaction was subjected to concentration by ultrafiltration tube, and then replacement with 1 × PBS buffer to obtain **ADC-5.** The mass was measured by ESI-TOFMS. The peak of the raw material trastuzumab was observed at 145870. Regarding the reaction product, the peak of the product coupled with two drug linkers **D5** was observed at 148311 (FIG. 11a).

### 57-2. Confirmation of heavy chain selectivity of ADC-5:

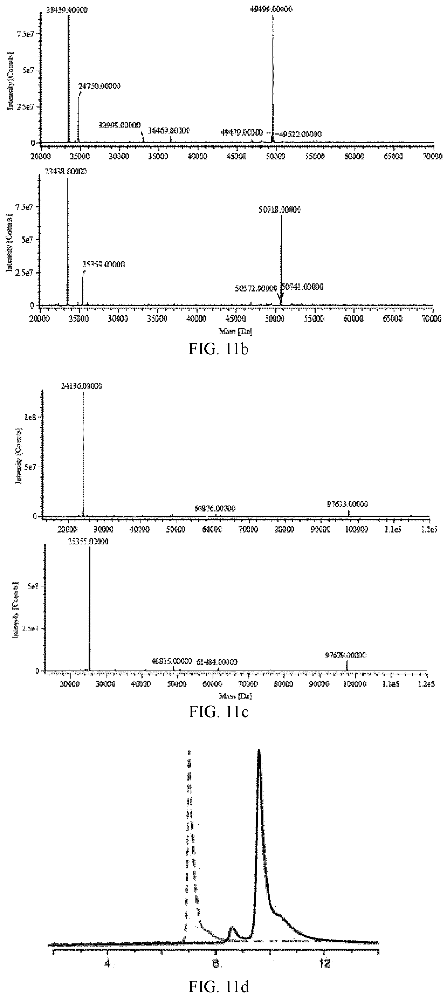

The condition of TCEP treatment was the same as that for ADC-1. The mass was measured by ESI-TOFMS. The same light chain peak was observed for the raw material and the product. The peak of the heavy chain of the raw material was observed at 49499. Regarding the product, the peak of the heavy chain introduced with one drug linker **D5** was observed at 50718 (FIG. 11b).

### 57-3. Confirmation of Fc selectivity of ADC-5:

The condition of IdeS protease treatment was the same as that for ADC-1. The mass was measured by ESI-TOFMS. The same Fab peak was observed for the raw material and the product. The peak of Fc of the raw material was observed at 24136. Regarding the product, the peak of the Fc region introduced with one drug linker **D5** was observed at 25355 (FIG. 11c).

### 57-4. HIC analysis (Hydrophobic Interaction Chromatography analysis) ofADC-5

The HIC analysis operation was the same as that for **ADC-1.** The retention time of 8.687 min was considered to be the compound that has one drug linker **D5** introduced into trastuzumab, and the retention time of 9.676 min was considered to be the compound that has two drug linkers **D5** introduced into trastuzumab (FIG. 11d).

### 57-5. Aggregation stability analysis of ADC-5 (molecular-exclusion chromatography)

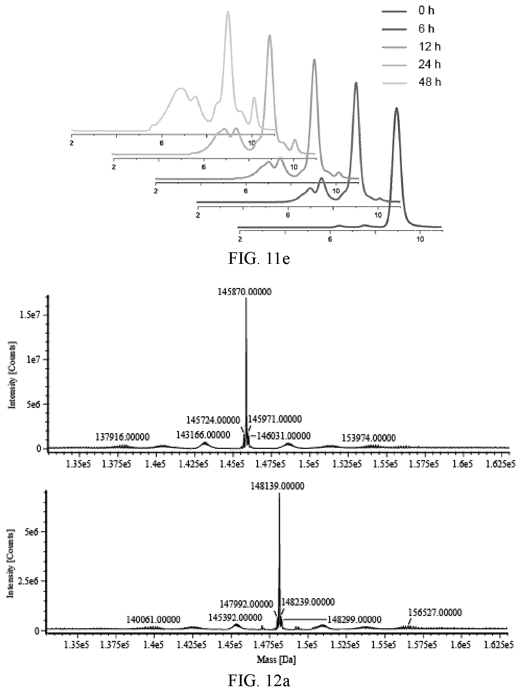

The aggregation stability analysis operation was the same as that for **ADC-1.** The retention time of 5.4-8.0 min was considered to be the peak of aggregates, and the retention time of 8.940 min was considered to be the peak of monomers (FIG. 11e).

### Example 58: Preparation and characterization of ADC-6 using a one-step method

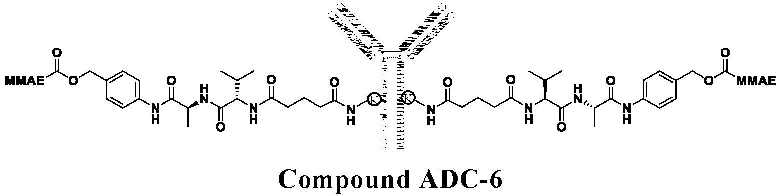

### 58-1. Preparation of Antibody ADC-6:

Trastuzumab (5 mg/mL) and compound **ATC-7** (10 eq, 0.334 mM) were added to PIPES buffer solution (50 mM, pH 7.4, containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain **ADC-6.** The mass was measured by ESI-TOFMS. The peak of the raw material trastuzumab was observed at 145870. Regarding the reaction product, the peak of the product coupled with two drug linkers **D6** was observed at 148139 (FIG. 12a).

### 58-2. Confirmation of heavy chain selectivity of ADC-6:

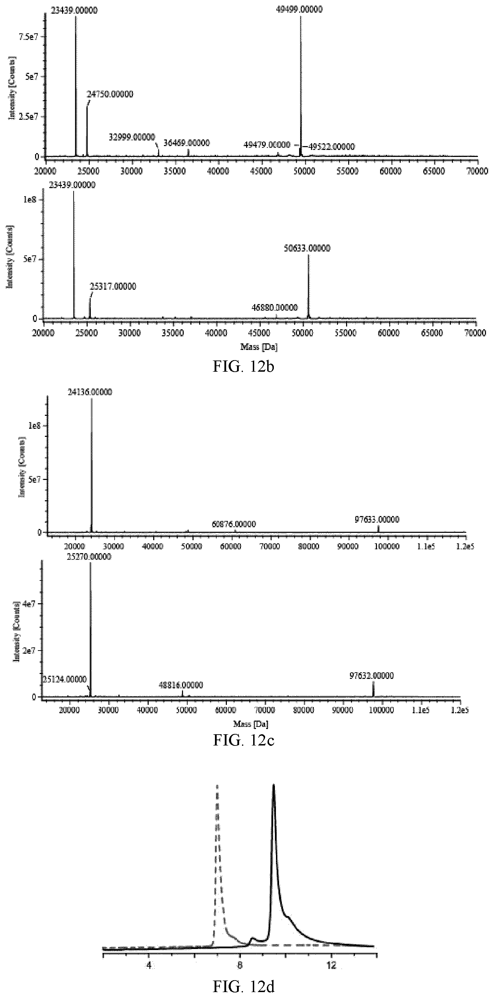

The condition of TCEP treatment was the same as that for ADC-1. The mass was measured by ESI-TOFMS. The same light chain peak was observed for the raw material and the product. The peak of the heavy chain of the raw material was observed at 49499. Regarding the product, the peak of the heavy chain introduced with one drug linker **D6** was observed at 50633 (FIG. 12b).

### 58-3. Confirmation of Fc selectivity of ADC-6:

The condition of IdeS protease treatment was the same as that for ADC-1. The mass was measured by ESI-TOFMS. The same Fab peak was observed for the raw material and the product. The peak of Fc of the raw material was observed at 24136. Regarding the product, the peak of the Fc region introduced with one drug linker **D6** was observed at 25270 (FIG. 12c).

### 58-4. HIC analysis (Hydrophobic Interaction Chromatography analysis) of ADC-6

The HIC analysis operation was the same as that for **ADC-1.** The retention time of 8.623 min was considered to be the compound that has one drug linker **D6** introduced into trastuzumab, and the retention time of 9.565 min was considered to be the compound that has two drug linkers **D6** introduced into trastuzumab (FIG. 12d).

### 58-5. Aggregation stability analysis of ADC-6 (molecular-exclusion chromatography)

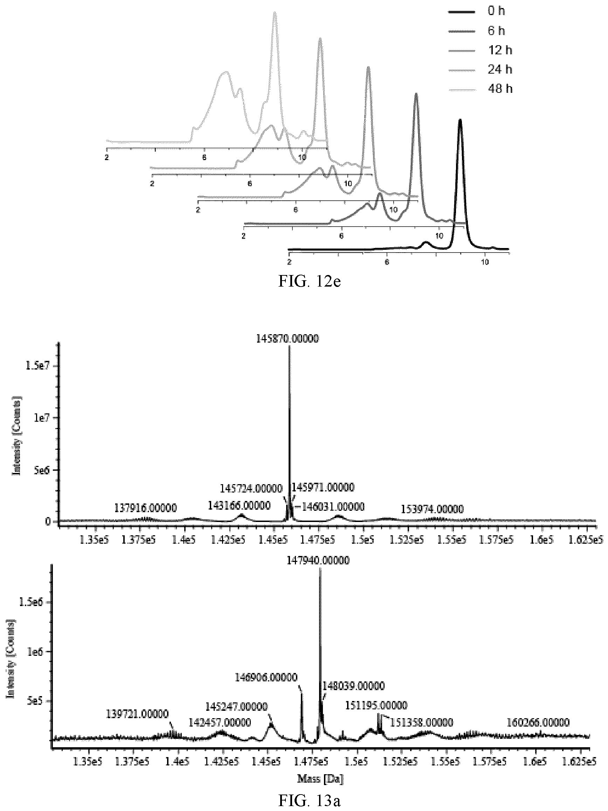

The aggregation stability analysis operation was the same as that for **ADC-1.** The retention time of 5.4-8.0 min was considered to be the peak of aggregates, and the retention time of 8.883 min was considered to be the peak of monomers (FIG. 12e).

### Example 59: Preparation and characterization of ADC-7 using a one-step method

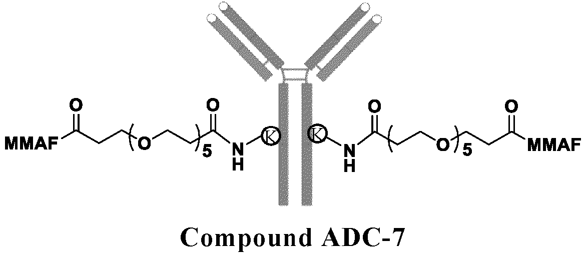

### 59-1. Preparation of Antibody ADC-7:

Trastuzumab (5 mg/mL) and compound **ATC-8** (10 eq, 0.334 mM) were added to PIPES buffer solution (50 mM, pH 7.4, containing 20% DMF) and the mixture was incubated at 37 °C for 10 h. The reaction was complete as monitored by LC-MS. The reaction was subjected to concentration by ultrafiltration tube, and then replacement with 1 × PBS buffer to obtain **ADC-7.** The mass was measured by ESI-TOFMS. The peak of the raw material trastuzumab was observed at 145870. Regarding the reaction product, the peak of the product coupled with two drug linkers **D7** was observed at 147938 (FIG. 13a).

### 59-2. Confirmation of heavy chain selectivity of ADC-7:

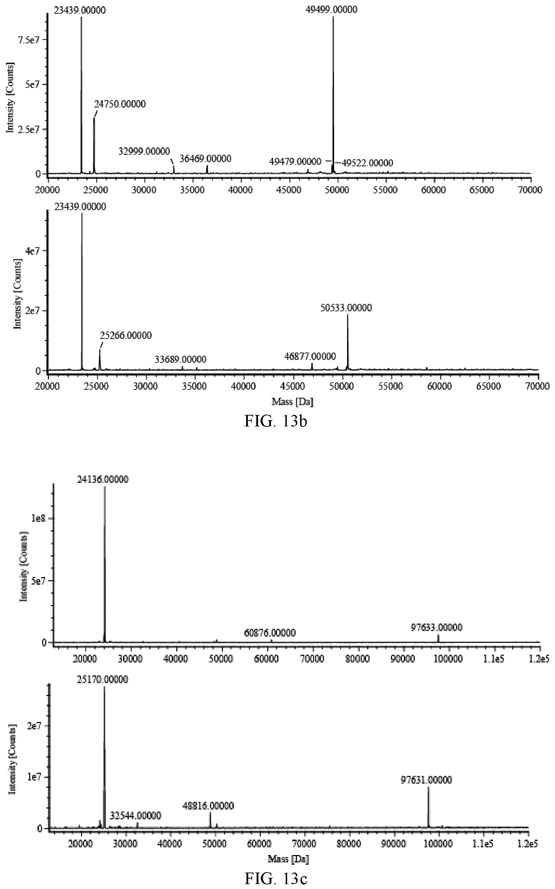

The condition of TCEP treatment was the same as that for ADC-1. The mass was measured by ESI-TOFMS. The same light chain peak was observed for the raw material and the product. The peak of the heavy chain of the raw material was observed at 49499. Regarding the product, the peak of the heavy chain introduced with one drug linker **D7** was observed at 50533 (FIG. 13b).

### 59-3. Confirmation of Fc selectivity of ADC-7:

The condition of IdeS protease treatment was the same as that for ADC-1. The mass was measured by ESI-TOFMS. The same Fab peak was observed for the raw material and the product. The peak of Fc of the raw material was observed at 24136. Regarding the product, the peak of the Fc region introduced with one drug linker **D7** was observed at 25170 (FIG. 13c).

### 59-4. HIC analysis (Hydrophobic Interaction Chromatography analysis) of ADC-7

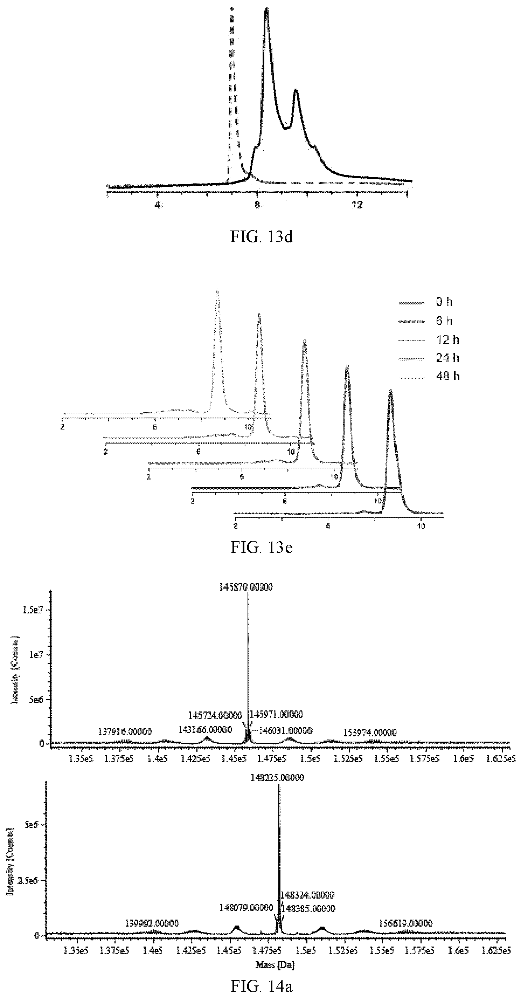

The HIC analysis operation was the same as that for **ADC-1.** The retention time of 8.623 min was considered to be a compound that has two drug linkers **D7** introduced into trastuzumab (FIG. 13d).

### 59-5. Aggregation stability analysis of ADC-7 (molecular-exclusion chromatography)

The aggregation stability analysis operation was the same as that for **ADC-1.** The retention time of 5.4-8.0 min was considered to be the peak of aggregates, and the retention time of 8.690 min was considered to be the peak of monomers (FIG. 13e).

### Example 60: Preparation and characterization of ADC-8 using a one-step method

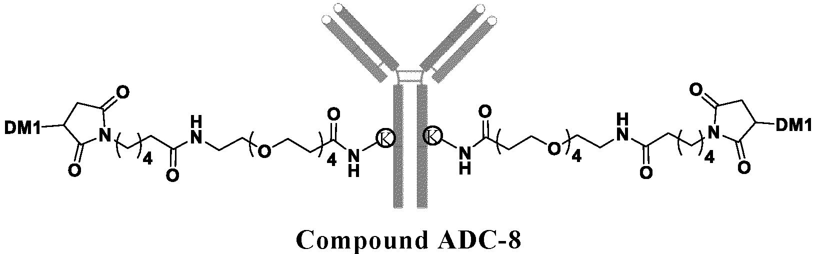

### 60-1. Preparation of Antibody ADC-8:

Trastuzumab (5 mg/mL) and compound **ATC-9** (10 eq, 0.334 mM) were added to PIPES buffer solution (50 mM, pH 7.4, containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain **ADC-8.** The mass was measured by ESI-TOFMS. The peak of the raw material trastuzumab was observed at 145870. Regarding the reaction product, the peak of the product coupled with two drug linkers **D8** was observed at 148225 (FIG. 14a).

### 60-2. Confirmation of heavy chain selectivity of ADC-8:

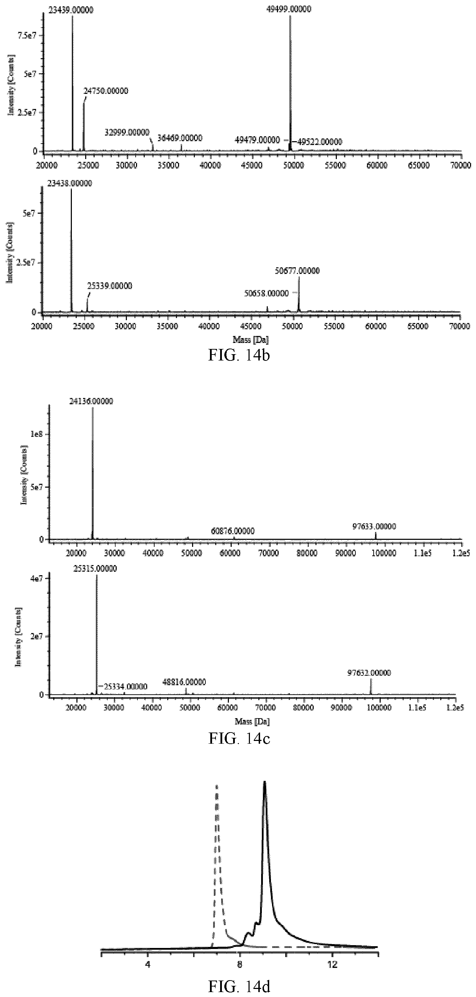

The condition of TCEP treatment was the same as that for ADC-1. The mass was measured by ESI-TOFMS. The same light chain peak was observed for the raw material and the product. The peak of the heavy chain of the raw material was observed at 49499. Regarding the product, the peak of the heavy chain introduced with one drug linker **D8** was observed at 50677 (FIG. 14b).

### 60-3. Confirmation of Fc selectivity of ADC-8:

The condition of IdeS protease treatment was the same as that for ADC-1. The mass was measured by ESI-TOFMS. The same Fab peak was observed for the raw material and the product. The peak of Fc of the raw material was observed at 24136. Regarding the product, the peak of the Fc region introduced with one drug linker **D8** was observed at 25315 (FIG. 14c).

### 60-4. HIC analysis (Hydrophobic Interaction Chromatography analysis) of ADC-8

The HIC analysis operation was the same as that for **ADC-1.** The retention time of 8.370 min was considered to be the compound that has one drug linker **D8** introduced into trastuzumab, and the retention time of 9.063 min was considered to be the compound that has two drug linkers **D8** introduced into trastuzumab (FIG. 14d).

### 60-5. Aggregation stability analysis of ADC-8 (molecular-exclusion chromatography)

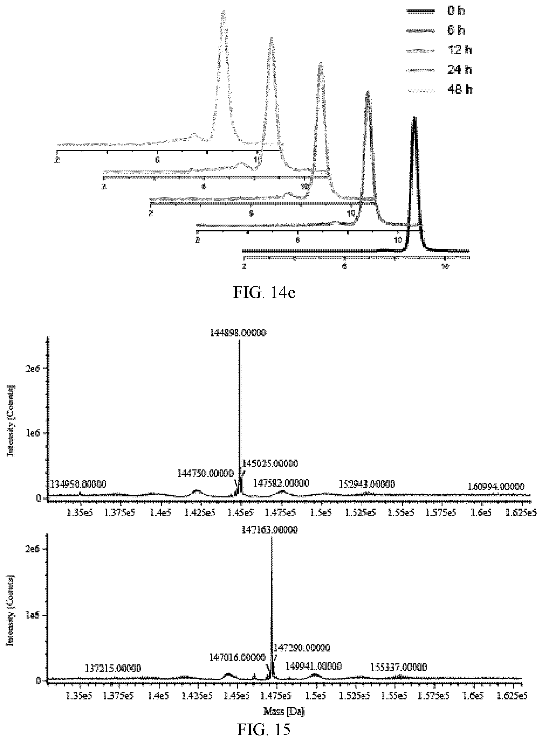

The aggregation stability analysis operation was the same as that for **ADC-1.** The retention time of 5.4-8.0 min was considered to be the peak of aggregates, and the retention time of 8.623 min was considered to be the peak of monomers (FIG. 14e).

### Example 61: Preparation and characterization of ADC-9 using a one-step method

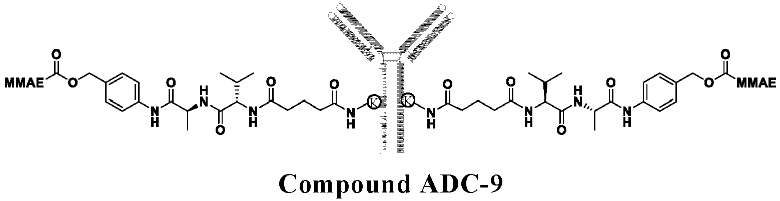

### Preparation of ADC-9:

Rituximab (5 mg/mL) and compound **ATC-7** (10 eq, 0.334 mM) were added to PIPES buffer solution (50 mM, pH 7.4, containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain **ADC-9.** The mass was measured by ESI-TOFMS. The peak of the raw material Rituximab was observed at 144898. Regarding the reaction product, the peak of the product coupled with two drug linkers **D6** was observed at 147136 (FIG. 15).

### Example 62: Preparation and characterization of ADC-10 using a one-step method

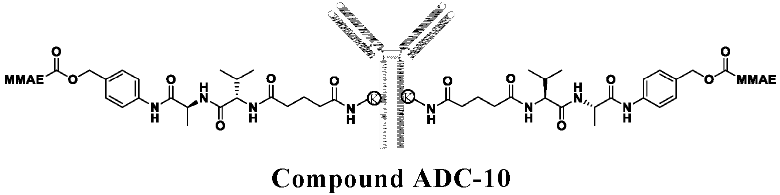

### Preparation of ADC-10:

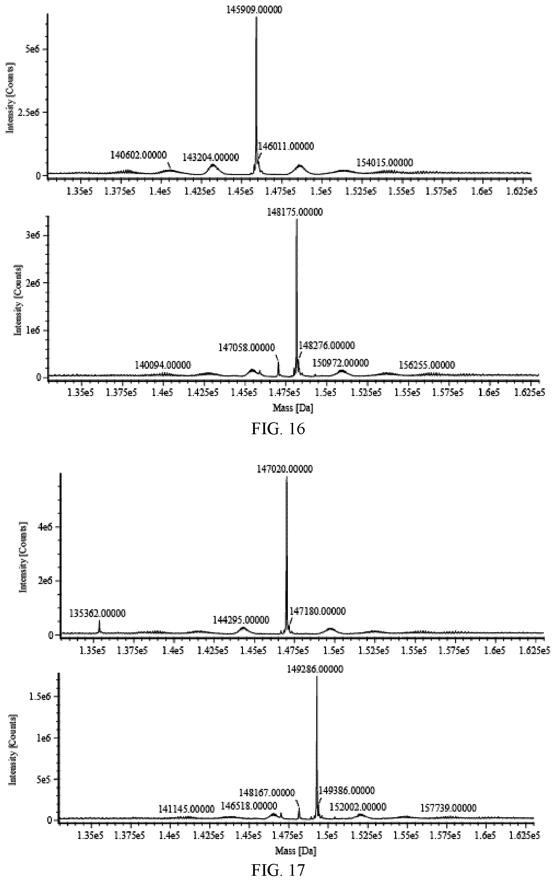

Pertuzumab (5 mg/mL) and compound **ATC-7** (10 eq, 0.334 mM) were added to PIPES buffer solution (50 mM, pH 7.4, containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain **ADC-10.** The mass was measured by ESI-TOFMS. The peak of the raw material Pertuzumab was observed at 145909. Regarding the reaction product, the peak of the product coupled with two drug linkers **D6** was observed at 148175 (FIG. 16).

### Example 63: Preparation and characterization of ADC-11 using a one-step method

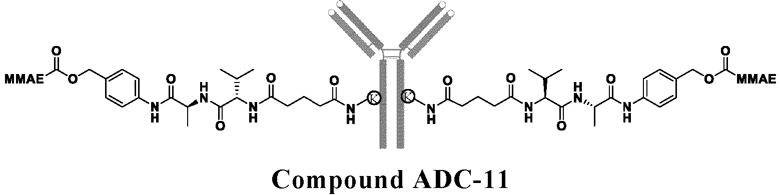

### Preparation of ADC-11:

Bevacizumab (5 mg/mL) and compound **ATC-7** (10 eq, 0.334 mM) were added to PIPES buffer solution (50 mM, pH 7.4, containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain **ADC-11.** The mass was measured by ESI-TOFMS. The peak of the raw material Bevacizumab was observed at 147020. Regarding the reaction product, the peak of the product coupled with two drug linkers **D6** was observed at 149286 (FIG. 17).

### Example 64: Preparation and characterization of ADC-12 using a one-step method

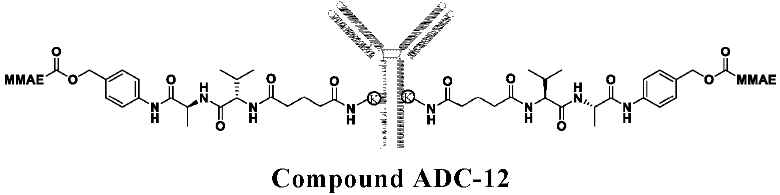

### Preparation of ADC-12:

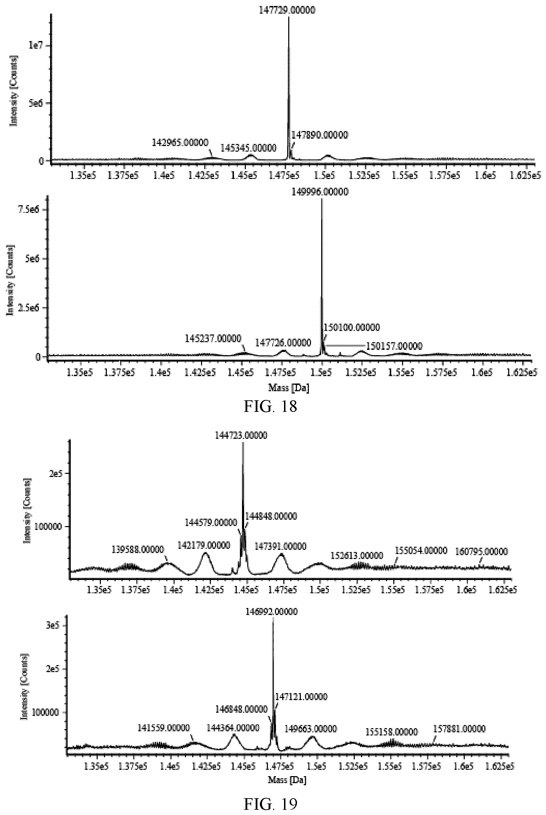

Toripalimab (5 mg/mL) and compound **ATC-7** (10 eq, 0.334 mM) were added to PIPES buffer solution (50 mM, pH 7.4, containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain **ADC-12.** The mass was measured by ESI-TOFMS. The peak of the raw material Toripalimab was observed at 147729. Regarding the reaction product, the peak of the product coupled with two drug linkers D6 was observed at 149996 (FIG. 18).

### Example 65: Preparation and characterization of ADC-13 using a one-step method

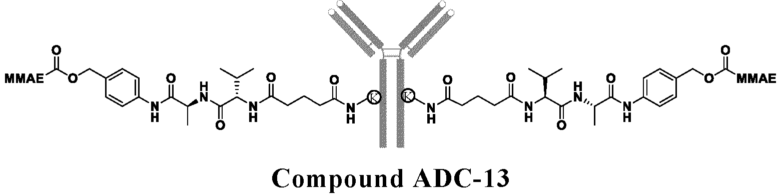

### Preparation of ADC-13:

Panituzumab (2 mg/mL) and compound **ATC-7** (10 eq, 0.134 mM) were added to 1 × PBS buffer solution (containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain **ADC-13.** The mass was measured by ESI-TOFMS. The peak of the raw material Panituzumab was observed at 144723. Regarding the reaction product, the peak of the product coupled with two drug linkers **D6** was observed at 146992 (FIG. 19).

### Example 66: Preparation and characterization of ADC-14 using a one-step method

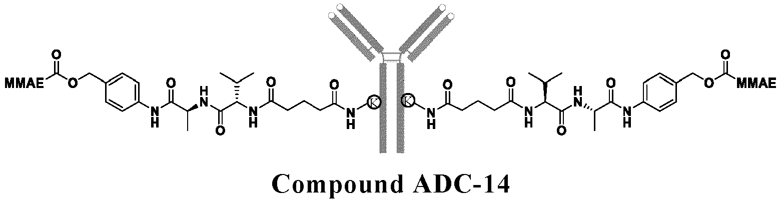

### Preparation of ADC-14:

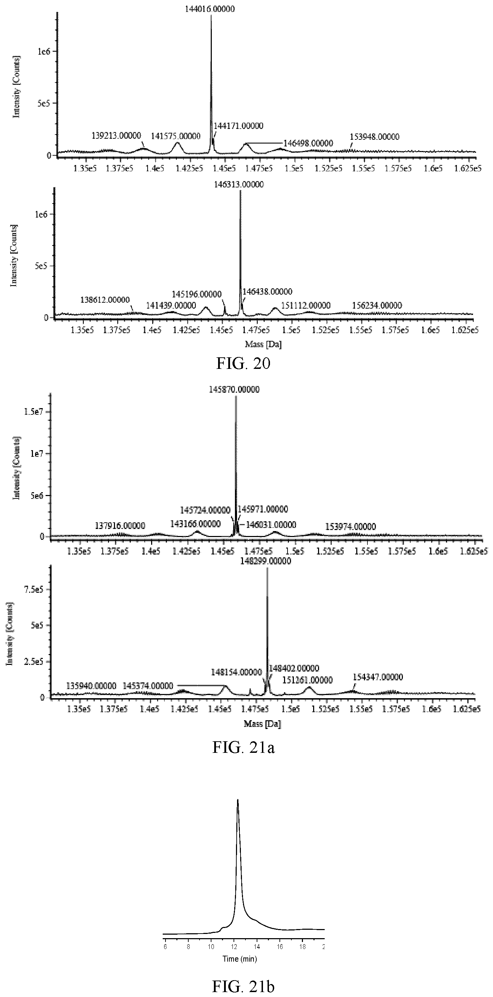

Nivolumab (2 mg/mL) and compound **ATC-7** (10 eq, 0.134 mM) were added to 1 × PBS buffer solution (containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain **ADC-13.** The mass was measured by ESI-TOFMS. The peak of the raw material Nivolumab was observed at 144016. Regarding the reaction product, the peak of the product coupled with two drug linkers **D6** was observed at 146313 (FIG. 20).

### Example 67: Preparation and characterization of ADC-15 using a one-step method

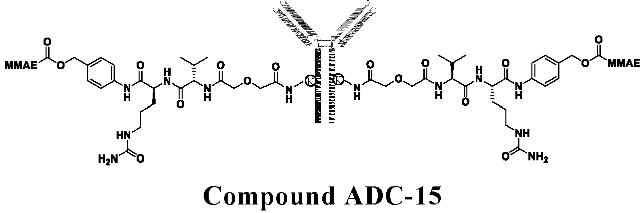

### Preparation of ADC-15:

Trastuzumab (5 mg/mL) and compound **ATC-40** (10 eq, 0.334 mM) were added to PIPES buffer solution (50 mM, pH 7.4, containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain **ADC-15.** The mass was measured by ESI-TOFMS. The peak of the raw material trastuzumab was observed at 145860. Regarding the reaction product, the peak was observed at 148299 (FIG. 21a).

The HIC analysis operation was the same as that for **ADC-1** (FIG. 21b).

### Example 68: Preparation and characterization of ADC-16 using a one-step method

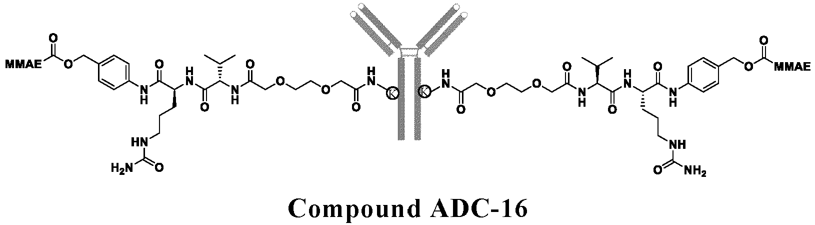

### Preparation of ADC-16:

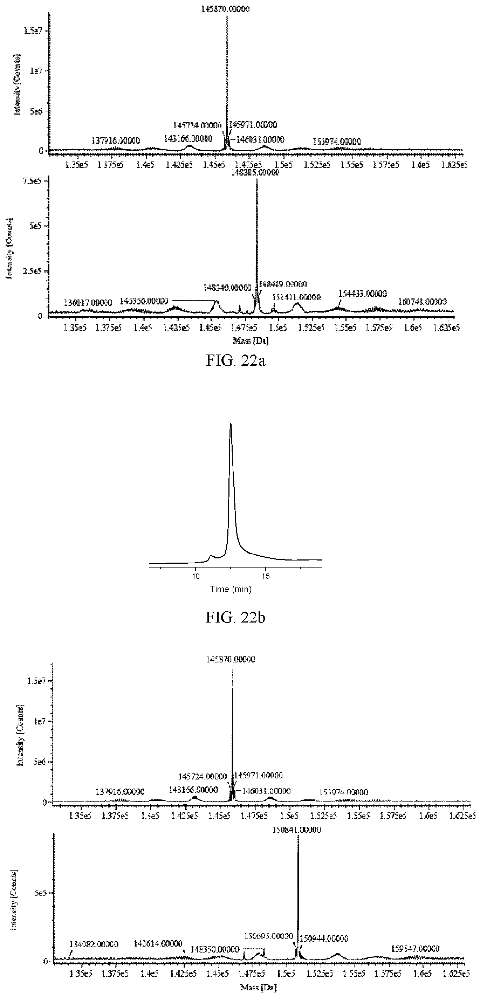

Trastuzumab (5 mg/mL) and compound **ATC-41** (10 eq, 0.334 mM) were added to PIPES buffer solution (50 mM, pH 7.4, containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain **ADC-16.** The mass was measured by ESI-TOFMS. The peak of the raw material trastuzumab was observed at 145860. Regarding the reaction product, the peak was observed at 148385 (FIG. 22a).

The HIC analysis operation was the same as that for **ADC-1** (FIG. 22b).

### Example 69: Preparation and characterization of ADC-17 using a one-step method

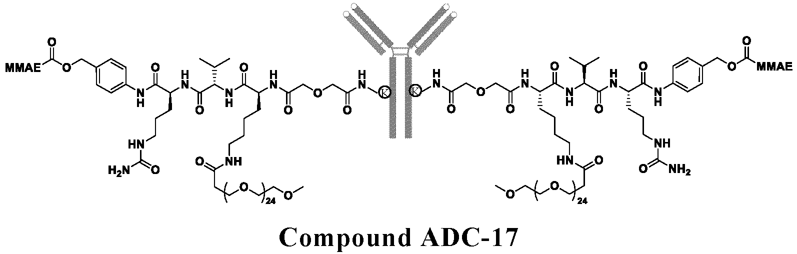

### Preparation of ADC-17:

Trastuzumab (5 mg/mL) and compound **ATC-42** (10 eq, 0.334 mM) were added to PIPES buffer solution (50 mM, pH 7.4, containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain **ADC-17.** The mass was measured by ESI-TOFMS. The peak of the raw material trastuzumab was observed at 145860. Regarding the reaction product, the peak was observed at 150841 (FIG. 23a).

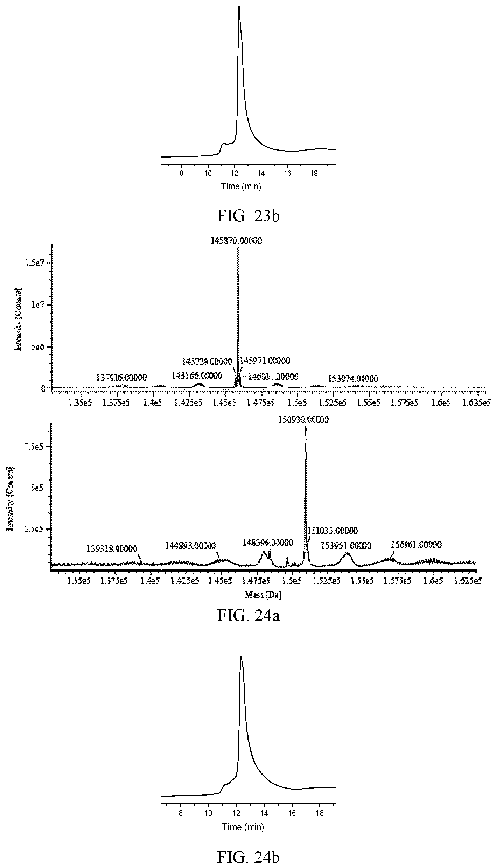

The HIC analysis operation was the same as that for **ADC-1** (FIG. 23b).

### Example 70: Preparation and characterization of ADC-18 using a one-step method

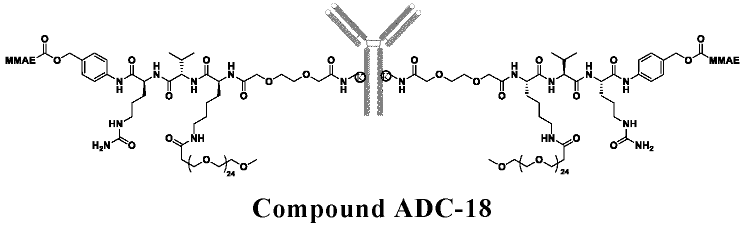

### Preparation of ADC-18:

Trastuzumab (5 mg/mL) and compound **ATC-43** (10 eq, 0.334 mM) were added to PIPES buffer solution (50 mM, pH 7.4, containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain **ADC-18.** The mass was measured by ESI-TOFMS. The peak of the raw material trastuzumab was observed at 145860. Regarding the reaction product, the peak was observed at 150930 (FIG. 24a).

The HIC analysis operation was the same as that for **ADC-1 (****FIG.** 24b).

### Example 71: Preparation and characterization of ADC-19 using a one-step method

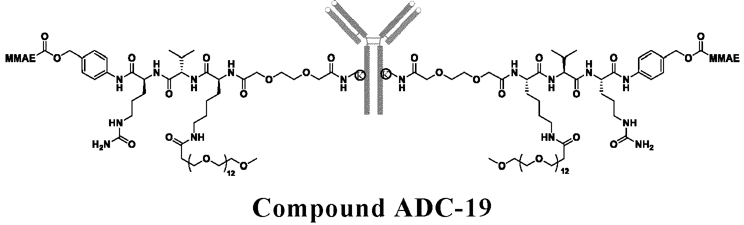

### Preparation of ADC-19:

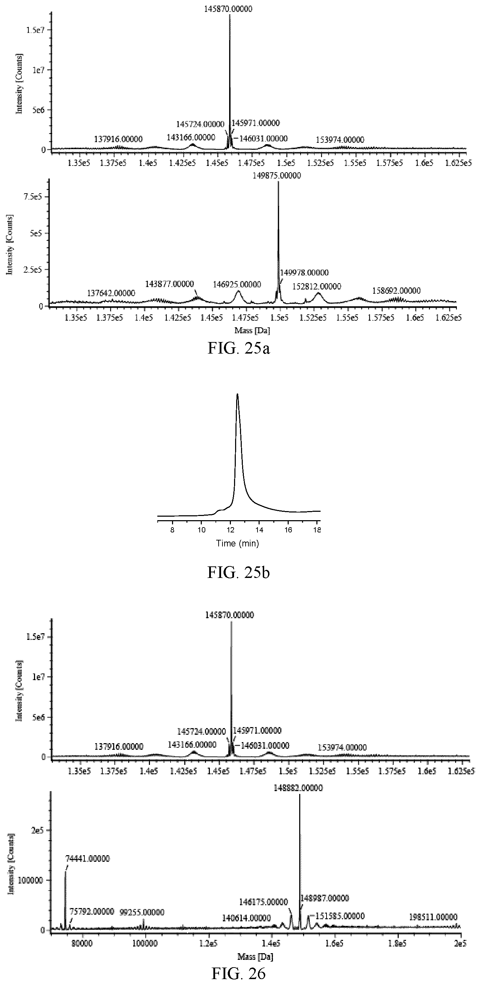

Trastuzumab (5 mg/mL) and compound **ATC-44** (10 eq, 0.334 mM) were added to PIPES buffer solution (50 mM, pH 7.4, containing 20% DMF) and the mixture was incubated at 37 °C for 2 h. The reaction was complete as monitored by LC-MS. The reaction was subjected to concentration by ultrafiltration tube, and then replacement with 1 × PBS buffer to obtain **ADC-19.** The mass was measured by ESI-TOFMS. The peak of the raw material trastuzumab was observed at 145860. Regarding the reaction product, the peak was observed at 149875 (FIG. 25a).

The HIC analysis operation was the same as that for ADC-1 (FIG. 25b).

### Example 72: Preparation and characterization of ADC-20 using a one-step method

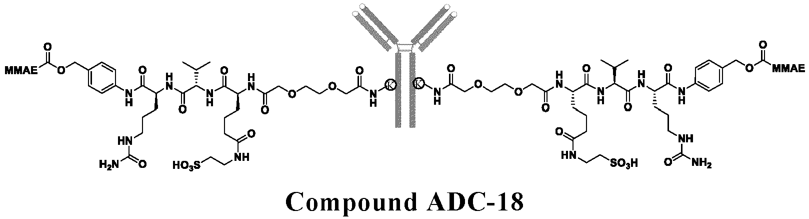

### Preparation of ADC-20:

Trastuzumab (5 mg/mL) and compound **ATC-45** (10 eq, 0.334 mM) were added to PIPES buffer solution (50 mM, pH 7.4, containing 20% DMF) and the mixture was incubated at room temperature for 1 h. The reaction was complete as monitored by LC-MS. The reaction was subjected to concentration by ultrafiltration tube, and then replacement with 1 × PBS buffer to obtain **ADC-20.** The mass was measured by ESI-TOFMS. The peak of the raw material trastuzumab was observed at 145860. Regarding the reaction product, the peak was observed at 148882 (FIG. 26).

### Example 73: Preparation and characterization of DAR4 ADC-21 using a one-step method

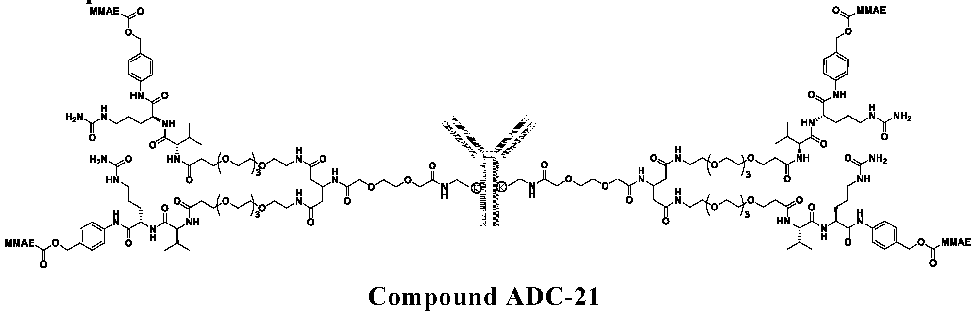

### Preparation of ADC-21:

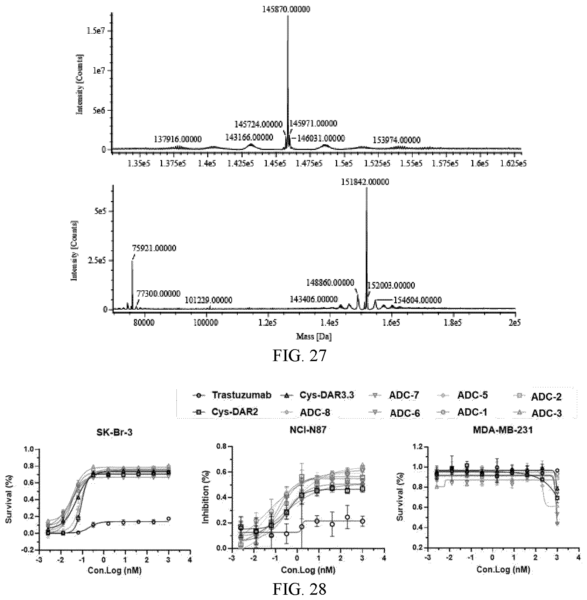

Trastuzumab (5 mg/mL) and compound **ATC-46** (10 eq, 0.334 mM) were added to sodium acetate buffer solution (50 mM, pH 5.5, containing 20% DMF) and the mixture was incubated at 37 °C for 1 h. The reaction was complete as monitored by LC-MS. The reaction was subjected to concentration by ultrafiltration tube, and then replacement with 1 × PBS buffer to obtain **ADC-21.** The mass was measured by ESI-TOFMS. The peak of the raw material trastuzumab was observed at 145860. Regarding the reaction product, the peak was observed at 151842 (FIG. 27).

### Test Example 1

### Activity data experimental process and result analysis

The cell activity and toxicity of some of the above ADCs were determined using the MTT method. Cells were digested with trypsin, and collected by centrifugation after termination. The cells were resuspended in 1-3 mL of culture medium. The cells were counted after mixing, and added with culture medium to adjust the concentration of the cell suspension. A 96-well plate was used for cell experiments. Each well was added with 90 µL of cell suspension to make the cell density of cells to be tested of ~6000/well. 3 replicate wells were set up. The edge wells were filled by adding 100 µL 1 x PBS, and then zero wells (90 µL culture medium) and control wells (90 µL cells, without drugs) were set up at the same time. The plate was incubated in a 5% CO₂, 37 °C incubator for 12 h to allow the cells to fully adhere to the wall. Nine concentration gradients were set up for each sample, with the highest concentration being 1 µM and 5-fold gradient dilutions. Each well was added with 10 µL ADC molecules of the corresponding concentration gradient, and 6 control wells were also added with 10 µL of culture medium respectively. After incubation at 37 °C with 5% CO₂ for 72 h, each well was added with 10 µL of MTT solution (5 mg/mL) and continued to culture for 4 h. Subsequently, each well was added with 90 µL of SDS lysis buffer. After incubation at 37 °C for 7 h, the absorbance value of each well at 570 nm was measured using an enzyme-linked immunosorbent assay instrument and data were processed using GraphPad Prism 8 software (FIG. 28).

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A conjugate containing a ligand affinity directing group, wherein the conjugate is of Formula I:
AT-CL-R (I)
wherein,
AT is an affinity moiety for target protein (TP);
CL is a cleavable fragment; and possesses divalent fragments as shown below in the CL; 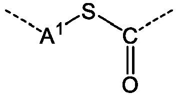
wherein A¹ is each independently optionally substituted C₁₋₁₀ alkylene, optionally substituted C₆₋₁₀ aryl, or optionally substituted 5 to 10 membered heteroaryl:
R is a group that needs to be modified onto the target protein;
unless otherwise specified, said substituted means that one or more H atoms in the group are substituted with substituents selected from the group consisting of: halogen, C₁₋₆ alkyl, and C₁₋₆ haloalkyl.

2. The conjugate of claim 1, wherein the target protein is an antibody or fusion protein containing Fc domain; and/or AT is a polypeptide having the ability to bind to Fc in the the antibody.

3. The conjugate of claim 1, wherein AT is the core sequence derived from an Fc binding polypeptide containing the sequence shown in Formula II:
(Xₛ₁)t₁-Cys-Aa1-Aa2-Aa3-Aa4-Aa5-Aa6-Aa7-Aa8-Aa9-Cys-(Yₛ₂)t₂ (II)
wherein X is located at the N-terminal of the polypeptide, and Y is located at the C-terminal of the polypeptide;
s1 = 0, 1, 2 or 3;
s2 = 0, 1, 2 or 3;
X and Y are each independently amino acid residues;
t1 and t2 are each independently an integer of 0 to 10;
Cys is a cysteine residue;
Aa1, Aa2, Aa3, Aa5, Aa7, Aa8, and Aa9 are each independently amino acid residues;
Aa4 and Aa6 are each independently an amino acid residue, and at least one of Aa4 and Aa6 is an amino acid residue containing an NH₂ group on its side chain or an amino acid residue containing an -COOH group on its side chain;
and/or
CL is as shown below 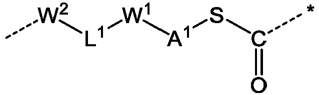
wherein,
* represents the connection site with R
W¹ is selected from the group consisting of: null (single bond), -NH-, -C(O)-, -C(O)-NH-, and -NH-C(O)-;
W² is null (single bond), -NH-, or -C(O)-;
L¹ is null (single bond) or a divalent linker group;
A¹ is as defined in claim 1;
and/or
R is a moiety comprising one or more active groups selected from the group consisting of: active group that can be further modified, and active group that are biologically active and/or detectable.

4. The conjugate of claim 3, wherein
the active group that can be further modified refers to active groups that can undergo bioorthogonal reaction; preferably, the active group that can undergo bioorthogonal reaction is selected from the group consisting of: azido, aldehyde group, thiol, alkynyl, alkynyl, halogen, tetrazinyl , nitrone group, hydroxyl amino, nitrile group, hydrazine group, ketone group, boronic acid group, cyanobenzothiazolyl, allyl, phosphine group, maleimide group, disulfide group, thioester group, α-halogenated carbonyl, isonitrile group, sydnone group, seleno, conjugated dienyl, phosphate group, cycloalkynyl and cycloalkenyl, and combinations thereof; and/or
the active group that isbiologically active and/or detectable is selected from the group consisting of: maytansine, DM-1, DM-4, MMAE, MMAF, SN-38, Dxd, PBD and its analogs, amanitin, vincristine, vinblastine, vinorelbine, VP-16, camptothecin, paclitaxel, docetaxel, epothilone A, epothilone B, nocodazole, colchicine, estramustine, cemadotin, eleutherobin, fluorescent reagents, monosaccharides, disaccharides, oligosaccharides, polyethylene glycol (PEG), immune agonists, cytotoxin, radioactive therapeutic substances, and molecular imaging agents, and combinations thereof.

5. The conjugate of claim 1, wherein the conjugate is of formula III-A1, III-A2 or III-A3; 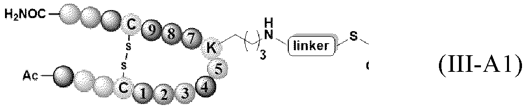 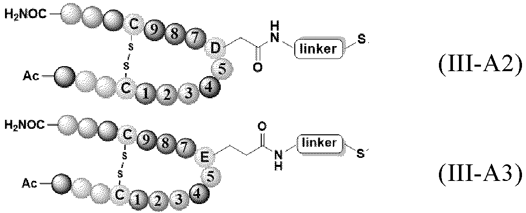 wherein
the circles labeled with 1, 2, 3, 4, 5, 7, 8, and 9 are defined as Aa1 Aa2, Aa3, Aa4, Aa5, Aa7, Aa8, and Aa9 in formula II of claim 3;
the end close to the circle labeled with 1 is the N-terminal of the polypeptide, and the end close to the circle labeled with 9 is the C-terminal of the polypeptide;
Ac is a N-terminal protecting group or absent;
each blank circle is defined as X and Y in formula II;
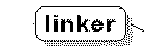 is -W²-L¹-W¹-A¹-;
Circle C represents a cysteine residue, and - S-S- represents a disulfide bond formed by the side chain SH of the cysteine residue;
Circle K is the main chain moiety of a lysine residue; Circle D is the main chain moiety of an aspartic acid residue (D); Circle E is the main chain moiety of a glutamic acid residue (E);
R is as defined in claim 1 or 3.

6. The conjugate of claim 1, wherein the conjugate is of formula III-B1, III-B2 or III-B3; 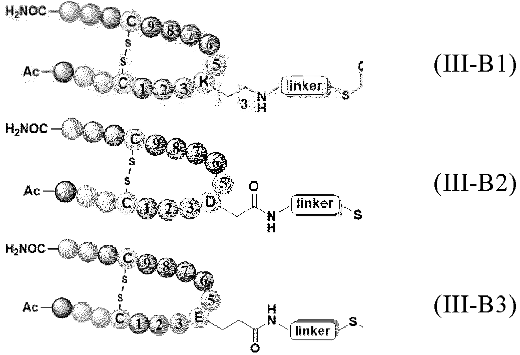 wherein,
the circles labeled with 1, 2, 3, 5, 6, 7, 8, and 9 are defined as Aa1 Aa2, Aa3, Aa5, Aa6, Aa7, Aa8, and Aa9 in formula II of claim 3;
the end close to the circle labeled with 1 is the N-terminal of the polypeptide, and the end close to the circle labeled with 9 is the C-terminal of the polypeptide;
Ac is a N-terminal protecting group or absent;
each blank circle is defined as X and Y in formula II;
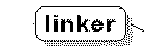 is -W²-L¹-W¹-A¹-;
Circle C represents a cysteine residue, and - S-S- represents a disulfide bond formed by the side chain SH of the cysteine residue;
Circle K is the main chain moiety of a lysine residue; Circle D is the main chain moiety of an aspartic acid residue (D); Circle E is the main chain moiety of a glutamic acid residue (E);
R is as defined in claim 1 or 3.

7. The conjugate of claim 1, wherein the conjugate is a conjugate of formula III-A1-1, III-A2-1 or III-A3-1; 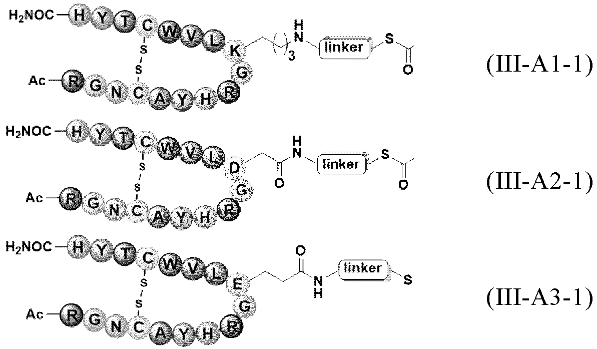 wherein,
Circle R is the N-terminal of the polypeptide, and Circle H is the C-terminal of the polypeptide;
Ac is a N-terminal protecting group or absent;
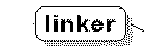 is -W²-L¹-W¹-A¹-;
Circle R represents an arginine residue, Circle G represents a glycine residue, Circle N represents an asparagine residue, Circle A represents an alanine residue, Circle Y represents a tyrosine residue, Circle H represents a histidine residue, Circle L represents a leucine residue, Circle V represents a valine residue, Circle W represents a tryptophan residue, and Circle T represents a threonine residue;
Circle C represents a cysteine residue, and - S-S- represents a disulfide bond formed by the side chain SH of the cysteine residue;
Circle K is the main chain moiety of a lysine residue; Circle D is the main chain moiety of an aspartic acid residue; Circle E is the main chain moiety of a glutamic acid residue.
R is as defined in claim 1 or 3.

8. The conjugate of any one of claims 1-7, wherein the conjugate is selected from Table A1 and Table A2.

9. A method for modifying a protein, comprising steps of:
(1) providing the conjugate of any one of claims 1 to 8;
(2) contacting the target protein that needs to be modified with the conjugate, and allowing the conjugate to react with the side chain amino group and/or the terminal amino group on the protein, thereby obtaining the modified target protein.

10. The method according to claim 9, wherein the modification can obtain the modified target protein through a one-step reaction.

11. A site-selectively modified protein of formula IV, wherein
TP-(NHCO-R)ₚ (IV)
wherein, TP is a target protein moiety, R is as defined in claim 1 or 3, and subscript p is an integer from 1 to 8.

12. An antibody-drug conjugate, wherein the antibody-drug conjugate is of formula V-A:
Ab-(NHCO-L₂-(L₃-D)ₒ)ₚ (V-A)
wherein Ab is an antibody, subscript o is an integer from 1 to 10, subscript p is an integer from 1 to 8, L₂ and L₃ are each independently null or a linker fragment, and D is each independently the same or different active groups that are biologically active and/or detectable.

13. The antibody-drug conjugate of claim 12, wherein group D is site-directly coupled to the antibody constant region (Fc region); preferably, group D is site-directly coupled to a region consisting of amino acid residues at positions 246-248 in the human IgG Fc region.

14. The protein of claim 12, wherein the antibody-drug conjugate is selected from the group consisting of: ADC-1, ADC-2, ADC-3, ADC-4, ADC-5, ADC-6, ADC-7, ADC-8, ADC-9, ADC-10, ADC-11, ADC-12, ADC13, ADC-14, ADC-15, ADC-16, ADC-17, ADC-18, ADC-19, ADC-20 and ADC-21; preferably, the antibody-drug conjugate is selected from the group consisting of: ADC-5, ADC-6, ADC-7, ADC-8, ADC-9, ADC-10, ADC-11, ADC-12, ADC13, ADC-14, ADC-15, ADC-16, ADC-17, ADC-18, ADC-19, ADC-20 and ADC-21.

15. Use of the conjugate of claim 1 for site-specific modification of proteins.

16. Use of the conjugate of claim 1 in the preparation of antibody drug conjugates (ADCs), preferably, the use does not rely on bioorthogonal reactions, and the conjugate can directly modify the drug molecule that needs to be modified to the target protein onto the antibody in a site-specific manner through a one-step reaction.
